# EUROPEAN PATENT APPLICATION

(11) **EP 4 353 713 A1**
(43) Date of publication of application: **17.04.2024**
(21) Application number: 22820195.0
(22) Date of filing: 06.06.2022
(51) Int. Cl.: C07D 235/26, A61K 31/4184, A61K 31/454, A61P 3/10, A61P 9/00, A61P 9/12, A61P 13/10, A61P 13/12, A61P 15/00, A61P 17/00, A61P 17/04, A61P 25/04, A61P 25/08, A61P 25/16, A61P 25/18, A61P 25/20, A61P 25/22, A61P 25/24, A61P 25/28, A61P 27/02, A61P 35/00

(54) **T-TYPE CALCIUM CHANNEL BLOCKER**

(30) Priority: 07.06.2021 JP 2021095405
(71) Applicant: Kinki University, Higashiosaka-shi, Osaka 577-8502 (JP); National University Corporation University Of Toyama, Toyama-shi, Toyama 930-8555 (JP); Fuso Pharmaceutical Industries, Ltd., Osaka-shi, Osaka 541-0045 (JP)
(72) Inventor: KAWABATA, Atsufumi, Higashiosaka-shi, Osaka 577-8502 (JP); SEKIGUCHI, Fumiko, Higashiosaka-shi, Osaka 577-8502 (JP); TOYOOKA, Naoki, Toyama-shi, Toyama 930-8555 (JP); OKADA, Takuya, Toyama-shi, Toyama 930-8555 (JP); NISHIKAWA, Hiroyuki, Osaka-shi, Osaka 536-8523 (JP)
(74) Representative: Vossius & Partner Patentanwälte Rechtsanwälte mbB
(86) International application number: PCT/JP2022/022847
(87) International publication number: WO 2022/260016

(57) **Abstract**

The present invention provides a compound of formula (I) : 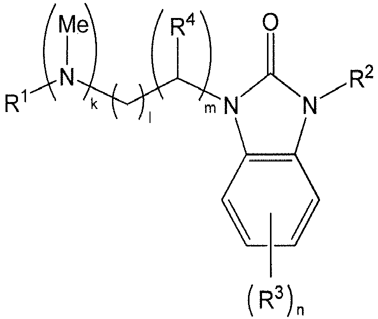 wherein R¹, R², R³, R⁴, k, 1, m and n are as defined in the specification,
or a pharmaceutically acceptable salt thereof with the effect of inhibiting T-type calcium channels and a medicament useful for the treatment of a disease associated with the activation of T-type calcium channels.

## Description

### TECHNICAL FIELD

The present invention relates to a T-type calcium channel inhibitor. More specifically, the present invention relates to a novel compound having 1,3-hydro-2H-benzimidazol-2-one structure with the effect of inhibiting T-type calcium channels. Also, the present invention can be used as a medicament for treating a disease associated with the activation of T-type calcium channels such as pain and pruritus which can inhibit the risk of side effects by decreasing the effect of inhibiting dopamine D₂ receptor (hereinafter referred to as D₂ receptor, as necessary).

### BACKGROUND ART

Ion channels are channels extending through the cell membrane and are classified broadly into two types: ligand-dependent channels and potential-dependent channels. In recent years, as a type of the mechanism of neuropathic pain, a potential-dependent sodium channel and a potential-dependent calcium channel have been found as a target. As drugs targeting a potential-dependent sodium channel, for example, lidocaine, carbamazepine, lamotrigine, mexiletine and the like are known. As drugs targeting a potential-dependent calcium channel, for example, gabapentin, pregabalin, ziconotide and the like are known.

The neuropathic pain is refractory and has the problem of inadequate response to existing analgesic drugs. The efficacy of even an established drug therapy is difficult to predict accurately, and other drugs must often be used in combination. In the Guidelines for the Pharmacologic Management of Neuropathic Pain from Japan Society of Pain Clinicians, high-potential-activated calcium channel inhibitors are listed as a primary choice, but in many cases, an adequate therapeutic effect is not obtained. These inhibitors have the problem of causing side effects such as lightheadedness.

Potential-dependent calcium channels are classified into two types: high-potential-activated types and low-potential-activated types based on a difference in activation and deactivation potentials. L-type, N-type, P/Q-type and R-type calcium channels are activated by large depolarization, and therefore classified as high-potential-activated calcium channels. On the other hand, T-type calcium channels are activated by small depolarization, and therefore classified as low-potential-activated calcium channels.

Recently, T-type calcium channels have been reported to be involved in the onset and development of neuropathic pain, and the potential of T-type calcium channel inhibitors as therapeutic agents for neuropathic pain has been suggested. Examples of the T-type calcium channel inhibitor include mibefradil (for example, Non-Patent Document 1), ethosuximide, and (1S,2S)-2-[2-[[3-(1H-benzimidazol-2-yl)propyl]methyamino]ethyl]-6-fluoro-1,2,3,4-tetrahydro-1-(1-methylethyl)-2-naphthalenyl-cyclopropane carboxylate hydrochloride (NNC55-0396) (for example, Non-Patent Document 2) .

Also, it has been known that T-type calcium channels are associated with the pathology of various diseases and disorders including epilepsy, essential tremor, schizophrenia, Parkinson's disease, depression, anxiety, sleep disorder, mental illness, schizophrenia, cardiac arrhythmia, hypertension, cancer, diabetes, overactive bladder, chronic kidney disease, sterility and sexual dysfunction, besides pains such as neuropathic pain, inflammatory pain and cancer pain (Patent Document 1).

1-{1-[4,4-Bis(4-fluorophenyl)butyl]piperidin-4-yl}-1,3-dihydro-2H-benzimidazol-2-one (hereinafter also referred to as pimozide, as necessary) is currently known as a compound having 1,3-dihydro-2H-benzimidazol-2-one structure with the effect of inhibiting T-type calcium channels. However, the compound has the effect of inhibiting D₂ receptor stronger than the effect of inhibiting T-type calcium channels. Pimozide has the problem of causing undesired effects such as extrapyramidal symptoms due to the stronger effect of inhibiting D₂ receptor. As a result, pimozide could not lead to treat a disease by the effect of inhibiting T-type calcium channels. No compound having 1,3-dihydro-2H-benzimidazol-2-one structure, which can reduce the effect of inhibiting D₂ receptors and effectively inhibit the risk of side effects, has been known yet.

As described above, some compounds, which can be used as a T-type calcium channel inhibitor, have been found. However, there is a need to further find a novel compound which can be used as the T-type calcium channel inhibitor in view of various factors such as side effects.

### PRIOR ART DOCUMENTS

### PATENT DOCUMENTS

Patent Document 1: WO 2015/050212

### NON-PATENT DOCUMENTS

Non-Patent Document 1: Todorovic, Neuron, 2001, 31(1), p.75-85
Non-Patent Document 2: Huang, J Pharmacol Exp Ther., 309(1), p.193-199

### SUMMARY OF INVENTION

### (PROBLEM TO BE SOLVED BY THE INVENTION)

An object of the present invention is to find a novel compound with the effect of inhibiting T-type calcium channels and to provide a medicament useful for the treatment of a disease associated with the activation of T-type calcium channels (for example, pain, pruritus) (for example, T-type calcium channel inhibitor) which can inhibit the risk of side effects by decreasing the effect of inhibiting D₂ receptor.

### (MEANS FOR SOLVING THE PROBLEMS)

The present inventors have extensively studied to reach the above object, and then have found that a compound of the following formula (I) or a pharmaceutically acceptable salt thereof (hereinafter referred to as "the present compound", as necessary) has the effect of inhibiting T-type calcium channels. In addition, the present inventors have found that the risk of side effects can be inhibited by decreasing the effect of inhibiting D₂ receptor. Based on the new findings, the present invention has been completed.

That is, the prevent invention provide the following embodiments.
[1] A compound of formula (I): 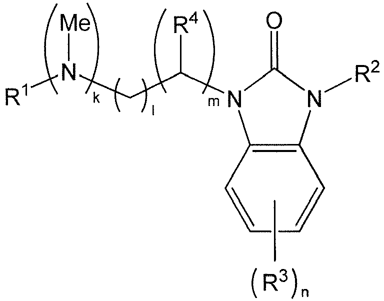 or a pharmaceutically acceptable salt thereof, wherein
   R¹ is C₁₋₁₀ alkyl, C(O)-C₁₋₁₀ alkyl, C₁₋₆ alkylene-R^{1a}, C(O)-C₁₋₆ alkylene-R^{1a}, C₁₋₆ alkylene-CHR^{1b}R^{1b} or C(O)-C₁₋₆ alkylene-CHR^{1b}R^{1b} wherein the alkyl and alkylene may be optionally substituted with the same or different one or more groups selected from the group consisting of halogen, amino and hydroxy at any replaceable positions;
   R² is hydrogen, C₁₋₁₀ alkyl, C(O)-C₁₋₁₀ alkyl, C₁₋₆ alkylene-R^{2a}, C(O)-C₁₋₆ alkylene-R^{2a}, C₁₋₆ alkylene-CHR^{2b}R^{2b} or C(O)-C₁₋₆ alkylene-CHR^{2b}R^{2b} wherein the alkyl and alkylene may be optionally substituted with the same or different one or more groups selected from the group consisting of halogen, amino and hydroxy at any replaceable positions;
   R^{1a} and R^{2a} are each independently selected from the group consisting of C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl and optionally-substituted 5- to 10-membered heteroaryloxy;
   R^{1b} and R^{2b} are each independently selected from the group consisting of optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl;
   R³ is each independently selected from the group consisting of halogen, C₁₋₆ alkyl, C₁₋₆ haloalkyl, amino and hydroxy;
   R⁴ is C₁₋₆ alkyl, C₁₋₆ haloalkyl, C₁₋₆ alkoxy, C₂₋₆ alkenyl or C₂₋₆ alkynyl;
   k is 0 or 1;
   1 is 0 to 2;
   m is 0 or 1, provided that when k is 1 and m is 1, the terminal carbon atom of R⁴ may be combined with the carbon atom bonded to N in the parenthesis for k to form a 4- to 6-membered nitrogen-containing heterocycle which may be optionally substituted with the same or different one or more groups selected from the group consisting of halogen, hydroxy, C₁₋₆ alkyl and C₁₋₆ alkoxy at any replaceable positions; and
   n is 0 to 4,
   provided that the following compounds are excluded:
   1-[1-[4,4-bis(4-fluorophenyl)butyl]-4-piperidinyl]-1,3-dihydro-2H-benzimidazol-2-one;
   1-(1-benzylpiperidin-4-yl)-1H-benzo[d]imidazol-2(3H)-one;
   1-(1-(2-phenylethyl)piperidin-4-yl)-1H-benzo[d]imidazol-2(3H)-one;
   1-(1-(3-phenylpropyl)piperidin-4-yl)-1H-benzo[d]imidazol-2(3H)-one;
   1-(1-(4-phenylbutyl)piperidin-4-yl)-1H-benzo[d]imidazol-2(3H)-one.
[2] The compound according to the item [1] or a pharmaceutically acceptable salt thereof, wherein the formula (I) is formula (Ia): 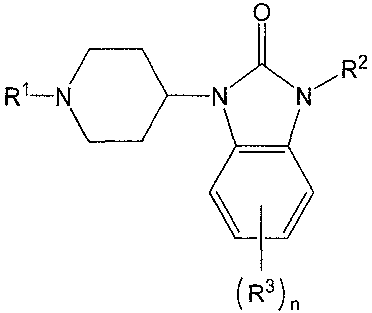 wherein R¹, R², R³ and n are as defined in the item [1].
[3] The compound according to the item [1] or a pharmaceutically acceptable salt thereof, wherein the formula (I) is formula (Ib): 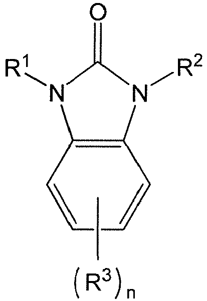 wherein R¹, R², R³ and n are as defined in the item [1].
[4] The compound according to any of the items [1] to [3] or a pharmaceutically acceptable salt thereof, wherein R¹ is C₁₋₆ alkyl, C(O)-C₁₋₆ alkyl, C₁₋₆ alkylene-R^{1a}, C(O)-C₁₋₆ alkylene-R^{1a}, C₁₋₄ alkylene-CHR^{1b}R^{1b} or C(O)-C₁₋₄ alkylene-CHR^{1b}R^{1b} wherein the alkyl and alkylene may be optionally substituted with the same or different one or more groups selected from the group consisting of halogen, amino and hydroxy at any replaceable positions;
   R² is C₁₋₆ alkyl, C(O)-C₁₋₆ alkyl, C₁₋₆ alkylene-R^{2a}, C(O)-C₁₋₆ alkylene-R^{2a}, C₁₋₄ alkylene-CHR^{2b}R^{2b} or C(O)-C₁₋₄ alkylene-CHR^{2b}R^{2b} wherein the C₁₋₆ alkyl and alkylene may be optionally substituted with the same or different one or more groups selected from the group consisting of halogen, amino and hydroxy at any replaceable positions;
   R^{1a} and R^{2a} are each independently selected from the group consisting of C₃₋₈ cycloalkyl, C₃₋₈ cycloalkoxy, C₆₋₁₀ aryl and C₆₋₁₀ aryloxy wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions;
   R^{1b} and R^{2b} are each independently selected from the group consisting of C₃₋₈ cycloalkyl and C₆₋₁₀ aryl wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions;
   R³ is each independently selected from the group consisting of halogen, amino and hydroxy; and
   n is 0 or 1.
[5] The compound according to any of the items [1] to [4] or a pharmaceutically acceptable salt thereof, wherein R¹ is C₁₋₆ alkyl, C₁₋₆ alkylene-R^{1a}, C(O)-C₁₋₆ alkylene-R^{1a} or C₁₋₄ alkylene-CHR^{1b}R^{1b} wherein the alkyl and alkylene may be optionally substituted with the same or different one or more groups selected from the group consisting of halogen, amino and hydroxy at any replaceable positions;
   R^{1a} is C₃₋₆ cycloalkyl, C₃₋₆ cycloalkyloxy, phenyl or phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; and
   R^{1b} is each independently selected from the group consisting of C₃₋₆ cycloalkyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions.
[6] The compound according to any of the items [1] to [5] or a pharmaceutically acceptable salt thereof, wherein R¹ is C₁₋₆ alkyl, C₁₋₆ alkylene-R^{1a}, C(O)-C₁₋₆ alkylene-R^{1a} or C₁₋₄ alkylene-CHR^{1b}R^{1b} wherein the alkyl and alkylene may be optionally substituted with the same or different one or more groups selected from the group consisting of halogen, amino and hydroxy at any replaceable positions;
   R^{1a} is selected from the group consisting of C₅₋₆ cycloalkyl, C₅₋₆ cycloalkyloxy, phenyl and phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; and
   R^{1b} is each independently selected from the group consisting of C₅₋₆ cycloalkyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions.
[7] The compound according to any of the items [1] to [6] or a pharmaceutically acceptable salt thereof, wherein R¹ is C₁₋₆ alkyl, C₁₋₆ alkylene-R^{1a}, C(O)-C₁₋₆ alkylene-R^{1a}, C₁₋₄ alkylene-CHR^{1b}R^{1b}; and
   R^{1a} is cyclohexyl, phenyl or phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; and
   R^{1b} is each independently selected from the group consisting of cyclohexyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions.
[8] The compound according to any of the items [1] to [7] or a pharmaceutically acceptable salt thereof, wherein R¹ is selected from the group consisting of C₁₋₅ alkyl, 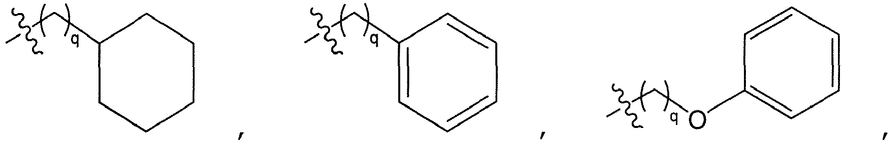 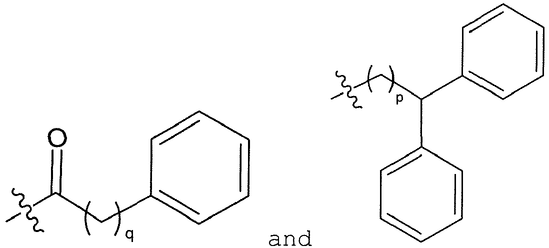 wherein p is 1 to 4 and q is 1 to 5 and wherein each of said groups may be optionally substituted with the same or different one or more groups selected from the group consisting of halogen and C₁₋₆ alkoxy at any replaceable positions.
[9] The compound according to any of the items [1] to [8] or a pharmaceutically acceptable salt thereof, wherein R¹ is selected from the group consisting of butyl, 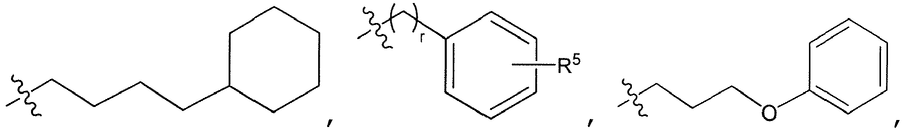 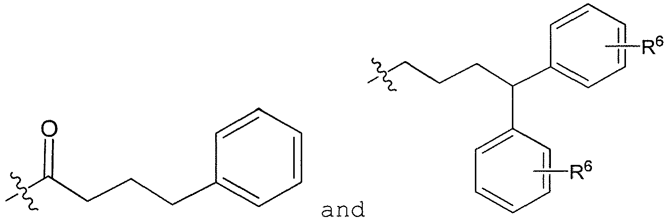 wherein R⁵ is hydrogen, halogen or methoxy, R⁶ is each independently hydrogen or halogen, and r is 1 to 5.
[10] The compound according to any of the items [1] to [9] or a pharmaceutically acceptable salt thereof, wherein R² is C₁₋₆ alkyl, C₁₋₆ alkylene-R^{2a}, C(O)-C₁₋₆ alkylene-R^{2a} or C₁₋₄ alkylene-CHR^{2b}R^{2b};
   R^{2a} is selected from the group consisting of C₃₋₆ cycloalkyl, C₃₋₆ cycloalkyloxy, phenyl and phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; and
   R^{2b} is each independently selected from the group consisting of C₃₋₆ cycloalkyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions.
[11] The compound according to any of the items [1] to [10] or a pharmaceutically acceptable salt thereof, wherein R² is C₁₋₆ alkyl, C₁₋₆ alkylene-R^{2a} or C₁₋₄ alkylene-CHR^{2b}R^{2b};
   R^{2a} is selected from the group consisting of C₅₋₆ cycloalkyl, C₅₋₆ cycloalkyloxy, phenyl and phenoxy wherein each of said groups may be optionally substituted with halogen or C₁₋₆ alkoxy at any replaceable positions; and
   R^{2b} is each independently selected from the group consisting of C₅₋₆ cycloalkyl and phenyl wherein each of said groups may be optionally substituted with halogen or C₁₋₆ alkoxy at any replaceable positions.
[12] The compound according to any of the items [1] to [11] or a pharmaceutically acceptable salt thereof, wherein R² is C₁₋₆ alkyl, C₁₋₆ alkylene-R^{2a} or C₁₋₄ alkylene-CHR^{2b}R^{2b};
   R^{2a} is cyclohexyl, phenyl or phenoxy wherein each of said groups may be optionally substituted with halogen or C₁₋₆ alkoxy at any replaceable positions; and
   R^{2b} is each independently selected from the group consisting of cyclohexyl and phenyl wherein each of said groups may be optionally substituted with halogen or C₁₋₆ alkoxy at any replaceable positions.
[13] The compound according to any of the items [1] to [12] or a pharmaceutically acceptable salt thereof, wherein R² is selected from the group consisting of C₁₋₅ alkyl, 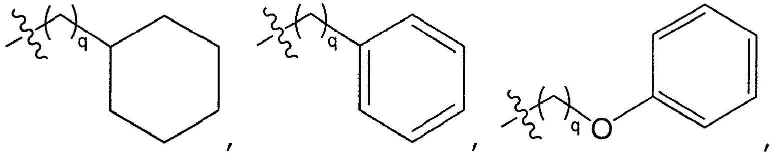 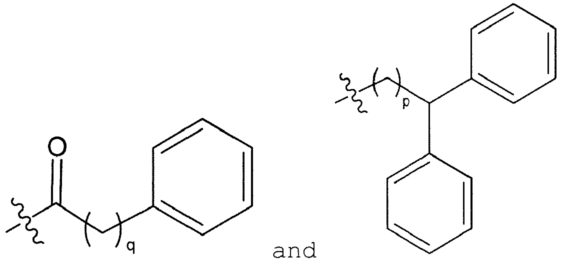 wherein p and q are as defied in the item [8] and wherein each of said groups may be optionally substituted with the same or different one or more groups selected from the group consisting of halogen and C₁₋₆ alkoxy at any replaceable positions.
[14] The compound according to any of the items [1] to [13] or a pharmaceutically acceptable salt thereof, wherein R² is butyl, 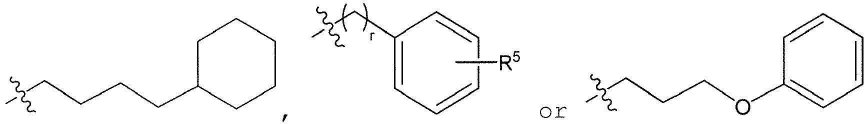 wherein R³ is hydrogen, halogen or methoxy, and r is 1 to 5.
[15] The compound according to the item [1] which selected from:
   1-(4-phenylbutyl)-3-(1-(4-phenylbutyl)piperidin-4-yl)-1H-benzo[d]imidazol-2(3H)-one (compound 5),
   1-butyl-3-(1-(4-phenylbutyl)piperidin-4-yl)-1H-benzo[d]imidazol-2(3H)-one (compound 6),
   1-(4-cyclohexylbutyl)-3-(1-(4-phenylbutyl)piperidin-4-yl)-1H-benzo[d]imidazol-2(3H)-one (compound 7), 1-(1-(4-cyclohexylbutyl)piperidin-4-yl)-1H-benzo[d]imidazol-2(3H)-one (compound 8),
   1-(4-cyclohexylbutyl)-3-(1-(4-cyclohexylbutyl)piperidin-4-yl)-1H-benzo[d]imidazol-2(3H)-one (compound 9),
   1,3-bis(4-phenylbutyl)-1H-benzo[d]imidazol-2(3H)-one (compound 10),
   1-(4-cyclohexylbutyl)-3-(4-phenylbutyl)-1H-benzo[d]imidazol-2(3H)-one (compound 11),
   1-(1-(4-cyclohexylbutyl)piperidin-4-yl)-3-(4-phenylbutyl)-1H-benzo[d]imidazol-2(3H)-one (compound 12),
   1-(1-butylpiperidin-4-yl)-3-(4-phenylbutyl)-1H-benzo[d]imidazol-2(3H)-one (compound 13),
   1-(4-phenylbutyl)-3-(1-(3-phenylpropyl)piperidin-4-yl)-1H-benzo[d]imidazol-2(3H)-one (compound 14),
   1-(1-(4-phenylbutanoyl)piperidin-4-yl)-3-(4-phenylbutyl)-1H-benzo[d]imidazol-2(3H)-one (compound 15),
   1-(4-phenylbutyl)-3-(1-(5-phenylpentyl)piperidin-4-yl)-1H-benzo[d]imidazol-2(3H)-one (compound 16),
   1-(1-(4-phenylbutyl)piperidin-4-yl)-3-(3-phenylpropyl)-1H-benzo[d]imidazol-2(3H)-one (compound 17),
   1-(1-(4-phenylbutyl)piperidin-4-yl)-3-(5-phenylpentyl)-1H-benzo[d]imidazol-2(3H)-one (compound 18),
   1-(1-benzylpiperidin-4-yl)-3-(4-phenylbutyl)-1H-benzo[d]imidazol-2(3H)-one (compound 19),
   1-benzyl-3-(1-(4-phenylbutyl)piperidin-4-yl)-1H-benzo[d]imidazol-2(3H)-one (compound 20),
   1-(1-(3-phenoxypropyl)piperidin-4-yl)-3-(4-phenylbutyl)-1H-benzo[d]imidazol-2(3H)-one (compound 21),
   1-(3-phenoxypropyl)-3-(1-(4-phenylbutyl)piperidin-4-yl)-1H-benzo[d]imidazol-2(3H)-one (compound 22),
   1-(3-phenoxypropyl)-3-(1-(3-phenoxypropyl)piperidin-4-yl)-1H-benzo[d]imidazol-2(3H)-one (compound 23),
   1-(1-(4-(2-methoxyphenyl)butyl)piperidin-4-yl)-3-(4-phenylbutyl)-1H-benzo[d]imidazol-2(3H)-one (compound 24),
   1-(4-(2-methoxyphenyl)butyl)-3-(1-(4-phenylbutyl)piperidin-4-yl)-1H-benzo[d]imidazol-2(3H)-one (compound 25),
   1-(4-(2-methoxyphenyl)butyl)-3-(1-(4-(2-methoxyphenyl)butyl)piperidin-4-yl)-1H-benzo[d]imidazol-2(3H)-one (compound 26),
   1-(1-(4-(2-chlorophenyl)butyl)piperidin-4-yl)-3-(4-phenylbutyl)-1H-benzo[d]imidazol-2(3H)-one (compound 27),
   1-(4-(2-chlorophenyl)butyl)-3-(1-(4-phenylbutyl)piperidin-4-yl)-1H-benzo[d]imidazol-2(3H)-one (compound 28),
   1-(4-(2-chlorophenyl)butyl)-3-(1-(4-(2-chlorophenyl)butyl)piperidin-4-yl)-1H-benzo[d]imidazol-2(3H)-one (compound 29),
   1-(1-(4-(2-fluorophenyl)butyl)piperidin-4-yl)-3-(4-phenylbutyl)-1H-benzo[d]imidazol-2(3H)-one (compound 30), 1-(4-(2-fluorophenyl)butyl)-3-(1-(4-phenylbutyl)piperidin-4-yl)-1H-benzo[d]imidazol-2(3H)-one (compound 31),
   1-(4-(2-fluorophenyl)butyl)-3-(1-(4-(2-fluorophenyl)butyl)piperidin-4-yl)-1H-benzo[d]imidazol-2(3H)-one (compound 32),
   1-(1-(4-(3-chlorophenyl)butyl)piperidin-4-yl)-3-(4-phenylbutyl)-1H-benzo[d]imidazol-2(3H)-one (compound 33),
   1-(4-(3-chlorophenyl)butyl)-3-(1-(4-phenylbutyl)piperidin-4-yl)-1H-benzo[d]imidazol-2(3H)-one (compound 34),
   1-(4-(3-chlorophenyl)butyl)-3-(1-(4-(3-chlorophenyl)butyl)piperidin-4-yl)-1H-benzo[d]imidazol-2(3H)-one (compound 35),
   1-(1-(4-(3-fluorophenyl)butyl)piperidin-4-yl)-3-(4-phenylbutyl)-1H-benzo[d]imidazol-2(3H)-one (compound 36),
   1-(4-(3-fluorophenyl)butyl)-3-(1-(4-phenylbutyl)piperidin-4-yl)-1H-benzo[d]imidazol-2(3H)-one (compound 37),
   1-(4-(3-fluorophenyl)butyl)-3-(1-(4-(3-fluorophenyl)butyl)piperidin-4-yl)-1H-benzo[d]imidazol-2(3H)-one (compound 38),
   1-(1-(4-(4-chlorophenyl)butyl)piperidin-4-yl)-3-(4-phenylbutyl)-1H-benzo[d]imidazol-2(3H)-one (compound 39),
   1-(4-(4-chlorophenyl)butyl)-3-(1-(4-phenylbutyl)piperidin-4-yl)-1H-benzo[d]imidazol-2(3H)-one (compound 40),
   1-(4-(4-chlorophenyl)butyl)-3-(1-(4-(4-chlorophenyl)butyl)piperidin-4-yl)-1H-benzo[d]imidazol-2(3H)-one (compound 41),
   1-(1-(4-(4-fluorophenyl)butyl)piperidin-4-yl)-3-(4-phenylbutyl)-1H-benzo[d]imidazol-2(3H)-one (compound 42),
   1-(4-(4-fluorophenyl)butyl)-3-(1-(4-phenylbutyl)piperidin-4-yl)-1H-benzo[d]imidazol-2(3H)-one (compound 43),
   1-(4-(4-fluorophenyl)butyl)-3-(1-(4-(4-fluorophenyl)butyl)piperidin-4-yl)-1H-benzo[d]imidazol-2(3H)-one (compound 44),
   5-fluoro-1-(4-phenylbutyl)-3-(1-(4-phenylbutyl)piperidin-4-yl)-1H-benzo[d]imidazol-2(3H)-one (compound 45),
   5-chloro-3-(4-phenylbutyl)-1-(1-(4-phenylbutyl)piperidin-4-yl)-1H-benzo[d]imidazol-2(3H)-one (compound 46),
   5-chloro-1-(4-phenylbutyl)-3-(1-(4-phenylbutyl)piperidin-4-yl)-1H-benzo[d]imidazol-2(3H)-one (compound 47),
   4-chloro-1-(4-phenylbutyl)-3-(1-(4-phenylbutyl)piperidin-4-yl)-1H-benzo[d]imidazol-2(3H)-one (compound 48),
   1-(1-(4,4-diphenylbutyl)piperidin-4-yl)-3-(4-phenylbutyl)-1H-benzo[d]imidazol-2(3H)-one(compound 49), or
   1-(1-(4,4-bis(4-fluorophenyl)butyl)piperidin-4-yl)-3-(4-phenylbutyl)-1H-benzo[d]imidazol-2(3H)-one (compound 50), or a pharmaceutically acceptable salt thereof.
[16] A pharmaceutical composition comprising a compound of formula (I): 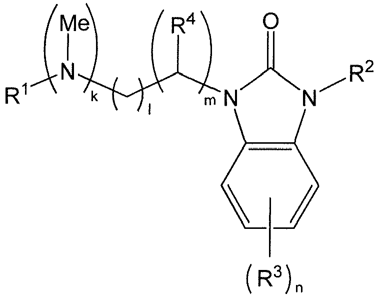 or a pharmaceutically acceptable salt thereof, wherein
   R¹ is C₁₋₁₀ alkyl, C(O)-C₁₋₁₀ alkyl, C₁₋₆ alkylene-R^{1a}, C(O)-C₁₋₆ alkylene-R^{1a}, C₁₋₆ alkylene-CHR^{1b}R^{1b} or C(O)-C₁₋₆ alkylene-CHR^{1b}R^{1b} wherein the alkyl and alkylene may be optionally substituted with the same or different one or more groups selected from the group consisting of halogen, amino and hydroxy at any replaceable positions;
   R² is hydrogen, C₁₋₁₀ alkyl, C(O)-C₁₋₁₀ alkyl, C₁₋₆ alkylene-R^{2a}, C(O)-C₁₋₆ alkylene-R^{2a}, C₁₋₆ alkylene-CHR^{2b}R^{2b} or C(O)-C₁₋₆ alkylene-CHR^{2b}R^{2b} wherein the alkyl and alkylene may be optionally substituted with the same or different one or more groups selected from the group consisting of halogen, amino and hydroxy at any replaceable positions;
   R^{1a} and R^{2a} are each independently selected from the group consisting of C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl and optionally-substituted 5- to 10-membered heteroaryloxy;
   R^{1b} and R^{2b} are each independently selected from the group consisting of optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl;
   R³ is each independently selected from the group consisting of halogen, C₁₋₆ alkyl, C₁₋₆ haloalkyl, amino and hydroxy;
   R⁴ is C₁₋₆ alkyl, C₁₋₆ haloalkyl, C₁₋₆ alkoxy, C₂₋₆ alkenyl or C₂₋₆ alkynyl;
   k is 0 or 1;
   1 is 0 to 2;
   m is 0 or 1, provided that when k is 1 and m is 1, the terminal carbon atom of R⁴ may be combined with the carbon atom bonded to N in the parenthesis for k to form a 4- to 6-membered nitrogen-containing heterocycle which may be optionally substituted with the same or different one or more groups selected from the group consisting of halogen, hydroxy, C₁₋₆ alkyl and C₁₋₆ alkoxy at any replaceable positions; and
   n is 0 to 4,
   provided that 1-[1-[4,4-bis(4-fluorophenyl)butyl]-4-piperidinyl]-1,3-dihydro-2H-benzimidazol-2-one is excluded, and a pharmaceutically acceptable carrier.
[17] The pharmaceutical composition according to the item [16] for treating a disease associated with the activation of T-type calcium channels.
[18] The pharmaceutical composition according to the item [17], wherein the disease is selected from pain, epilepsy, essential tremor, schizophrenia, Parkinson's disease, manic depression, bipolar disorder, depression, anxiety, dementia, Huntington's disease, sleep disorder, stroke, pruritus, atopic dermatitis, hyperaldosteronism, edema, ischemic heart disease, age-related macular degeneration, cancer, diabetes, sterility, sexual dysfunction, arrhythmia, hypertension, renal disease or overactive bladder.
[19] The pharmaceutical composition according to the item [17] or [18], wherein the disease is pain.
[20] The pharmaceutical composition according to the item [17] or [18], wherein the disease is pruritus.
[21] A T-type calcium channel inhibitor comprising a compound of formula (I): 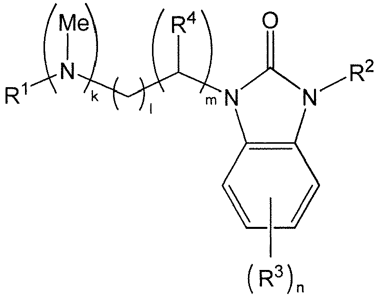 or a pharmaceutically acceptable salt thereof, wherein
   R¹ is C₁₋₁₀ alkyl, C(O)-C₁₋₁₀ alkyl, C₁₋₆ alkylene-R^{1a}, C(O)-C₁₋₆ alkylene-R^{1a}, C₁₋₆ alkylene-CHR^{1b}R^{1b} or C(O)-C₁₋₆ alkylene-CHR^{1b}R^{1b} wherein the alkyl and alkylene may be optionally substituted with the same or different one or more groups selected from the group consisting of halogen, amino and hydroxy at any replaceable positions;
   R² is hydrogen, C₁₋₁₀ alkyl, C(O)-C₁₋₁₀ alkyl, C₁₋₆ alkylene-R^{2a}, C(O)-C₁₋₆ alkylene-R^{2a}, C₁₋₆ alkylene-CHR^{2b}R^{2b} or C(O)-C₁₋₆ alkylene-CHR^{2b}R^{2b} wherein the alkyl and alkylene may be optionally substituted with the same or different one or more groups selected from the group consisting of halogen, amino and hydroxy at any replaceable positions;
   R^{1a} and R^{2a} are each independently selected from the group consisting of C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl and optionally-substituted 5- to 10-membered heteroaryloxy;
   R^{1b} and R^{2b} are each independently selected from the group consisting of optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl;
   R³ is each independently selected from the group consisting of halogen, C₁₋₆ alkyl, C₁₋₆ haloalkyl, amino and hydroxy;
   R⁴ is C₁₋₆ alkyl, C₁₋₆ haloalkyl, C₁₋₆ alkoxy, C₂₋₆ alkenyl or C₂₋₆ alkynyl;
   k is 0 or 1;
   1 is 0 to 2;
   m is 0 or 1, provided that when k is 1 and m is 1, the terminal carbon atom of R⁴ may be combined with the carbon atom bonded to N in the parenthesis for k to form a 4- to 6-membered nitrogen-containing heterocycle which may be optionally substituted with the same or different one or more groups selected from the group consisting of halogen, hydroxy, C₁₋₆ alkyl and C₁₋₆ alkoxy at any replaceable positions; and
   n is 0 to 4,
   provided that 1-[1-[4,4-bis(4-fluorophenyl)butyl]-4-piperidinyl]-1,3-dihydro-2H-benzimidazol-2-one is excluded.
   In addition, the present invention provides the following embodiments.
[22] A method of treating a disease associated with the activation of T-type calcium channels, which comprises administering a therapeutic effective amount of the compound according to any of the items [1] to [15] or a pharmaceutically acceptable salt thereof or the pharmaceutical composition according to the item [16] in a patient need thereof.
[23] The compound according to any of the items [1] to [15] or a pharmaceutically acceptable salt thereof for use in the treatment of a disease associated with the activation of T-type calcium channels.
[24] Use of the compound according to any of the items [1] to [15] or a pharmaceutically acceptable salt thereof in the manufacture of a medicament for treating a disease associated with the activation of T-type calcium channels.
[25] The method according to the item [22], the compound according to the item [23] or a pharmaceutically acceptable salt thereof or the use according to the item [24], wherein the disease is pain.
[26] The method according to the item [22], the compound according to the item [23] or a pharmaceutically acceptable salt thereof or the use according to the item [24], wherein the disease is pruritus.
[27] The pharmaceutical composition according to the item [18] or [19] or the method, the compound or a pharmaceutically acceptable salt thereof, or the use according to the item [25], wherein the pain is visceral pain or somatic pain.
[28] The pharmaceutical composition according to the item [18] or [19] or the method, the compound or a pharmaceutically acceptable salt thereof, or the use according to the item [25], wherein the pain is visceral pain, acute pain, chronic pain or neuropathic pain.
[29] The pharmaceutical composition according to the item [18] or [19] or the method, the compound or a pharmaceutically acceptable salt thereof, or the use according to the item [25], wherein the pain is acute pain or chronic pain.
[30] The pharmaceutical composition according to the item [18] or [19] or the method, the compound or a pharmaceutically acceptable salt thereof, or the use according to the item [25], wherein the pain is neuropathic pain.
[31] The pharmaceutical composition according to the item [18] or [19] or the method, the compound or a pharmaceutically acceptable salt thereof, or the use according to the item [25], wherein the pruritus is intractable pruritus.

### (EFFECTS OF THE INVENTION)

According to the present invention, a novel benzimidazolone compound with an excellent effect of inhibiting T-type calcium channels can be provided. Also, the present compound can be used as a new medicament for treating a disease associated with the activation of T-type calcium channels, for example, pain and pruritus, which can reduce the effect of inhibiting D₂ receptor and inhibit the risk of side effects.

### BRIEF DESCRIPTION OF THE DRAWINGS

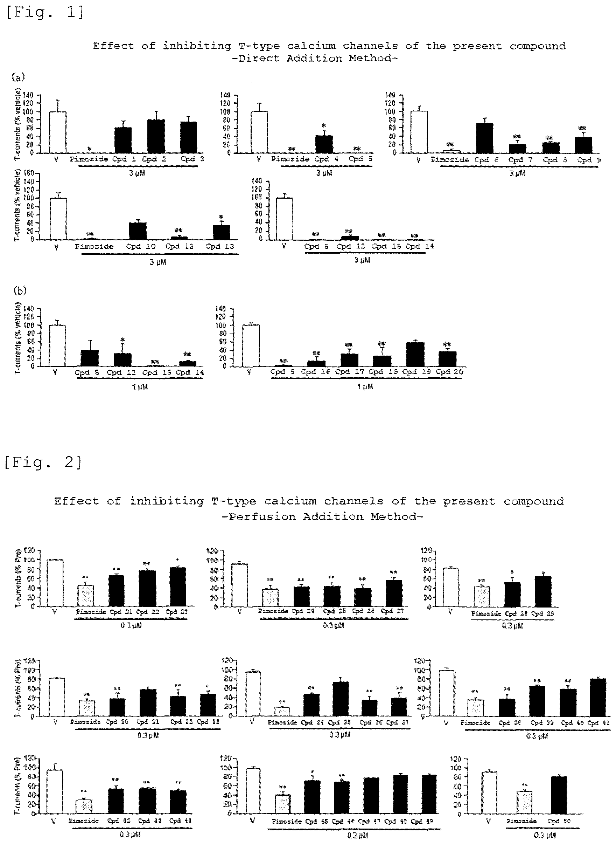

Fig. 1 shows the percentages (%) of the T channel current values after pimozide and the present compound (3 µM and 1 µM) were added relative to the T channel current value after vehicle (solvent, control group) was added (n = 3 to 10). (a) shows data obtained when the test compounds in a concentration of 3 µM were added directly, and (b) shows data obtained when the test compounds in a concentration of 1 µM were added directly.
Fig. 2 shows the percentage change in the T channel current values before the addition of vehicle and the present compound and after the addition of vehicle and each test compound (0.3 µM) by perfusion (n = 3 to 8).

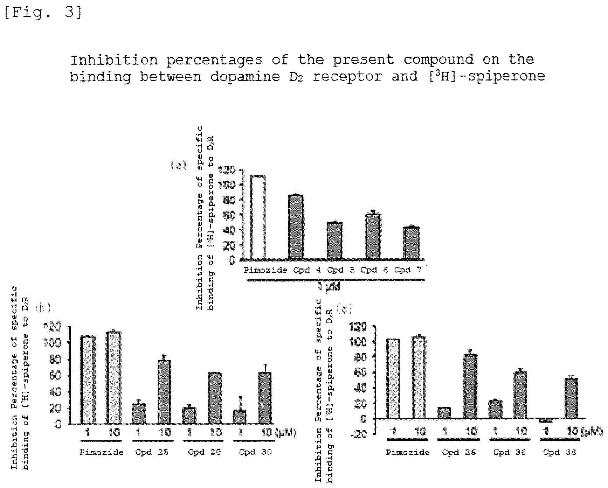

Fig. 3 shows the inhibition percentages of the present compound on the binding between dopamine D₂ receptor and [³H]-spiperone (n = 3). (a) shows the inhibition percentages of pimozide and compounds 4 to 7 in a concentration of 1µM on the specific binding of [³H]-spiperone to dopamine D₂ receptor, (b) shows the inhibition percentages of pimozide, compound 25, compound 28 and compound 30 in concentrations of 1 µM and 10 µM on the specific binding of [³H]-spiperone to dopamine D₂ receptor, and (c) shows the inhibition percentages of pimozide, compound 26, compound 36 and compound 38 in concentrations of 1 µM and 10 µM on the specific binding of [³H]-spiperone to dopamine D₂ receptor. The inhibition percentage of each compound is represented by the ratio to the inhibition percentage of pimozide when the ratio of pimozide is defined as 100%.

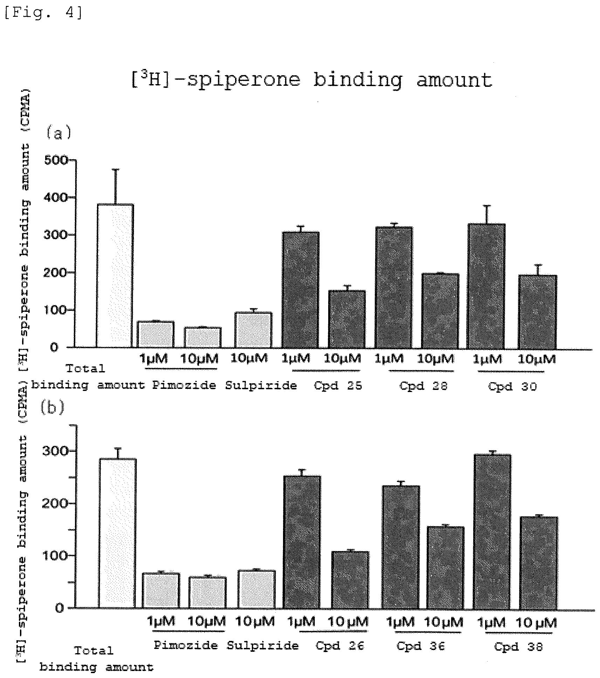

Fig. 4 shows the amounts of the specific binding of [³H]-spiperone to dopamine D₂ receptor after the addition of the solvent (total binding amount) and the addition of the test compound (1 µM and 10 µM) (n = 3). (a) shows the amount of the specific binding of [³H]-spiperone to dopamine D₂ receptor after the addition of the solvent (total binding amount), the amounts of the specific binding of [³H]-spiperone to dopamine D₂ receptor after the addition of pimozide, compound 25, compound 28 and compound 30 in concentrations of 1 µM and 10 µM, and the amount of the specific binding of [³H]-spiperone to dopamine D₂ receptor after the addition of sulpiride, and (b) shows the amount of the specific binding of [³H]-spiperone to dopamine D₂ receptor after the addition of the solvent (total binding amount), the amounts of the specific binding of [³H]-spiperone to dopamine D₂ receptor after the addition of pimozide, compound 26, compound 36 and compound 38 in concentrations of 1 µM and 10 µM, and the amount of the specific binding of [³H]-spiperone to dopamine D₂ receptor after the addition of sulpiride.

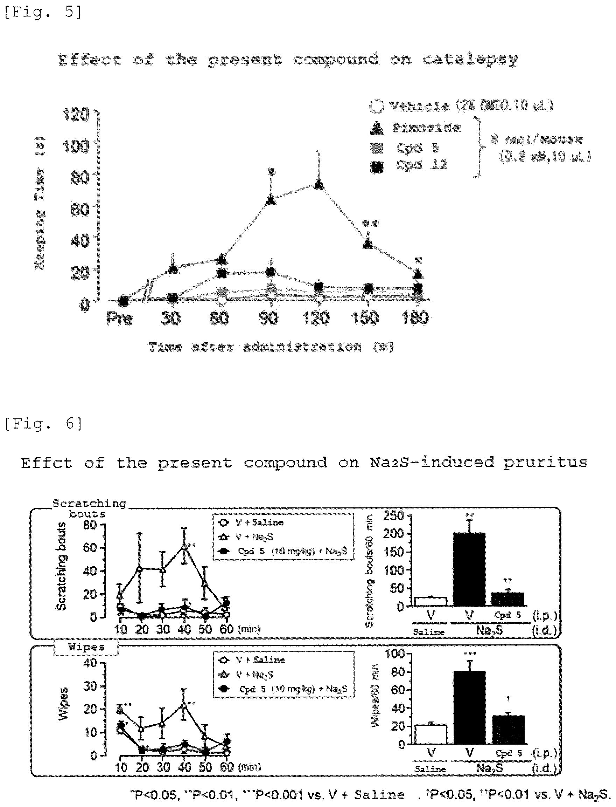

Fig. 5 shows the keeping time (s) of catalepsy 30, 60, 90, 120, 150 and 180 minutes after vehicle and the test compound were administered.
Fig. 6 shows the graphs of the mean value ± standard error of the counts of the scratching bouts and the wipes for 10, 20, 30, 40, 50 and 60 minutes after the intrabuccal administration on the right side as well as the mean value ± standard error of the total counts thereof for 60 minutes after the administration.

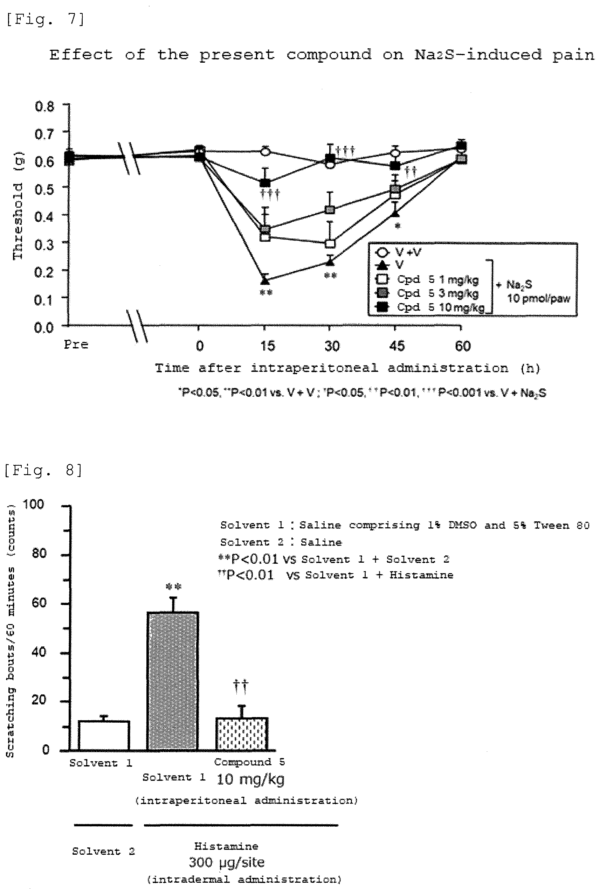

Fig. 7 shows the graph of the mean value ± standard error of the pain thresholds (g) before, immediately after, and 15 to 60 minutes after the intraplantar injection in the hind paws of the mice.
Fig. 8 shows the graph of the mean value ± standard error of the total counts of the scratching bouts by the hind paws to the administration site for 60 minutes after the intrabuccal administration on the right side of the mice (n = 6 to 7) in saline comprising 1% DMSO and 5% Tween 80 (Solvent 1) + saline (Solvent 2) administration group (control group), Solvent 1 + histamine administration group and compound 5 (10 mg/kg) + histamine administration group.

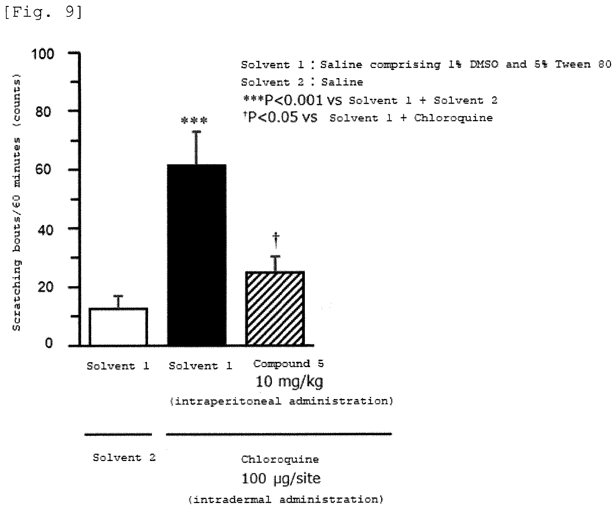

Fig. 9 shows the graph of the mean value ± standard error of the total counts of the scratching bouts by the hind paws to the administration site for 60 minutes after the intrabuccal administration on the right side of the mice (n = 10) in saline comprising 1% DMSO and 5% Tween 80 (Solvent 1) + saline (Solvent 2) administration group (control group), Solvent 1 + chloroquine administration group and compound 5 (10 mg/kg) + chloroquine administration group.

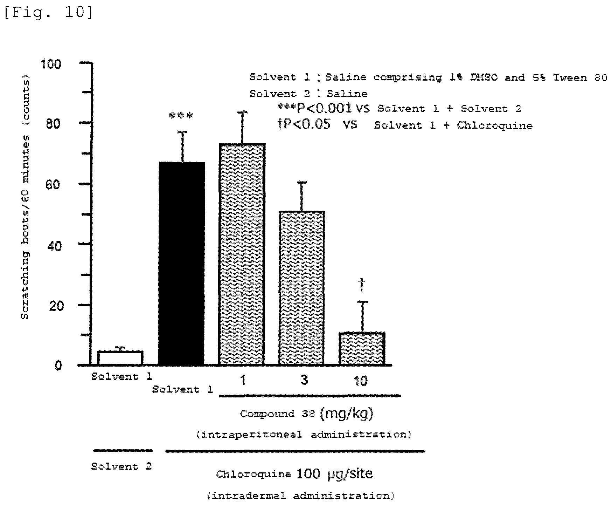

Fig. 10 shows the graph of the mean value ± standard error of the total counts of the scratching bouts by the hind paws to the administration site for 60 minutes after the intrabuccal administration on the right side of the mice (n = 6 to 8) in saline comprising 1% DMSO and 5% Tween 80 (Solvent 1) + saline (Solvent 2) administration group (control group), Solvent 1 + chloroquine administration group, compound 38 (3 mg/kg) + chloroquine administration group and compound 38 (10 mg/kg) + chloroquine administration group.

### DESCRIPTION OF EMBODIMENTS

Each term as used herein is explained below.

As used herein, the term "halogen" means fluorine, chlorine, bromine or iodine.

As used herein, the term "C₁₋₁₀ alkyl" means a linear or branched-chain saturated hydrocarbon group having 1 to 10 carbon atoms. The terms "C₁₋₆ alkyl" and "C₁₋₄ alkyl" mean alkyl having 1 to 6 carbon atoms and alkyl having 1 to 4 carbon atoms, respectively.

Examples of the "C₁₋₁₀ alkyl" include methyl, ethyl, propyl, isopropyl, butyl, isobutyl, sec-butyl, tert-butyl, pentyl, isopentyl, neopenthyl, hexyl, heptyl, octyl, nonyl, decyl, 1,1-dimethylbutyl, 2,2-dimethylbutyl, 3,3-dimethylbutyl, 2-ethylbutyl, and the like. They may be optionally substituted with one or more substituents of the present invention at any replaceable positions.

As used herein, the term "C₁₋₆ alkylene" means a bivalent linear or branched-chain saturated hydrocarbon group having 1 to 6 carbon atoms or a bivalent saturated hydrocarbon group with a cyclic structure having 3 to 6 carbon atoms. The term "C₁₋₄ alkylene" means alkylene having 1 to 4 carbon atoms.

Examples of the "C₁₋₆ alkylene" include methylene, ethylene, propylene, butylene, pentylene, hexylene, 1-methylmethylene, 1-ethylmethylene, 1-propylmethylene, 1-methylethylene, 2-methylethylene, 1-ethylethylene, and the like. They may be optionally substituted with one or more substituents of the present invention at any replaceable positions.

As used herein, the term "C₂₋₆ alkenyl" means a linear or branched-chain unsaturated hydrocarbon group having 2 to 6 carbon atoms which includes one or more carbon-carbon double bonds at an optional position. The "C₂₋₆ alkenyl" is preferably "C₂₋₄ alkenyl".

Examples of the "C₂₋₆ alkenyl" include ethenyl, propenyl, butenyl, pentenyl, hexenyl, and the like. They may be optionally substituted with one or more substituents of the present invention at any replaceable positions.

As used herein, the term "C₂₋₆ alkynyl" means a linear or branched-chain unsaturated hydrocarbon group having 2 to 6 carbon atoms which includes one or more carbon-carbon triple bonds at an optional position. The "C₂₋₆ alkynyl" is preferably "C₂₋₆ alkynyl".

Examples of the "C₂₋₆ alkynyl" include ethynyl, propynyl, butynyl, pentynyl, hexynyl, and the like. They may be optionally substituted with one or more substituents of the present invention at any replaceable positions.

As used herein, the term "C₁₋₆ haloalkyl" means the above-mentioned alkyl group having 1 to 6 carbon atoms in which one or more hydrogen atoms thereon are replaced with halogen atom(s). The number of replaceable hydrogen atoms can range from one up to the total number of hydrogen atoms in the parent alkyl group. When the group has two or more halogen atoms, the halogen atoms may be the same or different.

Examples of "C₁₋₆ haloalkyl" include fluoromethyl, chloromethyl, difluoromethyl, dichloromethyl, trifluoromethyl, trichloromethyl, 2,2,2-trifluoroethyl, and the like.

As used herein, the term "C₁₋₆ alkoxy" means a group wherein the above-mentioned alkyl group having 1 to 6 carbon atoms is linked through oxygen atom.

Examples of the "C₁₋₆ alkoxy" include methoxy, ethoxy, propoxy, isopropoxy, butyloxy, pentyloxy, isopentyloxy, neopentyloxy, hexyloxy, and the like. The alkyl moiety thereof may be optionally substituted with one or more substituents of the present invention at any replaceable positions.

As used herein, the term "C₃₋₈ cycloalkyl" means a mono- or poly-cyclic saturated or partially-unsaturated hydrocarbon group having 3 to 8 carbon atoms. The "C₃₋₈cycloalkyl" is preferably "C₃₋₆ cycloalkyl".

Examples of the "C₃₋₈ cycloalkyl" include cyclopropyl, cyclobutyl, cyclopentyl, cyclohexyl, cycloheptyl, cyclooctyl, adamantyl, norbornyl, cyclopropylene, cyclobutylene, and the like. They may be optionally substituted with one or more substituents of the present invention at any replaceable positions.

As used herein, the term "C₃₋₈ cycloalkoxy" means oxy group substituted with the above-mentioned C₃₋₈ cycloalkyl.

Examples of the "C₃₋₈ cycloalkoxy" include cyclopropoxy, cyclobutoxy, cyclopentyloxy, cyclohexyloxy, cyclohepthyloxy, and the like. They may be optionally substituted with one or more substituents of the present invention at any replaceable positions. The cycloalkyl moiety thereof may be optionally substituted with one or more substituents of the present invention at any replaceable positions.

As used herein, the term "3- to 10-membered heterocycloalkyl" means a 3- to 10-membered mono- or bi-cyclic non-aromatic heterocyclyl group having 1 or more (for example, 1 to 4) heteroatoms selected independently from the group consisting of nitrogen atom, oxygen atom and sulfur atom. Nitrogen and sulfur atoms may be optionally oxidized, and nitrogen atom may be optionally quaternized. The bicyclic heterocycloalkyl group includes a cyclic group in which a monocyclic heterocycloalkyl group and an aromatic ring (for example, benzene, pyridine) or a non-aromatic ring (for example, cyclohexyl, piperidine) are fused. The "3- to 10-membered heterocycloalkyl" is preferably 5 to 6-membered heterocycloalkyl.

Examples of the "3- to 10-membered heterocycloalkyl" include aziridinyl, azetidinyl, pyrrolidinyl, piperidinyl, piperazinyl, morpholinyl, homopiperidinyl, tetrahydrofuranyl, tetrahydropyranyl, and the like. They may be optionally substituted with one or more substituents of the present invention at any replaceable positions.

As used herein, the term "3- to 10-membered heterocycloalkoxy" means oxy group substituted with the above-mentioned 3- to 10-membered heterocycloalkyl.

Examples of the "3- to 10-membered heterocycloalkoxy" include aziridinyloxy, azetidinyloxy, pyrrolidinyloxy, piperidinyloxy, piperazinyloxy, morpholinyloxy, homopiperidinyloxy, tetrahydrofuranyloxy, tetrahydropyranyloxy, and the like. The heterocycloalkyl moiety thereof may be optionally substituted with one or more substituents of the present invention at any replaceable positions.

As used herein, the term "C₆₋₁₀ aryl" means a mono- or bi-cyclic aromatic hydrocarbon group having 6 to 10 carbon atoms. The "C₆₋₁₀ aryl" is preferably "C₆ aryl (phenyl)".

Examples of the "C₆₋₁₀ aryl" include phenyl, 1-naphthyl, 2-napthyl, and the like. They may be optionally substituted with one or more substituents of the present invention at any replaceable positions.

As used herein, the term "C₆₋₁₀ aryloxy" means oxy group substituted with the above-mentioned C₆₋₁₀ aryl. The "C₆₋₁₀ aryloxy" is preferably "C₆ aryloxy".

Examples of the "C₆₋₁₀ aryloxy" include phenoxy, 1-nepthyloxy, 2-napthyloxy, and the like. The aryl moiety thereof may be optionally substituted with one or more substituents of the present invention at any replaceable positions.

As used herein, the term "5- to 10-membered heteroaryl" means a 5- to 10-membered mono- or bi-cyclic aromatic heterocyclyl group having one or more (for example, 1 to 4) heteroatoms selected independently from the group consisting of nitrogen atom, oxygen atom and sulfur atom. The bicyclic heteroaryl group includes a cyclic group in which a monocyclic heteroaryl group and an aromatic ring (for example, benzene, pyridine) or a non-aromatic ring (for example, cyclohexyl, piperidine) are fused. The "5- to 10-membered heteroaryl" is preferably 5- to 6-membered heteroaryl.

Examples of the "5- to 10-membered heteroaryl" include pyridyl, pyridazinyl, isothiazolyl, pyrrolyl, furyl, thienyl, thiazolyl, imidazolyl, pyrimidinyl, thiadiazolyl, pyrazolyl, oxazolyl, isoxazolyl, pyrazinyl, triazinyl, triazolyl, imidazolidinyl, oxadiazolyl, tetrazolyl, indolyl, indazolyl, quinolyl, isoquinolyl, benzofuranyl, benzothienyl, benzoxazolyl, benzothiazolyl, benzisoxazolyl, benzisothiazolyl, benzotriazolyl, benzimidazolyl, and the like. They may be optionally substituted with one or more substituents of the present invention at any replaceable positions.

As used herein, the term "5- to 10-membered heteroaryloxy" means oxy group substituted with the above-mentioned 5- to 10-membered heteroaryl. The "5- to 10-membered heteroaryloxy" is preferably 5- or 6-membered heteroaryloxy.

Examples of the "5- to 10-membered heteroaryloxy" include pyridyloxy, pyridazinyloxy, pyrrolyloxy, furyloxy, thienyloxy, thiazolyloxy, pyrimidinyloxy, pyrazolyloxy, isoquinolyloxy, and the like. The heteroaryl moiety thereof may be optionally substituted with one or more substituents of the present invention at any replaceable positions.

As used herein, "4- to 6-membered nitrogen-containing heterocycle" means a saturated or partially-unsaturated 4-to 6-membered ring having one nitrogen atom. Examples of the "4- to 6-membered nitrogen-containing heterocycle" include azetidine, pyrrolidine, pyrroline, piperidine and piperideine. They may be optionally substituted with one or more substituents of the present invention at any replaceable positions.

As used herein, the term "optionally substituted" means a case in which the position is not substituted (non-substituted) and a case in which it is substituted at a position which can be substituted. The term "non-substituted" means that all of the replaceable positions in a group are hydrogen atoms. When a group is substituted, the group may be substituted with two or more substituents, if possible, and the substituents may be same or different.

As used herein, the substituent in "optionally-substituted C₃₋₈ cycloalkyl", "optionally-substituted C₃₋₈ cycloalkoxy", "optionally-substituted 3- to 10-membered heterocycloalkyl", "optionally-substituted 3- to 10-membered heterocycloalkoxy", "optionally-substituted C₆₋₁₀ aryl", "optionally-substituted C₆₋₁₀ aryloxy", "optionally-substituted 5- to 10-membered heteroaryl" and "optionally-substituted 5- to 10-membered heteroaryloxy" includes, for example, halogen, alkyl, alkenyl, alkynyl, cycloalkyl, heterocycloalkyl, haloalkyl, alkoxy, haloalkoxy, amino, nitro, cyano, hydroxy, mono- or di-alkylamino, carbamoyl, carboxyl, morpholinyl, formyl, acetyl, mesyl, benzoyl, acylamino, benzyl, aryl, heteroaryl, and the like. The substituent is preferably halogen, C₁₋₆ alkyl, C₁₋₆ alkoxy, C₁₋₆ haloalkyl, amino and hydroxy.

R¹, R², R³, R⁴, k, 1, m and n in the compound of the present invention represented by the formulae (I), (Ia) and (Ib) are preferably selected as follows, but the technical scope of the present invention is not limited to the scope of the compounds listed below.

R¹ includes
(1) C₁₋₁₀ alkyl;
(2) C(O)-C₁₋₁₀ alkyl;
(3) C₁₋₆ alkylene-R^{1a} wherein R^{1a} is C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl or optionally-substituted 5- to 10-membereed heteroaryloxy;
(4) C(O)-C₁₋₆ alkylene-R^{1a} wherein R^{1a} is C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl or optionally-substituted 5- to 10-membered heteroaryloxy;
(5) C₁₋₆ alkylene-CHR^{1b}R^{1b} wherein R^{1b} is each independently selected from the group consisting of optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl; and
(6) C(O)-C₁₋₆ alkylene-CHR^{1b}R^{1b} wherein R^{1b} is each independently selected from the group consisting of optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl wherein the alkyl and alkylene may be optionally substituted with the same or different one or more groups selected from the group consisting of halogen, amino and hydroxy at any replaceable positions.

R¹ is preferably,
(1) C₁₋₆ alkyl;
(2) C(O)-C₁₋₆ alkyl;
(3) C₁₋₆ alkylene-R^{1a} wherein R^{1a} is C₃₋₈ cycloalkyl, C₃₋₈ cycloalkoxy, 3- to 8-membered heterocycloalkyl, 3- to 8-membered heterocycloalkoxy, C₆₋₁₀ aryl, C₆₋₁₀ aryloxy, 5- to 10-membered heteroaryl or 5- to 10-membered heteroaryloxy wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions;
(4) C(O)-C₁₋₆ alkylene-R^{1a} wherein R^{1a} is C₃₋₈ cycloalkyl, C₃₋₈ cycloalkoxy, 3- to 8-membered heterocycloalkyl, 3- to 8-membered heterocycloalkoxy, C₆₋₁₀ aryl, C₆₋₁₀ aryloxy, 5- to 10-membered hetroaryl or 5- to 10-membered heteroaryloxy wherein the cycloalkyl, cycloalkoxy, heterocycloalkyl, heterocycloalkoxy, aryl, aryloxy, heteroaryl and heteroaryloxy may be optionally substituted with the same or different one or more groups selected from the group consisting of halogen, C₁₋₆ alkyl, C₁₋₆ alkoxy, C₁₋₆ haloalkyl, amino and hydroxy at any replaceable positions;
(5) C₁₋₄ alkylene-CHR^{1b}R^{1b} wherein R^{1b} is each independently selected from the group consisting of C₃₋₈ cycloalkyl, 3- to 8-membered heterocycloalkyl, C₆₋₁₀ aryl and 5- to 10-membered heteroaryl wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions; or
(6) C(O)-C₁₋₄ alkylene-CHR^{1b}R^{1b} wherein R^{1b} is each independently selected from the group consisting of C₃₋₈ cycloalkyl, 3- to 8-membered heterocycloalkyl, C₆₋₁₀ aryl and 5- to 10-membered heteroaryl wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions, more preferably,
   (1) C₁₋₆ alkyl;
   (2) C₁₋₆ alkylene-R^{1a} wherein R^{1a} is C₃₋₆ cycloalkyl, C₃₋₆ cycloalkyloxy, phenyl or phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions;
   (3) C(O)-C₁₋₆ alkylene-R^{1a} wherein R^{1a} is C₃₋₆ cycloalkyl, C₃₋₆ cycloalkyloxy, phenyl or phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; or
   (4) C₁₋₄ alkylene-CHR^{1b}R^{1b} wherein R^{1b} is each independently selected from the group consisting of C₃₋₆ cycloalkyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions, furthermore preferably,
      (1) C₁₋₆ alkyl;
      (2) C₁₋₆ alkylene-R^{1a} wherein R^{1a} is C₅₋₆ cycloalkyl, C₅₋₆ cycloalkyloxy, phenyl or phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions;
      (3) C(O)-C₁₋₆ alkylene-R^{1a} wherein R^{1a} is C₅₋₆ cycloalkyl, C₅₋₆ cycloalkyloxy, phenyl or phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; or
      (4) C₁₋₄ alkylene-CHR^{1b}R^{1b} wherein R^{1b} is each independently selected from the group consisting of C₅₋₆ cycloalkyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions, even more preferably,
         (1) C₁₋₆ alkyl;
         (2) C₁₋₆ alkylene-R^{1a} wherein R^{1a} is cyclohexyl, phenyl or phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions;
         (3) C(O)-C₁₋₆ alkylene-R^{1a} wherein R^{1a} is cyclohexyl, phenyl or phenoxy wherein each of said groups may be optionally substituted with the same or different one or more groups selected from the group consisting of halogen, C₁₋₆ alkyl, C₁₋₆ alkoxy and C₁₋₆haloalkyl at any replaceable positions; or
         (4) C₁₋₄ alkylene-CHR^{1b}R^{1b} wherein R^{1b} is each independently selected from the group consisting of cyclohexyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions, particularly preferably, a group selected from the group consisting of C₁₋₅ alkyl, 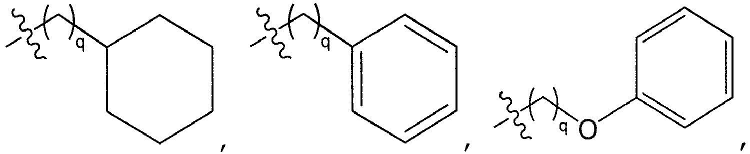 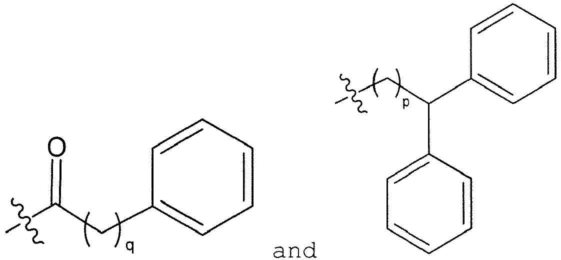 wherein p is 1 to 4 and q is 1 to 5 and wherein each of said groups may be optionally substituted with the same or different one or more groups selected from the group consisting of halogen and C₁₋₆ alkoxy at any replaceable positions, and most preferably, a group selected from the group consisting of butyl, 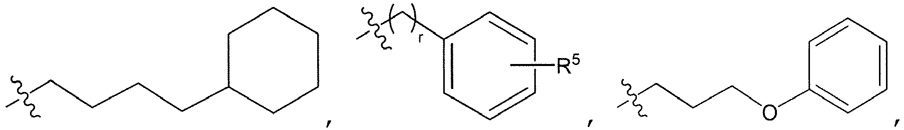 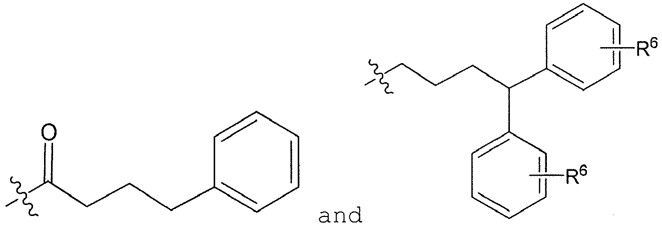 wherein R⁵ is hydrogen, halogen or methoxy, R⁶ is each independently hydrogen or halogen, and r is 1 to 5. The alkyl and alkylene in R¹ may be optionally substituted with the same or different one or more groups selected from the group consisting of halogen, amino and hydroxy at any replaceable positions.

R² includes
(1) hydrogen;
(2) C₁₋₁₀ alkyl;
(3) C (O) -C₁₋₁₀ alkyl;
(4) C₁₋₆ alkylene-R^{2a} wherein R^{2a} is C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl or optionally-substituted 5- to 10-membered heteroaryloxy;
(4) C(O)-C₁₋₆ alkylene-R^{2a} wherein R^{2a} is C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl or optionally-substituted 5- to 10-membered heteroaryloxy;
(5) C₁₋₆ alkylene-CHR^{2b}R^{2b} wherein R^{2b} is each independently selected from optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl; and
(6) C(O)-C₁₋₆ alkylene-CHR^{2b}R^{2b} wherein R^{2b} is each independently selected from the group consisting of optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl. The alkyl and alkylene in R² may be optionally substituted with the same or different one or more groups selected from the group consisting of halogen, amino and hydroxy at any replaceable positions.

R² is preferably,
(1) C₁₋₆ alkyl;
(2) C(O)-C₁₋₆ alkyl;
(3) C₁₋₆ alkylene-R^{2a} wherein R^{2a} is C₃₋₈ cycloalkyl, C₃₋₈ cycloalkoxy, 3- to 8-membered heterocycloalkyl, 3- to 8-membered heterocycloalkoxy, C₆₋₁₀ aryl, C₆₋₁₀ aryloxy, 5- to 10-membered heteroaryl or 5- to 10-membered heteroaryloxy wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions;
(4) C(O)-C₁₋₆ alkylene-R^{2a} wherein R^{2a} is C₃₋₈ cycloalkyl, C₃₋₈ cycloalkoxy, 3- to 8-membered heterocycloalkyl, 3- to 8-membered heterocycloalkoxy, C₆₋₁₀ aryl, C₆₋₁₀ aryloxy, 5- to 10-membered heteroaryl or 5- to 10-membered heteroaryloxy wherein the cycloalkyl, cycloalkoxy, heterocycloalkyl, heterocycloalkoxy, aryl, aryloxy, heteroaryl and heteroaryloxy may be optionally substituted with the same or different one or more groups selected from the group consisting of halogen, C₁₋₆ alkyl, C₁₋₆ alkoxy, C₁₋₆ haloalkyl, amino and hydroxy at any replaceable positions;
(5) C₁₋₄ alkylene-CHR^{2b}R^{2b} wherein R^{2b} is each independently selected from the group consisting of C₃₋₈ cycloalkyl, 3- to 8-membered heterocycloalkyl, C₆₋₁₀ aryl and 5- to 10-membered heteroaryl wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions; or
(6) C(O)-C₁₋₄ alkylene-CHR^{2b}R^{2b} wherein R^{2b} is each independently selected from the group consisting of C₃₋₈ cycloalkyl, 3- to 8-membered heterocycloalkyl, C₆₋₁₀ aryl and 5- to 10-membered heteroaryl wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions, more preferably,
   (1) C₁₋₆ alkyl;
   (2) C₁₋₆ alkylene-R^{2a} wherein R^{2a} is selected from the group consisting of C₃₋₆ cycloalkyl, C₃₋₆ cycloalkyloxy, phenyl and phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions;
   (3) C(O)-C₁₋₆ alkylene-R^{2a} wherein R^{2a} is selected from the group consisting of C₃₋₆ cycloalkyl, C₃₋₆ cycloalkyloxy, phenyl and phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; or
   (4) C₁₋₄ alkylene-CHR^{2b}R^{2b} wherein R^{2b} is each independently selected from the group consisting of C₃₋₆ cycloalkyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions, furthermore preferably,
      (1) C₁₋₆ alkyl;
      (2) C₁₋₆ alkylene-R^{2a} wherein R^{2a} is selected from the group consisting of C₅₋₆cycloalkyl, C₅₋₆ cycloalkyloxy, phenyl and phenoxy wherein each of said groups may be optionally substituted with halogen or C₁₋₆ alkoxy at any replaceable positions; or
      (3) C₁₋₄ alkylene-CHR^{2b}R^{2b} wherein R^{2b} is each independently selected from the group consisting of C₅₋₆ cycloalkyl and phenyl wherein each of said groups may be optionally substituted with halogen or C₁₋₆ alkoxy at any replaceable positions, even more preferably,
         (1) C₁₋₆ alkyl;
         (2) C₁₋₆ alkylene-R^{2a} wherein R^{2a} is cyclohexyl, phenyl or phenoxy wherein each of said groups may be optionally substituted with halogen or C₁₋₆alkoxy at any replaceable positions; or
         (3) C₁₋₄ alkylene-CHR^{2b}R^{2b} wherein R^{2b} is each independently selected from the group consisting of cyclohexyl and phenyl wherein each of said groups may be optionally substituted with halogen or C₁₋₆ alkoxy at any replaceable positions, particularly preferably, a group selected from the group consisting of C₁₋₅ alkyl, 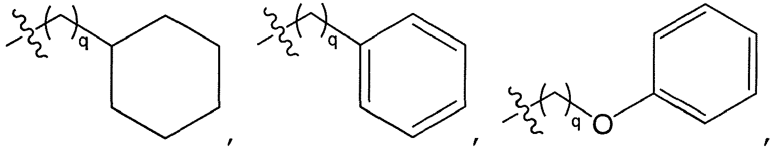 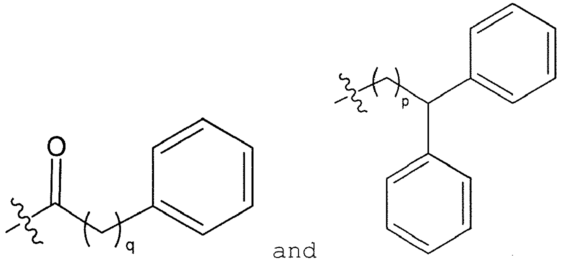 wherein p and q are as defined in the above R¹ and wherein each of said groups may be optionally substituted with the same or different one or more groups selected from the group consisting of halogen and C₁₋₆ alkoxy at any replaceable positions, most preferably, butyl, 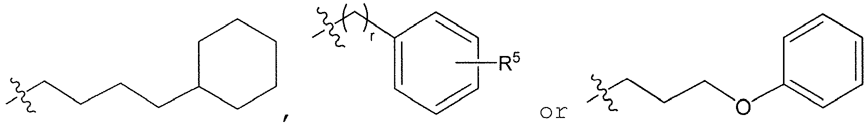 wherein R⁵ is hydrogen, halogen or methoxy, and r is 1 to 5.

R³ includes halogen, C₁₋₆ alkyl, C₁₋₆ haloalkyl, amino and hydroxy. Among them, it is preferably halogen, more preferably fluoro and chloro.

R⁴ includes C₁₋₆ alkyl, C₁₋₆ haloalkyl, C₁₋₆ alkoxy, C₂₋₆ alkenyl and C₂₋₆ alkynyl. Among them, it is preferably C₁₋₆ alkyl, more preferably methyl.

k is 0 or 1, l is 0 to 2, preferably 0 or 2, m is 0or 1, and n is 0 to 4, preferably 0 or 1. When k is 1 and m is 1, the terminal carbon atom of R⁴ may be combined with the carbon atom bonded to N in the parenthesis for k to form a 4- to 6-membered nitrogen-containing heterocycle which may be optionally substituted with the same or different one or more groups selected from the group consisting of halogen, hydroxy, C₁₋₆ alkyl and C₁₋₆ alkoxy at any replaceable positions. Preferably, when k is 1, l is 2 and R⁴ is methyl, the carbon atom of R⁴ is combined with the carbon atom bonded to N in the parenthesis for k to form piperidine ring. Also, it is preferable that k, l and m are 0, and N atom in 1,3-dihydro-2H-benzimidazol-2-one structure and R¹ are directly bonded.

As used herein, the term "pharmaceutically acceptable salt" includes, for example, a salt with an inorganic acid, a salt with an organic acid. Examples of the salt with an inorganic acid include inorganic acid salts such as hydrochloride, hydrobromide, hydroiodide, nitrate, sulfate and phosphate. Examples of the salt with an organic acid include organic acid salts such as acetate, oxalate, fumarate, maleate, succinate, malate, citrate, tartrate, adipate, gluconate, glucoheptonate, glucuronate, terephthalate, methanesulfonate, alanine, lactate, hippurate, 1,2-ethanedisulfonate, isethionate, lactobionate, oleate, gallate, pamoate, polygalacturonate, stearate, tannate, trifluoromethanesulfonate, benzenesulfonate, p-toluenesulfonate, naphthalenesulfonate and sulfosalicylate.

The compound of the present invention may be present in the form of a hydrate and/or solvate. The hydrate or the solvate such as ethanol solvate thereof is also included in the compound of the present invention. In addition, the compound of the present invention encompasses all embodiments of crystalline forms.

The compound of the present invention may be present in the form of a tautomer. The tautomer thereof is also included in the compound of the present invention.

The compound of the present invention may contain one or more asymmetric carbon atoms. The compound of the present invention encompasses not only racemic forms of the compounds of formulae (I), (Ia) and (Ib) but also optically-active forms of the compounds. When the compounds of formulae (I), (Ia) and (Ib) contain two or more asymmetric carbon atoms, the compounds can result in various stereoisomerisms. Thus, the compound of the present invention also encompasses the stereoisomer of the compounds and a mixture or isolate thereof.

Also, the compound of the present invention encompasses compounds wherein one or more of ¹H in the compounds of formulae (I), (Ia) and (Ib) are replaced with ²H(D) (i.e. deuterated form).

Hereinafter, the processes of preparing the compound of the present invention are exemplified along with examples, but the processes of the present invention should not be limited to the examples.

Each compound used as a starting compound may be used in the salt form. The shown processes are just examples to prepare the compounds and may be optionally modified by those skilled in the organic synthesis field.

In each Preparation Process described below, protecting groups can be used as necessary, even if the use of protecting groups is not explicitly stated. And, the protecting groups can be deprotected after a reaction is completed or a series of reactions have been carried out to obtain the desired compound.

As such protecting groups, for example, general protecting groups described in T. W. Greene, and P. G. M. Wuts, "Protective Groups in Organic Synthesis", 3rd Ed., John Wiley & Sons, Inc., New York (1999*),* and the like may be used. Examples of amino-protecting group include benzyloxycarbonyl, tert-butoxycarbonyl, acetyl, benzyl, and the like, and examples of hydroxy-protecting group include trialkylsilyl, acetyl, benzyl, and the like.

The introduction and elimination of protecting groups can be carried out by a method commonly used in synthetic organic chemistry (for example, *see* T. W. Greene, and P. G. M. Wuts, "Protective Groups in Organic Synthesis", 3rd Ed., John Wiley & Sons, Inc., New York (1999*)),* or a similar method.

### Preparation Process 1

Among the compounds of formula (I), the compounds of formulae (I-a) and (I-b) can be prepared according to, for example, the following preparation process.

The staring compound, *tert*-butyl 4-(2-oxo-2,3-dihydro-1H-benzo[d]imidazol-1-yl)piperidine-1-carboxylate (1) can be prepared according to a known process, for example, the method described in J. Med. Chem., 2018, 61, 8895-8907. 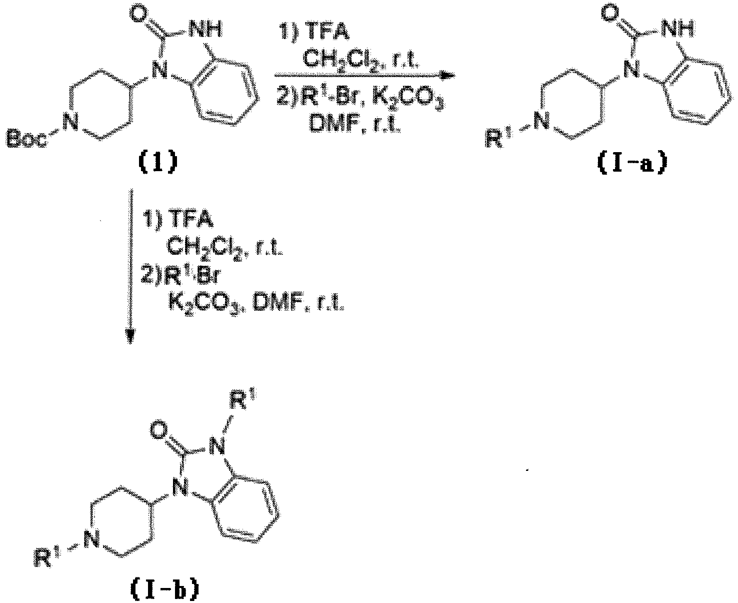 wherein R¹ is as defined in the above item [1].

### Preparation Process 2

Among the compounds of formula (I), the compound of formula (I-c) can be prepared according to, for example, the following preparation process: 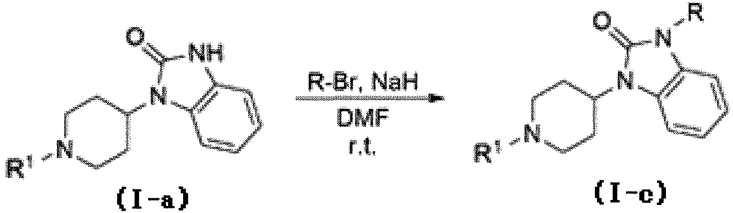 wherein R¹ is as defined in the above item [1] and R is as defined in R¹ of the above item [1].

### Preparation Process 3

Among the compounds of formula (I), the compounds of formulae (I-d) and (I-e) can be prepared according to, for example, the following preparation process.

The starting compound, o-phenylenediamine may be a commercially available product or be prepared according to a known process. 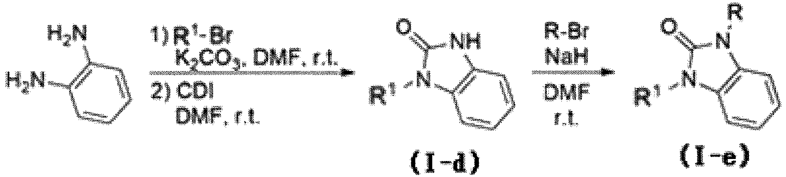 wherein R¹ is as defined in the above item [1] and R is as defined in R¹ of the above item [1].

### Preparation Process 4

Among the compounds of formula (I), the compounds of formulae (I-f) and (I-g) can be prepared according to, for example, the following preparation process: 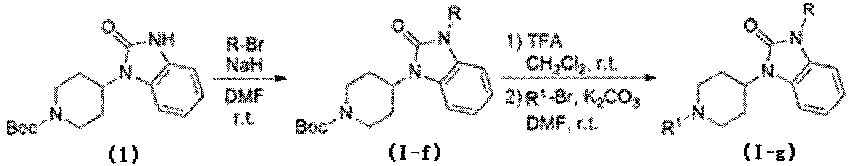 wherein R¹ is as defined in the above item [1] and R is as defined in R¹ of the above item [1].

### Preparation Process 5

Among the compounds of formula (I), the compound of formula (I-h) can be prepared according to, for example, the following preparation process: 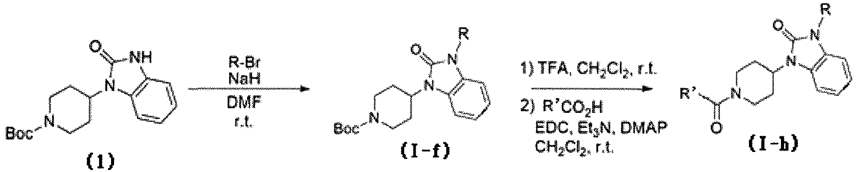 wherein R is as defined in R¹ of the above item [1], R' is C₁₋₆ alkylene-R^{1a} or C₁₋₆ alkylene-CHR^{1b}R^{1b} wherein the alkylene may be optionally substituted with the same or different one or more groups selected from the group consisting of halogen, amino and hydroxy at any replaceable positions, and R^{1a} is selected from the group consisting of C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl and optionally-substituted 5-to 10-membered heteroaryloxy, R^{1b} is each independently selected from optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl.

### Preparation Process 6

Among the compounds of formula (I), the compound of formulae (I-j) and (I-k) can be prepared according to, for example, the following preparation process. The compound of formula (I-i) can be prepared according to a known process. 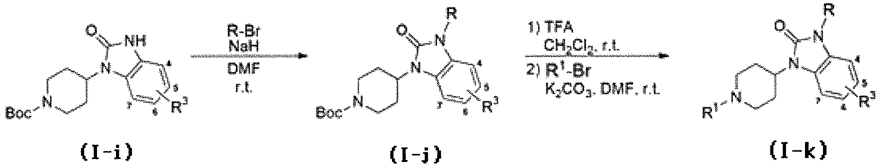 wherein R¹ and R³ are as defined in the item [1] and R is as defined in R¹ of the above item [1].

As appropriate, the solvent, acid and base to be used in the above preparation processes should be selected according to various factors such as the type of starting compound. Also, the conditions such as reaction temperature in the above preparation processes should be selected as appropriate.

The compound of the present invention or an intermediate thereof can be isolated and purified by the methods well known in the art. Examples of the methods include extraction, distribution, reprecipitation, column chromatography (for example, silica gel column chromatography, ion-exchange column chromatography, or preparative liquid chromatography), and recrystallization. The solvents used for recrystallization may include, for example, alcohol-based solvents such as methanol, ethanol and 2-propanol; ether-based solvents such as diethyl ether; ester-based solvents such as ethyl acetate; aromatic hydrocarbon-based solvents such as benzene and toluene; ketone-based solvents such as acetone; halogen-based solvents such as dichloromethane and chloroform; hydrocarbon-based solvents such as hexane; non-protic solvents such as dimethylformamide and acetonitrile; water; or a mixed solvent selected from two or more of the above solvents. Other purification methods, for example, those disclosed in Experimental Chemistry Textbook Vol. 1 (the Chemical Society of Japan, ed., Maruzen) can also be used herein.

As used herein, the term "disease associated with the activation of T-type calcium channels" means a disease caused due to the activation of T-type calcium channels or the associated symptom. Examples thereof include pain, epilepsy, essential tremor, schizophrenia, Parkinson's disease, manic depression, bipolar disorder, depression, anxiety, dementia, Huntington's disease, sleep disorder, stroke, pruritus, atopic dermatitis, hyperaldosteronism, edema, ischemic heart disease, age-related macular degeneration, cancer, diabetes, sterility, sexual dysfunction, arrhythmia, hypertension, renal disease and overactive bladder, but are not limited thereto. In the present invention, the disease is preferably pain and pruritus, more preferably visceral pain, somatic pain and pruritus, furthermore preferably visceral pain, acute pain, chronic pain, neuropathic pain. The pain is particularly preferably neuropathic pain.

As used herein, the term "treatment (treating)" means all types of treatment (for example, improvement, alleviation, suppression of progression) of a disease and the associated symptom in mammals, particularly in humans. Also, the term includes inhibiting the development and/or progression of a disease.

As used herein, the term "patient" means humans and animals such as dogs, cats and horses. Among them, human is preferred.

As used herein, the term "therapeutically effective amount" means an amount for treating a disease and the associated symptom or an amount for slowing progression of a disease and the associated symptom, relative to untreated subjects. The term also means an effective amount for promoting a normal physiological function.

The effective amount encompasses an amount of the present compound alone, an amount of a combination of the present compounds and/or an amount of the present compound in combination with another active ingredient useful for the treatment of a disease.

The compound of the present invention can be used in the form of pharmaceutical composition. The pharmaceutical composition of the present invention may comprise a pharmaceutically acceptable carrier.

The compound of the present invention has the effect of inhibiting T-type calcium channels. Thus, the pharmaceutical composition of the present invention can be used as a novel medicament of treating a disease such as pain (for example, visceral pain, somatic pain), epilepsy, essential tremor, schizophrenia, Parkinson's disease, manic depression, bipolar disorder, depression, anxiety, dementia, Huntington's disease, sleep disorder, stroke, pruritus (for example, intractable pruritus caused by chronic kidney disease, chronic liver disease), atopic dermatitis, hyperaldosteronism, edema, ischemic heart disease, age-related macular degeneration, cancer, diabetes, sterility, sexual dysfunction, arrhythmia, hypertension, renal disease, overactive bladder. The pharmaceutical composition of the present invention can be used as preferably a medicament for treating visceral pain, somatic pain and pruritus, more preferably a medicament for treating visceral pain, acute pain, chronic pain, neuropathic pain and pruritus, furthermore preferably a medicament for treating neuropathic pain and a medicament for treating pruritus.

The compound and pharmaceutical composition of the present invention may be administered as a preparation in an appropriate dosage form by oral administration or parenteral administration.

The dosage form of the compound and the pharmaceutical composition of the present invention includes, for example, tablets (for example, orally disintegrating tablets, chewable tablets, effervescent tablets, dispersible tablets, soluble tablets), capsules, granules (for example, effervescent granules), powders, liquids and solutions for oral administration (for example, elixirs, suspensions, emulsions, lemonades), syrups (for example, preparations for syrup), jellies for oral administration, tablets for oral cavity application (for example, troches/lozenges, sublingual tablets, buccal tablets, mucoadhesive tablets, medicated chewing gums), sprays for oral cavity application, semi-solid preparations for oral cavity application, preparations for gargles, injections (for example, parenteral infusions, implants/pellets, sustained release injections), dialysis agents (for example, peritoneal dialysis agents and hemodialysis agents), inhalations (for example, dry powder inhalers, inhalation solutions, inhalation aerosolized agents), suppositories, semi-solid preparations for rectal application, enema agents, ophthalmic preparations, ophthalmic ointment, ear drops, nasal preparations (for example, nasal dry powder inhalers and nasal solutions), tablets for vaginal use, suppositories for vaginal use, solid dosage forms for cutaneous application (for example, powders for cutaneous application), liquids and solutions for cutaneous application (for example, liniments and lotions), sprays for cutaneous application (for example, aerosols for cutaneous application and pump sprays for cutaneous application), ointments, creams, gels, patches (for example, tapes/plasters and cataplasms/gel patches), but are not limited thereto. The preparation of the present invention can be prepared by a known method by using the compound of the present invention and a pharmaceutically acceptable additive.

Examples of the pharmaceutically acceptable additive (carrier) include a stabilizing agent, a surfactant, a solubilizer, a buffer, a suspending agent, an antioxidant agent, a coating agent, a moistening agent, a moisture regulator, an algefacient, a coloring agent, a flavor, an isotonicity agent, an emulsifier, a pH adjuster, a skin protectant, a dispersant, a propellant, a fragrance, an antiseptic agent, and a solvent. The additive (carrier) can be used for the intended use.

The compound of the present invention may be used in combination with another drug as long as the drug does not prevent the therapeutic effect of the compound of the present invention against a disease, for example, pain and pruritus.

The dosage varies according to the type of compounds or various factors such as disease, age, body weight, sex, symptom of patients and administration route. The compound of the present invention is typically administrated to an adult (body wight: 60 kg) at a dose of 0.1 to 1000 mg/day, preferably 0.1 to 300 mg/day, which is administered once a day or in 2 or 3 divided doses. Also, the compound of the present invention may be administered once every few days to every few weeks.

### EXAMPLES

Hereinafter, the present invention is illustrated in more detail with Reference Examples, Examples and Test Examples, but the present invention should not be limited thereto. The names of compounds used in the following Reference Examples and Examples do not necessarily follow the IUPAC nomenclature, and the identification of each compound was performed by NMR spectroscopy.

The following abbreviations may be used in Examples for the sake of simplicity. The signs used in NMR are follows: s means singlet, d means is doublet, dd is doublet of doublet, ddd means doublet of doublet of doublet, t means triplet, td means triplet of doublet, tt means triplet of triplet, q means quartet, qd means quartet of doublet, quin means quintet, sext means sextet, m means multiplet, br means broad, brs means broad singlet, and J means coupling constant.

### Reference Example 1

### 1-(4-Phenylbutyl)-1H-benzo[d]imidazol-2(3H)-one (2)

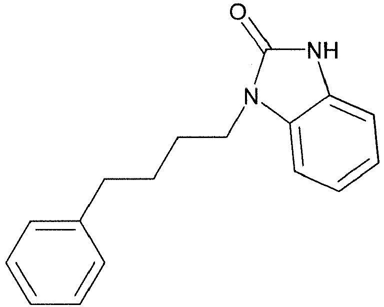

To a solution of o-phenylenediamine (200 mg, 1.85 mmol) in N,N-dimethylformamide (5 mL) were added 4-phenylbutyl bromide (432 mg, 2.04 mmol) and then potassium carbonate (141 mg, 2.04 mmol) under Ar atmosphere, and the mixture was stirred at room temperature for 16 hours. After the reaction was completed, the reaction solution was diluted with diethyl ether (1.5 mL) and water (1.5 mL) was added thereto. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then N,N-dimethylformamide (5 mL) was added to the resulting residue. To the mixture was then added carbonyldiimidazole (600 mg, 0.37 mmol) at room temperature and the mixture was stirred at room temperature for 15 hours. After the reaction is completed, the solvent in the mixture was removed using a rotary evaporator, and then the resulting residue was purified by silica gel column chromatography (n-hexane: ethyl acetate = 1:1) to give the title compound (163 mg, 33% 2 steps, 0.61 mmol).

¹H-NMR (400 MHz, CDCl₃) δ: 1.74 (2H, quin, J = 7.2 Hz), 1.83 (2H, quin, J = 7.2 Hz), 2.68 (2H, t, J = 7.2 Hz), 3.91 (2H, t, J = 7.2 Hz), 6.96-6.97 (1H, m), 7.06-7.11 (3H, m), 7.16-7.20 (3H, m), 7.27-7.29 (2H, m), 8.73 (1H, br).

### Reference Example 2

### tert-Butyl 4-(2-oxo-3-(4-phenylbutyl)-2,3-dihydro-1H-benzo[d]imidazol-1-yl)piperidine-1-carboxylate (3)

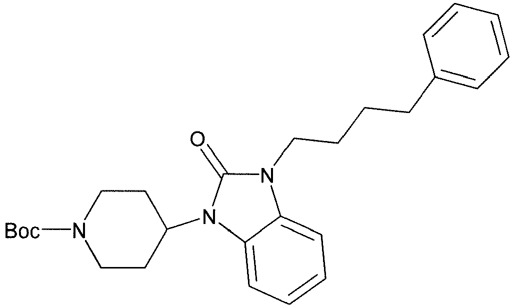

To a solution of tert-butyl 4-(2-oxo-2,3-dihydro-1H-benzo[d]imidazol-1-yl)piperidine-1-carboxylate (1) (98 mg, 0.31 mmol) in N,N-dimethylformamide (1.5 mL) was added sodium hydride (11 mg, 60%, 0.46 mmol) under Ar atmosphere and ice temperature, and the mixture was stirred at room temperature for 30 minutes. To the mixture was then added 4-phenylbutyl bromide (78 mg, 0.37 mmol) under ice temperature, and the mixture was stirred at room temperature for 1 hour. After the reaction was completed, the reaction solution was diluted with diethyl ether (1.5 mL) and water (1.5 mL) was added thereto. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 2:1) to give the title compound (113 mg, 82%, 0.25 mmol).

¹H-NMR (400 MHz, CDCl₃) δ: 1.50 (9H, s), 1.66-1.82 (6H, m), 2.31 (2H, qd, J = 12.8, 4.4 Hz), 2.66 (2H, t, J = 8.0 Hz), 2.86 (2H, br), 3.88 (2H, t, *J* = 8.0 Hz), 4.31 (2H, br), 4.49 (1H, tt, *J* = 12.8, 4.4 Hz), 6.95-6.97 (1H, m), 7.05 (2H, ddd, *J* = 6.8, 4.4, 2.0 Hz), 7.11-7.19 (4H, m), 7.25-7.28 (2H, m).

### Reference Example 3

### tert-Butyl 4-(2-oxo-3-(3-phenylpropyl)-2,3-dihydro-1H-benzo[d]imidazol-1-yl)piperidine-1-carboxylate (4)

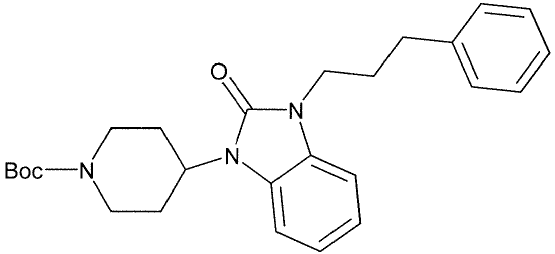

To a solution of *tert*-butyl 4-(2-oxo-2,3-dihydro-1H-benzo[d]imidazol-1-yl)piperidine-1-carboxylate (1) (98 mg, 0.31 mmol) in N,N-dimethylformamide (1.5 mL) was added sodium hydride (11 mg, 60%, 0.46 mmol) under Ar atmosphere and ice temperature, and the mixture was stirred at room temperature for 30 minutes. To the mixture was then added 3-phenylpropyl bromide (0.37 mmol) under ice temperature, and the mixture was stirred at room temperature for 1 hour. After the reaction was completed, the reaction solution was diluted with diethyl ether (1.5 mL) and water (1.5 mL) was added thereto. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 5:1 to 1:1) to give the title compound (Yield: 95%).

¹H-NMR (400 MHz, CDCl₃) δ: 1.50 (9H, s), 1.81 (2H, d, J = 11.2 Hz), 2.09 (2H, quin, J = 7.6 Hz), 2.31 (2H, qd, *J* = 12.8, 4.4 Hz), 2.72 (2H, t, *J* = 7.6 Hz), 2.86 (2H, br), 3.91 (2H, t, J = 7.6 Hz), 4.31 (2H, br), 4.49 (1H, tt, J = 12.8, 4.4 Hz), 6.90-6.92 (1H, m), 7.04-7.06 (2H, m), 7.11-7.14 (1H, m), 7.20 (3H, d, *J* = 7.2 Hz), 7.28-7.30 (2H, m).

### Reference Examples 4 to 13

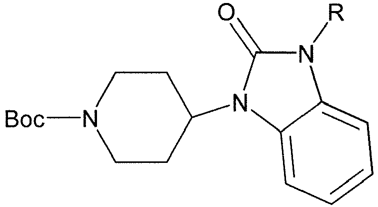

According to the process in Reference Example 3, the compounds of Reference Examples 4 to 13 were prepared by using the corresponding alkyl bromide instead of 3-phenylpropyl bromide.

**[Table 1-1]**

| Reference Example | R | Name | NMR data |
|---|---|---|---|
| Reference Example 4 | (CH₂)₅C₆H₅ | *tert*-butyl 4-(2-oxo-3-(5-phenylpentyl)-2,3-dihydro-1*H-*benzo[d]imidazol-1-yl)piperidine-1-carboxylate (5) | Yield: 89%; ¹H-NMR (400 MHz, CDCl₃) δ: 1.50-1.57 (1H, m), 1.62 (9H, s), 1.78 (2H, quin, *J* = 7.6 Hz), 1.85-1.92 (5H, m), 2.43 (2H, qd, *J* = 12.4, 4.4 Hz), 2.71 (2H, t, *J* = 7.6 Hz), 2.97 (2H, br), 3.98 (2H, t, *J* = 7.6 Hz), 4. 42 (1H, br), 4.60 (1H, tt, *J* = 12.4, 4.4 Hz), 7.09-7.11 (1H, m), 7.18 (2H, td, *J* = 6.8, 1.6 Hz), 7.23-7.29 (4H, m), 7.35-7.39 (2H, m). |
| Reference Example 5 | CH₂C₆H₅ | *tert-*butyl 4-(3-benzyl-2-oxo-2,3-dihydro-1*H-*benzo[d]imidazol-1-yl)piperidine-1-carboxylate (6) | Yield: 88%; ¹H-NMR (400 MHz, CDCl₃) δ: 1.45 (9H, s), 1.79 (2H, d, *J* = 11.6 Hz), 2.28 (2H, qd, *J* = 12.8, 4.4 Hz), 2.81 (2H, br), 2.67 (2H, br), 4.48 (1H, tt, *J* = 12.8, 4.4 Hz), 5.00 (2H, s), 6.84 (1H, dd, *J* = 8.0, 1.2 Hz), 6.93 (1H, td, *J* = 8.0, 1.2 Hz), 6.97 (1H, td, *J* = 8.0, 1.2 Hz), 7.08 (1H, dd, *J* = 8.0, 1.2 Hz), 7.17-7.25 (5H, m). |
| Reference Example 6 | (CH₂)₃OC₆H₅ | *tert*-butyl 4-(2-oxo-3-(3-phenoxypropyl)-2,3-dihydro-1*H-*benzo[d]imidazol-1-yl)piperidine-1-carboxylate (7) | Yield: 92%; ¹H-NMR (400 MHz, CDCl₃) δ: 1.50 (9H, s), 1.80 (2H, d, *J* = 11.2 Hz), 2.24 (2H, quin, *J* = 6.0 Hz), 2.31 (2H, qd, *J* = 12.4, 4.8 Hz), 2.86 (2H, br), 4.02 (2H, t, *J* = 6.4 Hz), 4.09 (2H, t, *J* = 6.4 Hz), 4.30 (2H, br), 4.48 (1H, tt, *J* = 12.4, 4.8 Hz), 6.88 (2H, dd, *J* = 8.0, 1.2 Hz), 6.95 (1H, tt, *J* = 8.0, 1.2 Hz), 7.01-7.06 (3H, m), 7.11-7.13 (1H, m), 7.25-7.30 (2H, m). |

**[Table 1-2]**

| | | | |
|---|---|---|---|
| Reference Example 7 | (CH₂)₄C₆H₄ (o-OMe) | *tert*-butyl 4-(3-(4-(2-methoxyphenyl)but yl)-2-oxo-2,3-dihydro-1H-benzo[*d*]imidazol-1-yl)piperidine-1-carboxylate (8) | Yield: 81%; ¹H-NMR (400 MHz, CDCl₃) δ: 1.50 (9H, s), 1.63-1.82 (7H, m), 2.31 (3H, qd, *J* = 12.4, 4.4 Hz), 2.66 (2H, t, *J* = 12.4 Hz), 2.86 (2H, t, *J* = 12.4 Hz), 3.80 (3H, s), 3.88 (2H, t, *J* = 7.2 Hz), 4.31 (2H, br), 4.49 (1H, tt, *J* = 12.4, 4.4 Hz), 6.83 (1H, d, *J* = 8.0 Hz), 6.87 (1H, dd, *J* = 8.0, 1.6 Hz), 6.97-7.02 (1H, m), 7.05 (2H, dt, *J* = 8.0, 1.6 Hz), 7.09-7.13 (2H, m), 7.16 (1H, dt, *J* = 8.0, 1.6 Hz). |
| Reference Example 8 | (CH₂)₄C₆H₄ (o-Cl) | *tert*-butyl 4-(3-(4-(2-chlorophenyl)buty l)-2-oxo-2,3-dihydro-1H-benzo[d]imidazol-1-yl)piperidine-1-carboxylate (9) | Yield: 69%; ¹H-NMR (400 MHz, CDCl₃) δ: 1.50 (9H, s), 1.67-1.74 (3H, m), 1.79-1.85 (4H, m), 2.31 (2H, qd, *J* = 12.8, 4.4 Hz), 2.77 (2H, t, *J* = 7.2 Hz), 2.86 (2H, br), 3.90 (2H, t, *J* = 7.2 Hz), 4.31 (2H, br), 4.49 (1H, tt, *J* = 12.8, 4.4 Hz), 6.98-7.00 (1H, m), 7.06 (2H, td, *J* = 7.6, 2.0 Hz), 7.09-7.20 (4H, m), 7.31 (1H, dd, *J* = 7.6, 2.0 Hz). |
| Reference Example 9 | (CH₂)₄C₆H₄ (o-F) | *tert*-butyl 4-(3-(4-(2-fluorophenyl)buty l)-2-oxo-2,3-dihydro-1H-benzo[d]imidazol-1-yl)piperidine-1-carboxylate (10) | Yield: 66%; ¹H-NMR (400 MHz, CDCl₃) δ: 1.50 (9H, s), 1.66-1.82 (6H, m), 2.31 (2H, qd, *J* = 12.8, 4.4 Hz), 2.69 (2H, t, *J* = 7.6 Hz), 2.86 (2H, t, *J* = 10.8 Hz), 3.89 (2H, t, *J* = 7.6 Hz), 4.31 (2H, d, *J* = 12.8 Hz), 4.48 (1H, tt, *J* = 12.8, 4.4 Hz), 6.96-7.07 (5H, m), 7.11-7.18 (3H, m). |

**[Table 1-3]**

| | | | |
|---|---|---|---|
| Reference Example 10 | (CH₂)₄C₆H ₄(*m*-Cl) | *tert*-butyl 4-(3-(4-(3-chloro)butyl)-2-oxo-2,3-dihydro-*1H-*benzo [*d*] imidazol -1-yl)piperidine-1-carboxylate (11) | Yield: 73%; ¹H-NMR (400 MHz, CDCl₃) δ: 1.50 (9H, s), 1.64-1.82 (6H, m), 2.31 (2H, qd, *J* = 12.4, 4.0 Hz), 2.63 (2H, t, *J* = 7.2 Hz), 2.86 (2H, br), 3.89 (2H, t, *J* = 7.2 Hz), 4.31 (2H, br), 4.48 (1H, tt, *J* = 12.4, 4.0 Hz), 6.95-6.97 (1H, m), 7.02-7.09 (3H, m), 7.12-7.20 (4H, m). |
| Reference Example 11 | (CH₂)₄C₆H ₄(*m*-F) | *tert*-butyl 4-(3-(4- (3-fluorophenyl)but yl)-2-oxo-2,3-dihydro-1*H-*benzo[*d*]imidazol -1-yl)piperidine-1-carboxylate (12) | Yield: 72%; ¹H-NMR (400 MHz, CDCl₃) δ: 1.50 (9H, s), 1.65-1.82 (6H, m), 2.31 (2H, qd, *J* = 12.0, 4.4 Hz), 2.65 (2H, t, *J* = 7.6 Hz), 2.86 (2H, t, *J* = 12.0 Hz), 3.89 (2H, t, *J* = 7.6 Hz), 4.30 (2H, br), 4.48 (1H, tt, *J* = 12.0, 4.4 Hz), 6.84-6.97 (4H, m), 7.02-7.09 (2H, m), 7.12-7.14 (1H, m), 7.18-7.24 (1H, m). |
| Reference Example 12 | (CH₂)₄C₆H ₄(P-Cl) | *tert*-butyl 4-(3-(4-(4-chlorophenyl)but yl) -2-oxo-2, 3-dihydro-1*H-*benzo [*d*] imidazol -1-yl)piperidine-1-carboxylate (13) | Yield: 64%; ¹H-NMR (400 MHz, CDCl₃) δ: 1.50 (9H, s), 1.63-1.81 (6H, m), 2.31 (2H, qd, *J* = 12.4, 4.0 Hz), 2.62 (2H, t, *J* = 7.2 Hz), 2.86 (2H, br), 3.88 (3H, t, *J* = 7.2 Hz), 4.31 (2H, br), 4.48 (1H, tt, *J* = 12.4, 4.0 Hz), 6.94-6.96 (1H, m), 7.04-7.08 (4H, m), 7.12-7.14 (1H, m), 7.20-7.24 (2H, m). |
| Reference Example 13 | (CH₂)₄C₆H ₄(*p*-F) | *tert*-butyl 4-(3-(4-(4-fluorophenyl)but yl)-2-oxo-2,3-dihydro-1*H-*benzo[*d*]imidazol -1-yl)piperidine-1-carboxylate (14) | Yield: 61%; ¹H-NMR (400 MHz, CDCl₃) δ: 1.50 (9H, s), 1.63-1.82 (6H, m), 2.31 (2H, qd, *J* = 12.4, 4.4 Hz), 2.62 (2H, t, *J* = 7.2 Hz), 2.86 (2H, br), 3.88 (2H, t, *J* = 7.2 Hz), 4.30 (2H, br), 4.48 (1H, tt, *J* = 12.4, 4.4 Hz), 6.90-6.97 (3H, m), 7.02-7.14 (5H, m). |

### Reference Example 14

### tert-Butyl 4-(6-fluoro-2-oxo-3-(4-phenylbutyl)-2,3-dihydro-1H-benzo[d]imidazol-1-yl)piperidine-1-carboxylate (19)

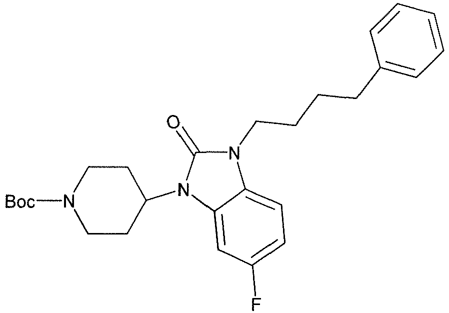

To a solution of *tert*-butyl 4-(6-fluoro-2-oxo-2,3-dihydro-1H-benzo[d]imidazol-1-yl)piperidine-1-carboxylate (15) (0.31 mmol) in N,N-dimethylformamide (1.5 mL) was added sodium hydride (11 mg, 60%, 0.46 mmol) under Ar atmosphere and ice temperature, and the mixture was stirred at room temperature for 30 minutes. To the mixture was then added 4-phenylbutyl bromide (79 mg, 0.37 mmol) at ice temperature and the mixture was stirred at room temperature for 1 hour. After the reaction was completed, the reaction solution was diluted with diethyl ether (1.5 mL) and water (1.5 mL) was further added thereto. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 5:1 to 1:1) to give the title compound (Yield: 45%).

¹H-NMR (500 MHz, CDCl₃) δ: 1.50 (9H, s), 1.67-1.80 (6H, m), 2.24 (2H, dq, *J* = 8.0, 4.5 Hz), 2.65 (2H, t, *J* = 8.0 Hz), 2.85 (2H, br), 3.85 (2H, t, *J* = 8.0 Hz), 4.32 (2H, br), 4.44 (1H, m), 6.76 (1H, dt, *J* = 9.0, 2.0 Hz), 6.82 (1H, q, *J* = 4.5 Hz), 6.86 (1H, dd, *J* = 9. 0, 2.0 Hz), 7.16 (2H, d, *J* = 7.5 Hz), 7.18 (1H, t, *J* = 7.5 Hz) 7.25 (2H, m).

### Reference Example 15

### tert-Butyl 4-(5-chloro-2-oxy-3-(4-phenylbutyl)-2,3-dihydro-1H-benzo[d]imidazol-1-yl)piperidine-1-carboxylate (20)

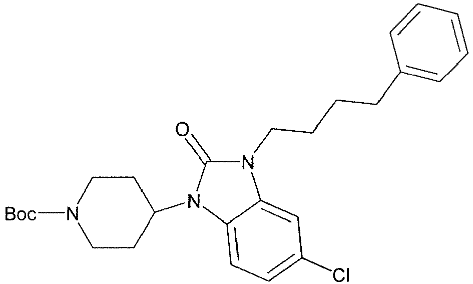

The compound of Reference Example 15 (Yield: 58%) was prepared according to the process in Reference Example 14 by using *tert*-butyl 4-(5-chloro-2-oxo-2,3-dihydro-1H-benzo[d]imidazol-1-yl)piperidine-1-carboxylate (16) instead of *tert*-butyl 4-(6-fluoro-2-oxo-2,3-dihydro-1H-benzo[d]imidazol-1-yl)piperidine-1-carboxylate (15).

¹H-NMR (400 MHz, CDCl₃) δ: 1.56 (9H, s), 1.75-1.86 (6H, m), 2.30 (2H, dq, *J* = 6.0, 3.2 Hz), 2.72 (2H, t, *J* = 6.0 Hz), 2.90 (2H, br), 3.89 (2H, t, *J* = 6.0 Hz), 4.36 (2H, br), 4.50 (1H, tt, *J* = 10.0, 3.2 Hz), 6.99 (1H, s), 7.06 (2H, s), 7.21-7.33 (5H, m).

### Reference Example 16

### tert-Butyl 4-(6-chloro-2-oxo-3-(4-phenylbutyl)-2,3-dihydro-1H-benzo[d]imidazol-1-yl)piperidine-1-carboxylate (21)

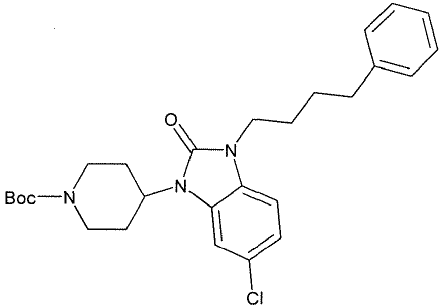

The compound of Reference Example 16 (Yield: 35%) was prepared according to the process in Reference Example 14 by using *tert*-butyl 4-(6-chloro-2-oxo-2,3-dihydro-1H-benzo[d]imidazol-1-yl)piperidine-1-carboxylate (17) instead of *tert*-butyl 4-(6-fluoro-2-oxo-2,3-dihydro-1H-benzo[d]imidazol-1-yl)piperidine-1-carboxylate (15).

¹H-NMR (400 MHz, CDCl₃) δ: 1.51 (9H, s), 1.66-1.81 (6H, m), 2.22-2.33 (2H, m), 2.65 (2H, t, *J* = 7.2 Hz), 2.85 (2H, br), 3.84-3.90 (2H, m), 4.31 (2H, br), 4.41-4.47 (1H, m), 6.84 (1H, d, *J* = 8.0 Hz), 7.02-7.20 (5H, m), 7.25-7.28 (2H, m).

### Reference Example 17

### tert-Butyl 4-(7-chloro-2-oxo-3-(4-phenylbutyl)-2,3-dihydro-1H-benzo[d]imidazol-1-yl)piperidine-1-carboxylate (22)

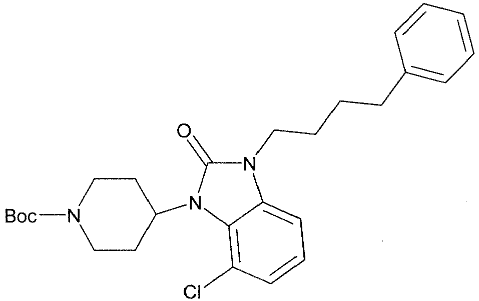

The compound of Reference Example 17 (Yield: 69%) was prepared according to the process in Reference Example 14 by using *tert*-butyl 4-(7-chloro-2-oxo-2,3-dihydro-1H-benzo[d]imidazol-1-yl)piperidine-1-carboxylate (18) instead of *tert*-butyl 4-(6-fluoro-2-oxo-2,3-dihydro-1H-benzo[d]imidazol-1-yl)piperidine-1-carboxylate (15).

¹H-NMR (400 MHz, CDCl₃) δ: 1.48 (9H, s), 1.64-1.82 (7H, m), 2.65 (4H, t, *J* = 6.4 Hz), 2.79 (2H, br), 3.84 (2H, t, *J* = 6.4 Hz), 4.33 (2H, br), 5.14 (1H, br), 6.82 (1H, d, *J* = 7.6 Hz), 6.95-7.02 (2H, m), 7.14-7.19 (3H, m), 7.25-7.28 (2H, m) .

### Example 1

### 1-(1-Benzylpiperidin-4-yl)-1H-benzo[d]imidazol-2(3H)-one (compound 1)

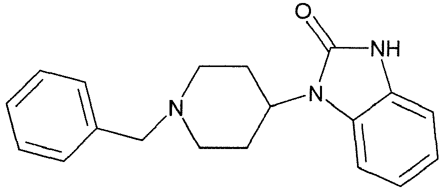

To a solution of tert-butyl 4-(2-oxo-2,3-dihydro-1H-benzo[d]imidazol-1-yl)piperidine-1-carboxylate (1) (100 mg, 0.32 mmol) in dichloromethane (1.5 mL) was added trifluoroacetic acid (0.24 mL, 3.15 mmol) under Ar atmosphere and ice temperature, and the mixture was stirred at room temperature for 3 hours. After the reaction was completed, the solvent in the mixture was removed using a rotary evaporator, and N,N-dimethylformamide (1.5 mL) was added to the resulting residue.

To the mixture were then added benzyl bromide (0.35 mmol) and then potassium carbonate (221 mg, 1.60 mmol) at room temperature, and the mixture was stirred at room temperature for 22 hours. After the reaction was completed, the reaction solution was diluted with diethyl ether (1.5 mL) and water (1.5 mL) was further added thereto. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 1:1 to 1:5) to give the title compound (Yield: 81%, 2 steps).

The product was analyzed by NMR. The result of NMR was completely identical to that of J. Med. Chem., 2018, 61, 8895-8907.

### Example 2

### 1-(1-(2-Phenylethyl)piperidin-4-yl)-1H-benzo[d]imidazol-2(3H)-one (compound 2)

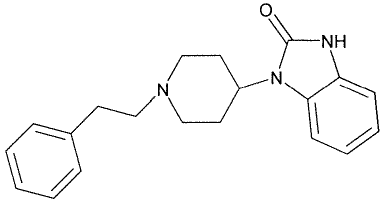

The title compound (Yield: 53%, 2 steps) was prepared according to the process in Example 1 by using 2-phenylethyl bromide instead of benzyl bromide.

The product was analyzed by NMR. The result of NMR was completely identical to that of the commercial compound of Aquila Pharmatech LLC (No. AQ303831).

### Example 3

### 1-(1-(3-Phenylpropyl)piperidin-4-yl)-1H-benzo[d]imidazol-2(3H)-one (compound 3)

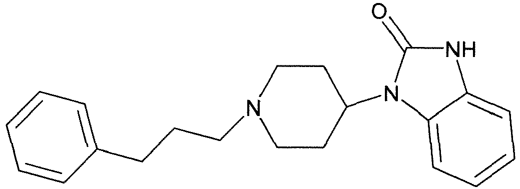

The title compound (Yield: 78%, 2 steps) was prepared according to the process in Example 1 by using 3-phenylpropyl bromide instead of benzyl bromide.

The product was analyzed by NMR. The result of NMR was completely identical to that of J. Med. Chem., 2018, 61, 8895-8907.

### Examples 4 and 5

### 1-(1-(4-Phenylbutyl)piperidin-4-yl)-1H-benzo[d]imidazol-2(3H)-one (compound 4) and 1-(4-phenylbutyl)-3-(1-(4-phenylbutyl)piperidin-4-yl)-1H-benzo[d]imidazol-2(3H)-one (compound 5)

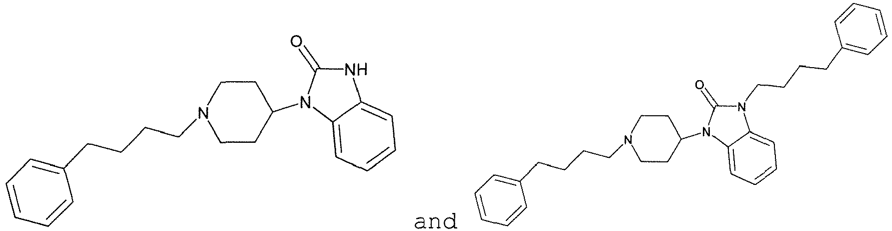

The title compounds (compounds 4 and 5) were prepared according to the process in Example 1 by using 4-phenylbutyl bromide instead of benzyl bromide.

Compound 4 (Yield: 25%, 2 steps) was analyzed by NMR. The result of NMR was completely identical to that of the commercial compound of Chemieliva Pharmaceutical Co., Ltd (No. CB0902185).

The NMR data of compound 5 (Yield: 11%, 2 steps) is as follows: ¹H-NMR (400 MHz, CDCl₃) δ: 1.53-1.80 (10H, m), 2.10 (2H, t, *J* = 11.6 Hz), 2.39-2.47 (4H, m), 2.65 (4H, t, *J* = 7.6 Hz), 3.06 (2H, d, *J* = 11.6 Hz), 3.88 (2H, t, *J* = 6.4 Hz), 4.39 (1H, tt, *J* = 12.4, 4.4 Hz), 6.93-6.95 (1H, m), 7.00-7.07 (2H, m), 7.14-7.20 (6H, m), 7.24-7.31 (5H, m).

### Example 6

### 1-Butyl-3-(1-(4-phenylbutyl)piperidin-4-yl)-1H-benzo[d]imidazol-2(3H)-one (compound 6)

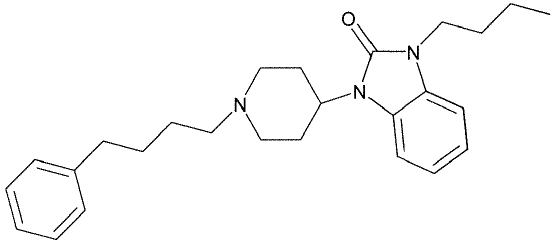

To a solution of compound 4 (29 mg, 0.07 mmol) in N,N-dimethylformamide (1.5 mL) was added sodium hydride (4 mg, 60%, 0.10 mmol) under Ar atmosphere and ice temperature, and the mixture was stirred at room temperature for 30 minutes. To the mixture was then added butyl bromide (0.10 mmol) under ice temperature, and the mixture was stirred at room temperature for 20 hours. After the reaction was completed, the reaction solution was diluted with diethyl ether (1.5 mL) and water (1.5 mL) was further added thereto. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (dichloromethane: methanol = 20:1 to 10:1) to give the title compound (Yield: 57%).

¹H-NMR (400 MHz, CDCl₃) δ: 0.95 (3H, t, *J* = 7.2 Hz), 1.39 (2H, sext, *J* = 7.2 Hz), 1.57-1.87 (10H, m), 2.11 (2H, t, *J* = 11.2 Hz), 2.42 (4H, br), 2.65 (2H, t, *J* = 8.0 Hz), 3.07 (2H, d, *J* = 11.2 Hz), 3.87 (2H, t, *J* = 7.2 Hz), 4.41 (1H, tt, *J* = 12.0, 4.0 Hz), 6.98-7.09 (3H, m), 7.14-7.20 (3H, m), 7.25-7.32 (3H, m).

### Example 7

### 1-(4-Cyclohexylbutyl)-3-(1-(4-phenylbutyl)piperidin-4-yl)-1H-benzo[d]imidazol-2(3H)-one (compound 7)

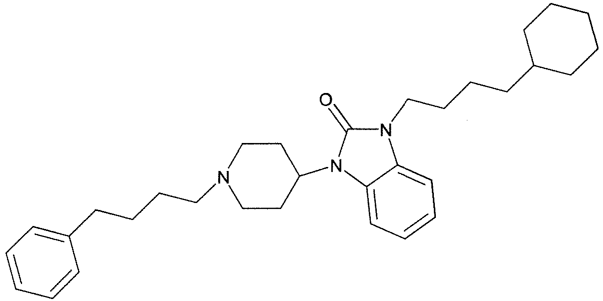

The title compound (Yield: 36%) was prepared according to the process in Example 6 by using 4-cyclohexylbutyl bromide instead of butyl bromide.

¹H-NMR (400 MHz, CDCl₃) δ: 0.79-0.87 (2H, m), 1.12-1.22 (7H, m), 1.32-1. 39 (2H, m), 1.52-1.74 (12H, m), 1.78 (2H, dd, J = 11.6, 1.6 Hz), 2.10 (2H, t, J = 11.6 Hz), 2.38-2.46 (3H, m), 2.64 (2H, t, *J* = 7.6 Hz), 3.05 (2H, d, *J* = 11.6 Hz), 3.84 (2H, t, *J* = 7.6 Hz), 4.39 (1H, tt, *J* = 12.4, 4.4 Hz), 6.98 (1H, dd, *J* = 7.6, 1.6 Hz), 7.02 (1H, td, *J* = 7.6, 1.6 Hz), 7.06 (1H, td, *J* = 7.6, 1.6 Hz), 7.14-7.19 (3H, m), 7.25-7.30 (3H, m).

### Examples 8 and 9

### 1-(1-(4-cyclohexylbutyl)piperidin-4-yl)-1H-benzo[d]imidazol-2(3H)-one (compound 8) and 1-(4-cyclohexylbutyl)-3-(1-(4-cyclohexylbutyl)piperidin-4-yl)-1H-benzo[d]imidazol-2(3H)-one (compound 9)

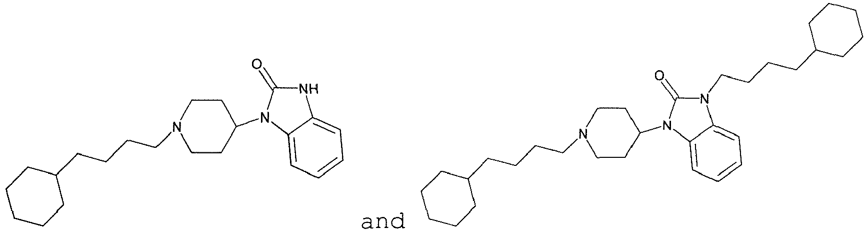

To a solution of tert-butyl 4-(2-oxo-2,3-dihydro-1H-benzo[d]imidazol-1-yl)piperidine-1-carboxylate (1) (100 mg, 0.32 mmol) in dichloromethane (1.5 mL) was added trifluoroacetic acid (0.24 mL, 3.15 mmol) under Ar atmosphere and ice temperature, and the mixture was stirred at room temperature for 3 hours. After the reaction was completed, the solvent in the mixture was removed using a rotary evaporator, and N,N-dimethylformamide (1.5 mL) was added to the resulting residue. To the mixture were further added (4-bromobutyl)cyclohexane (77 mg, 0.35 mmol) and then potassium carbonate (221 mg, 1.60 mmol) at room temperature, and the mixture was stirred at room temperature for 22 hours. After the reaction was completed, the reaction solution was diluted with diethyl ether (1.5 mL) and water (1.5 mL) was further added. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 1:3) to give the title compounds.

Compound 8 (Yield: 28%, 2 steps): ¹H-NMR (400 MHz, CDCl₃) δ: 0.83-0.91 (2H, m), 1.09-1.30 (9H, m), 1.51 (2H, br), 1.63-1.71 (5H, m), 1.83 (2H, d, *J* = 10.0 Hz), 2.14 (2H, br), 2.40 (2H, br), 2.49 (1H, d, *J* = 10.0 Hz), 3.12 (2H, d, *J* = 8.8 Hz), 4.39 (1H, t, J = 12.0 Hz), 7.02-7.11 (4H, m).

Compound 9 (Yield: 16%, 2 steps): ¹H-NMR (400 MHz, CDCl₃) δ: 0.83-0.90 (4H, m), 1.10-1.23 (12H, m), 1.30-1.40 (4H, m), 1.46-1.53 (2H, m), 1.65-1.73 (12H, m), 1.80 (2H, d, *J* = 9.6 Hz), 2.11 (2H, t, *J* = 11.2 Hz), 2.35-2.39 (2H, m), 2.44 (2H, qd, J = 12.4, 4.0 Hz), 3.08 (2H, d, J = 11.2 Hz), 3.85 (2H, t, *J* = 6.8 Hz), 4.41 (1H, tt, *J* = 12.4, 4.0 Hz), 6.99 (1H, dd, *J* = 6.8, 1.2 Hz), 7.03 (1H, td, *J* = 6.8, 1.2 Hz), 7.07 (1H, td, *J* = 6.8, 0.8 Hz), 7.32 (1H, dd, *J* = 6.8, 0.8 Hz).

### Example 10

### 1,3-Bis(4-phenylbutyl)-1H-benzo[d]imidazol-2(3H)-one (compound 10)

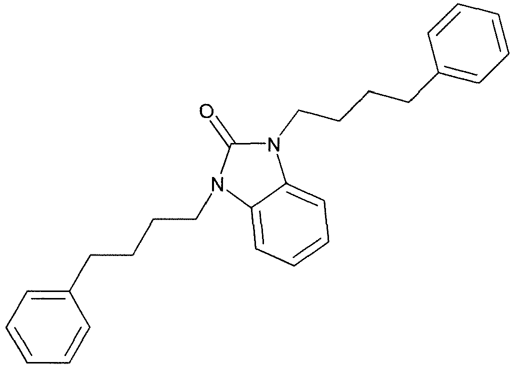

To a solution of the compound of Reference Example 1 (50 mg, 0.19 mmol) in N,N-dimethylamide (1.5 mL) was added sodium hydride (7 mg, 60%, 0.28 mmol) under Ar atmosphere and ice temperature, and the mixture was stirred at room temperature for 30 minutes. To the mixture was then added 4-phenylbutyl bromide(0.23mmol) under ice temperature, and the mixture was stirred at room temperature for 24 hours. After the reaction was completed, the reaction solution was diluted with diethyl ether (1.5 mL) and water (1.5 mL) was further added. The organic layer was separated and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 1:1) to give the title compound (Yield: 54%).

¹H-NMR (400 MHz, CDCl₃) δ: 1.64-1.72 (4H, m), 1.75-1.82 (4H, m), 2.65 (4H, t, *J* = 7.6 Hz), 3.89 (4H, t, *J* = 7.6 Hz), 6.92-6.96 (2H, m), 7.03-7.07 (2H, m).

### Example 11

### 1-(4-Cyclohexylbutyl)-3-(4-phenylbutyl)-1H-benzo[d]imidazol-2(3H)-one (compound 11)

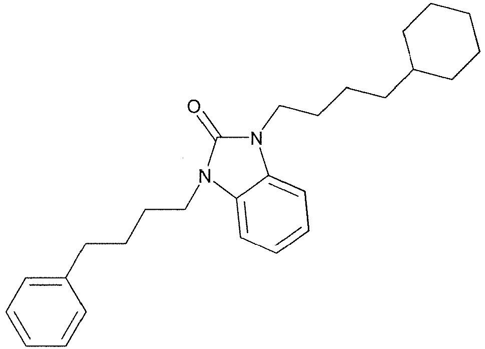

The title compound (Yield: 79%) was prepared according to the process in Example 10 by using 4-cyclohexylbutyl bromide instead of 4-phenylbutyl bromide.

¹H-NMR (400 MHz, CDCl₃) δ: 0.80-0.88 (2H, m), 1.10-1.24 (6H, m), 1.33-1.40 (2H, m), 1.65-1.83 (11H, m), 2.66 (2H, t, J = 7.6 Hz), 3.87 (2H, *J* = 7.6 Hz), 3.90 (2H, t, J = 7.6 Hz), 6.94-7.00 (2H, m), 7.05-7.08 (2H, m), 7.15-7.19 (3H, m), 7.24-7.28 (2H, m).

### Example 12

### 1-(1-(4-Cyclohexylbutyl)piperidin-4-yl)-3-(4-phenylbutyl)-1H-benzo[d]imidazol-2(3H)-one (compound 12)

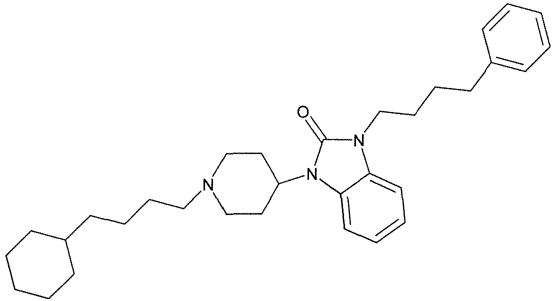

To a solution of the compound of Reference Example 2 (65 mg, 0.15 mmol) in dichloromethane (1.5 mL) was added trifluoroacetic acid (0.11 mL, 1.45 mmol) under Ar atmosphere and ice temperature, and the mixture was stirred at room temperature for 3 hours. After the reaction was completed, the solvent in the mixture was removed using a rotary evaporator, and N,N-dimethylamide (1.5 mL) was added to the resulting residue. To the mixture were then added 4-cyclohexylbutyl bromide (0.16 mmol) and then potassium carbonate (100mg,0.73mmol) at room temperature, and the mixture was stirred at room temperature for 15 hours. After the reaction was completed, the reaction solution was diluted with diethyl ether (1.5 mL) and water (1.5 mL) was further added thereto. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 1:1 to 1:5) to give the title compound (Yield: 44%, 2 steps).

¹H-NMR (400 MHz, CDCl₃) δ: 0.83-0.91 (2H, m), 1.12-1.36 (8H, m), 1.46-1.53 (2H, m), 1.63-1.81 (11H, m), 2.11 (2H, td, J = 10.8, 2.0 Hz), 2.37 (2H, t, J = 7.6 Hz), 2.44 (2H, qd, J = 12.0, 4.0 Hz), 2.65 (2H, t, J = 7.6 Hz), 3.08 (2H, d, J = 12.0 Hz), 4.40 (1H, tt, J = 12.0, 4.0 Hz), 6.94-6.96 (1H, m), 7.01-7.07 (2H, m), 7.15-7.19 (3H, m), 2.24-7.28 (2H, m), 7.30-7.33 (1H, m).

### Example 13

### 1-(1-Butylpiperidin-4-yl)-3-(4-phenylbutyl)-1H-benzo[d]imidazol-2(3H)-one (compound 13)

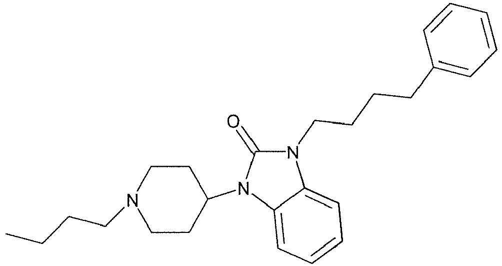

The title compound (Yield: 67%, 2 steps) was prepared according to the process in Example 12 by using butyl bromide instead of 4-cyclohexylbutyl bromide.

¹H-NMR (400 MHz, CDCl₃) δ: 1.02 (3H, t, *J* = 7.6 Hz), 1.43 (2H, sext, *J* = 7.6 Hz), 1.55-1.63 (2H, m), 1.74-1.89 (6H, m), 2.19 (2H, t, *J* = 10.0 Hz), 2.44-2.48 (2H, m), 2.52 (2H, qd, *J* = 12.0, 3.6 Hz), 2.73 (2H, t, *J* = 7.6 Hz), 3.16 (2H, d, *J* = 12.0 Hz), 4.48 (1H, tt, *J* = 12.0, 3.6 Hz), 7.01-7.04 (1H, m), 7.08-7.15 (2H, m), 7.22-7.26 (3H, m), 7.32-7.35 (2H, m), 7.38-7.40 (1H, m).

### Example 14

### 1-(4-Phenylbutyl)-3-(1-(3-phenylpropyl)piperidin-4-yl)-1H-benzo[d]imidazol-2(3H)-one (compound 14)

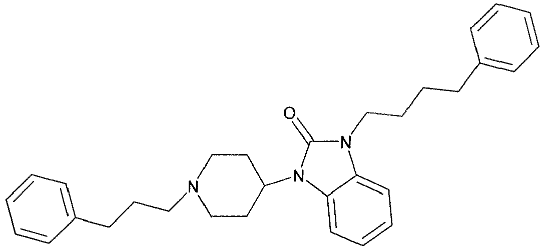

The title compound (Yield: 74%, 2 steps) was prepared according to the process in Example 12 by using 3-phenylpropyl bromide instead of 4-cyclohexylbutyl bromide.

¹H-NMR (400 MHz, CDCl₃) δ: 1.76-1.99 (8H, m), 2.21 (2H, t, *J* = 11.6 Hz), 2.48-2.58 (4H, m), 2.75 (2H, t, *J* = 7.6 Hz), 2.77 (2H, t, *J* = 7.6 Hz), 3.17 (2H, d, *J* = 12.4 Hz), 3.98 (2H, t, *J* = 7.6 Hz), 4.49 (1H, tt, *J =* 12.4, 4.4 Hz), 7.03-7.07 (1H, m), 7.11-7.17 (2H, m), 7.24-7.41 (11H, m).

### Example 15

### 1-(1-(4-Phenylbutanoyl)piperidin-4-yl)-3-(4-phenylbutyl)-1H-benzo[d]imidazol-2(3H)-one (compound 15)

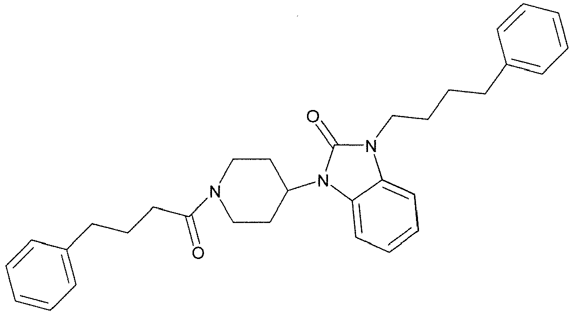

To a solution of the compound of Reference Example 2 (394 mg, 0.88 mmol) in dichloromethane (5 mL) was added trifluoroacetic acid (0.67 mL, 8.78 mmol) under Ar atmosphere and ice temperature, and the mixture was stirred at room temperature for 3 hours. After the reaction was completed, the solvent in the mixture was removed using a rotary evaporator, and dichloromethane (5 mL) was added to the resulting residue. To the mixture were then added 4-phenylbutyric acid (216 mg, 1.32 mmol), 4-dimethylaminopyridine (161 mg, 1.32 mmol), triethylamine (0.19 mL, 1.32 mmol) and then 1-(3-dimethylaminopropyl)-3-ethylcarbodiimide hydrochloride (252 mg, 1.32 mmol) at room temperature, and the mixture was stirred at room temperature for 15 hours. After the reaction was completed, 10% aqueous hydrochloric acid solution (3 mL) was added to the mixture. The organic layer was separated and then the aqueous layer was extracted with dichloromethane (2 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 1:1) to give the title compound (353 mg, 81%, 2 steps, 0.72 mmol).

¹H-NMR (400 MHz, CDCl₃) δ: 1.65-1.87 (7H, m), 1.98-2.06 (3H, m), 2.21-2.33 (2H, m), 2.39 (2H, t, *J* = 8.0 Hz), 2.65 (2H, t, *J* = 6.8 Hz), 2.71 (2H, t, *J* = 6.8 Hz), 3.12 (1H, t, *J =* 12.4 Hz), 3.87 (2H, t, *J* = 6.8 Hz), 4.53 (1H, tt, *J =* 12.4, 4.4 Hz), 4.87 (1H, d, *J* = 12.4 Hz), 6.94-6.97 (1H, m), 7.01-7.09 (3H, m) 7.14-7.31 (10H, m).

### Example 16

### 1-(4-Phenylbutyl)-3-(1-(5-phenylpentyl)piperidin-4-yl)-1H-benzo[d]imidazol-2(3H)-one (compound 16)

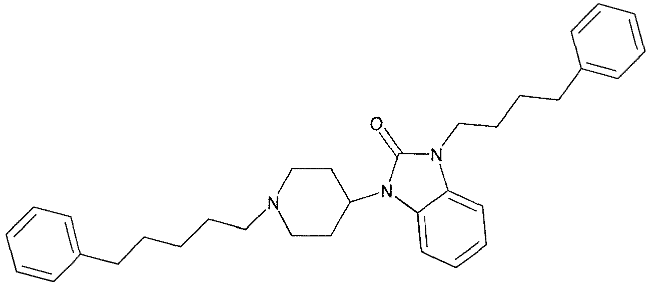

To a solution of the compound of Reference Example 2 (0.21 mmol) in dichloromethane (1 mL) was added trifluoroacetic acid (0.16 mL, 2.14 mmol) under Ar atmosphere and ice temperature, and the mixture was stirred at room temperature for 3 hours. After the reaction was completed, the solvent in the mixture was removed using a rotary evaporator, and N,N-dimethylamide (1 mL) was added to the resulting residue. To the mixture were then added 5-phenylpentyl bromide (0.43 mmol) and then potassium carbonate (147 mg, 1.07 mmol) at room temperature, and the mixture was stirred at room temperature for 22 hours. After the reaction was completed, the reaction solution was diluted with diethyl ether (1.5 mL) and water (1.5 mL) was further added thereto. The organic layer was separated and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 5:1 to 1:2) to give the title compound (Yield: 33%, 2 steps).

¹H-NMR (400 MHz, CDCl₃) δ: 1.34-1.42 (2H, m), 1.53-1.81 (10H, m), 2.12 (2H, t, *J* = 11.6 Hz), 2.37-2.50 (4H, m), 2.64 (2H, t, *J* = 8.0 Hz), 2.66 (2H, t, *J* = 8.0 Hz), 3.09 (2H, d, *J* = 10.8 Hz), 3.89 (2H, t, *J* = 8.0 Hz), 4.41 (1H, tt, *J* = 13.2, 4.0 Hz), 6.94-6.96 (1H, m), 7.02-7.08 (2H, m), 7,15-7.20 (6H, m), 7.25-7.32 (5H, m).

### Example 17

### 1-(1-(4-Phenylbutyl)piperidin-4-yl)-3-(3-phenylpropyl)-1H-benzo[d]imidazol-2(3H)-one (compound 17)

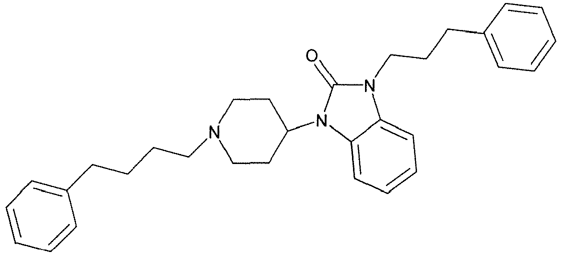

The title compound (Yield: 16%, 2 steps) was prepared according to the process in Example 16 by using the compound of Reference Example 3 and 4-phenylbutyl bromide instead of the compound of Reference Example 2 and 5-phenylpentyl bromide, respectively.

¹H-NMR (400 MHz, CDCl₃) δ: 1.59-1.76 (4H, m), 1.85 (2H, d, J = 10.8 Hz), 2.12-2.20 (4H, m), 2.45-2.53 (4H, m), 2.70 (2H, t, J = 7.6 Hz), 2.76 (2H, t, J = 7.6 Hz), 3.12 (2H, d, J = 10.8 Hz), 3.96 (2H, t, J = 7.6 Hz), 4.46 (1H, tt, J = 12.4, 4.0 Hz), 6.94-6.96 (1H, m), 7.06-7.12 (2H, m), 7.22-7.25 (5H, m), 7.30-7.37 (6H, m).

### Example 18

### 1-(1-(4-phenylbutyl)piperidin-4-yl)-3-(5-phenylpentyl)-1H-benzo[d]imidazol-2(3H)-one (compound 18)

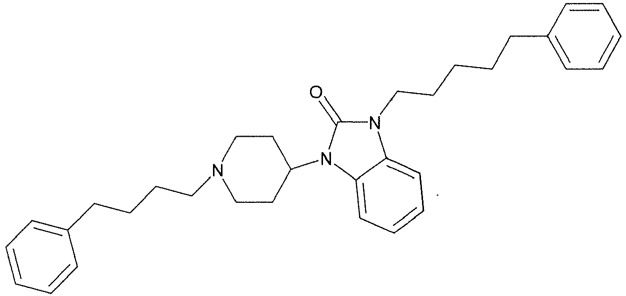

The title compound (Yield: 28%, 2 steps) was prepared according to the process in Example 16 by using the compound of Reference Example 4 and 4-phenylbutyl bromide instead of the compound of Reference Example 2 and 5-phenylpentyl bromide, respectively.

¹H-NMR (400 MHz, CDCl₃) δ: 1.40-1.48 (2H, m), 1.56-1.63 (2H, m), 1.65-1.73 (4H, m), 1.76-1.83 (4H, m), 2.13 (2H, t, J = 11.6 Hz), 2.42-2.50 (4H, m), 2.62 (2H, t, J = 8.0 Hz), 2.68 (2H, t, J = 8.0 Hz), 3.09 (2H, d, J = 11.6 Hz), 3.88 (2H, t, J = 8.0 Hz), 4.42 (1H, tt, J = 11.6, 4.4 Hz), 6.98-7.01 (1H, m), 7.04-7.11 (2H, m), 7.15-7.23 (6H, m), 7.27-7.34 (5H, m).

### Example 19

### 1-(1-benzylpiperidin-4-yl)-3-(4-phenylbutyl)-1H-benzo[d]imidazol-2(3H)-one (compound 19)

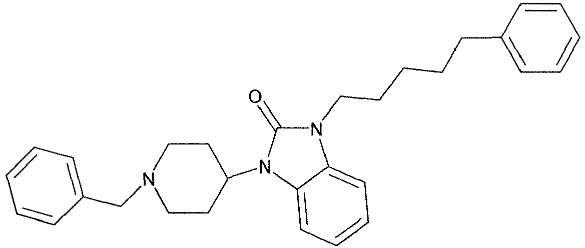

The title compound (Yield: 82%, 2 steps) was prepared according to the process in Example 16 by using the compound of Reference Example 4 and benzyl bromide instead of the compound of Reference Example 2 and 5-phenylpentyl bromide, respectively.

¹H-NMR (400 MHz, CDCl₃) δ: 1.66-1.82 (6H, m), 2.17 (2H, td, J = 12.0, 2.0 Hz), 2.46 (2H, qd, J = 12.0, 4.0 Hz), 2.66 (2H, t, J = 7.2 Hz), 3.03 (2H, d, J = 12.0 Hz), 3.88 (2H, t, J = 7.2 Hz), 4.39 (1H, tt, J = 12.0, 4.0 Hz), 6.93-6.97 (1H, m), 7.03-7.08 (2H, m), 7.15-7.19 (3H, m), 7.24-7.39 (7H, m).

### Example 20

### 1-Benzyl-3-(1-(4-phenylbutyl)piperidin-4-yl)-1H-benzo[d]imidazol-2(3H)-one (compound 20)

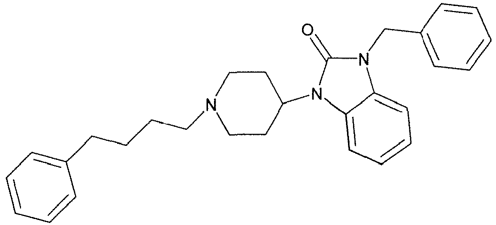

The title compound (Yield: 42%, 2 steps) was prepared according to the process in Example 16 by using the compound of Reference Example 5 and benzyl bromide instead of the compound of Reference Example 2 and 5-phenylpentyl bromide, respectively.

¹H-NMR (400 MHz, CDCl₃) δ: 1.46-1.56 (2H, m), 1.59-1.67 (2H, m), 1.79 (2H, dd, J = 12.0, 2.0 Hz), 2.07 (2H, t, J = 12.0 Hz), 2.37 (2H, t, J = 7.2 Hz), 2.41 (2H, qd, J = 12.0, 3.6 Hz), 2.61 (2H, t, J = 7.2 Hz), 3.03 (2H, d, J = 12.0 Hz), 4.40 (1H, tt, J = 12.0, 3.6 Hz), 6.88 (1H, dd, J = 8.0, 1.6 Hz), 6.93 (1H, td, J = 8.0, 1.6 Hz), 6.97 (1H, td, J = 8.0, 1.6 Hz), 7.12-7.16 (3H, m), 7.19-7.27 (8H, m).

### Example 21

### 1-(1-(3-Phenoxypropyl)piperidin-4-yl)-3-(4-phenylbutyl)-1H-benzo[d]imidazol-2(3H)-one (compound 21)

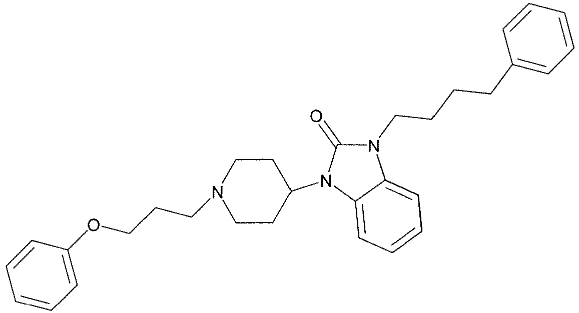

The title compound (Yield: 68%, 2 steps) was prepared according to the process in Example 16 by using 3-phenoxypropyl bromide instead of 5-phenylpentyl bromide.

¹H-NMR (400 MHz, CDCl₃) δ: 1.63-1.83 (8H, m), 2.03 (2H, br), 2.17 (2H, br), 2.46 (2H, br), 2.60 (2H, br), 2.66 (2H, t, J = 7.6 Hz), 3.11 (1H, br), 3.88 (2H, t, J = 7.6 Hz), 4.06 (2H, t, J = 6.4 Hz), 6.91-6.97 (4H, m), 7.05-0.06 (2H, m), 7.15-7.19 (3H, m), 7.25-7.32 (5H, m).

### Example 22

### 1-(3-Phenoxypropyl)-3-(1-(4-phenylbutyl)piperidin-4-yl)-1H-benzo[d]imidazol-2(3H)-one (compound 22)

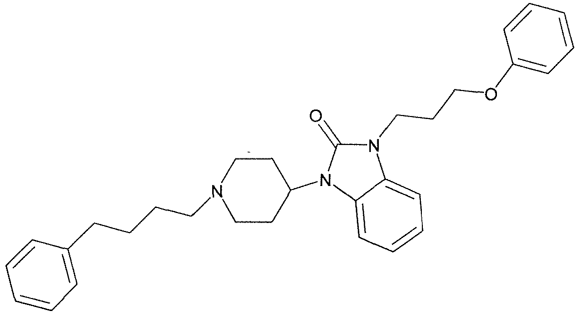

The title compound (Yield: 26%, 2 steps) was prepared according to the process in Example 16 by using the compound of Reference Example 6 and 4-phenylbutyl bromide instead of the compound of Reference Example 2 and 5-phenylpentyl bromide, respectively.

¹H-NMR (400 MHz, CDCl₃) δ: 1.25-1.29 (2H, m), 1.60-1.70 (4H, m), 1.79 (2H, d, J = 10.8 Hz), 2.15 (1H, br), 2.23 (2H, quin, J = 6.4 Hz), 2.44 (3H, br), 2.65 (2H, t, J = 7.6 Hz), 3.09 (2H, br), 4.01 (2H, t, J = 6.4 Hz), 4.08 (2H, t, J = 6.4 Hz), 4.41 (1H, t, J = 12.4 Hz), 6.87 (2H, d, J = 8.4 Hz), 6.94 (1H, t, J = 7.6 Hz), 6.98-7.05 (3H, m), 7.19 (3H, J = 7.6 Hz), 7.24-7.32 (5H, m).

### Example 23

### 1-(3-Phenoxypropyl)-3-(1-(3-phenoxypropyl)piperidin-4-yl)-1H-benzo[d]imidazol-2(3H)-one (compound 23)

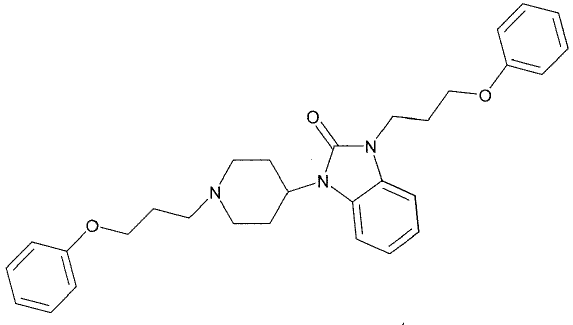

The title compound (Yield: 66%, 2 steps) was prepared according to the process in Example 16 by using the compound of Reference Example 6 and 3-phenoxypropyl bromide instead of the compound of Reference Example 2 and 5-phenylpentyl bromide, respectively.

¹H-NMR (400 MHz, CDCl₃) δ: 2.05 (2H, d, *J* = 11.6 Hz), 2.27 (2H, br), 2.48 (4H, quin, *J* = 6.4 Hz), 2.71 (2H, br), 2.85 (2H, m), 3.36 (2H, br), 4.25 (2H, t, *J* = 6.4 Hz), 4.29 (2H, t, *J* = 6.4 Hz), 4.33 (2H, t, *J* = 6.4 Hz), 4.66 (1H, br), 7.11-7.20 (6H, m), 7.25-7.30 (3H, m), 7.49-7.55 (5H, m).

### Example 24

### 1-(1-(4-(2-Methoxyphenyl)butyl)piperidin-4-yl)-3-(4-phenylbutyl)-1H-benzo[d]imidazol-2(3H)-one (compound 24)

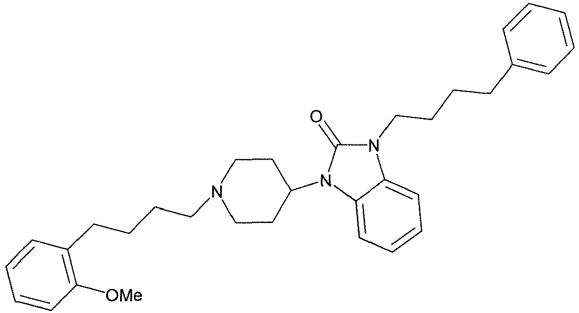

The title compound (Yield: 49%, 2 steps) was prepared according to the process in Example 16 by using 4-(2-methoxyphenyl)butyl bromide instead of 5-phenylpentyl bromide.

¹H-NMR (400 MHz, CDCl₃) δ: 1.61-1.82 (12H, m), 2.14 (2H, br), 2.44 (3H, br), 2.64-2.68 (3H, m), 3.09 (2H, br), 3.83 (3H, s), 3.88 (2H, t, *J* = 7.2 Hz), 4.41 (1H, t, *J* = 12.0 Hz), 6.85 (1H, d, *J* = 7.2 Hz), 6.90 (1H, td, *J* = 7.2, 0.8 Hz), 6.94-6.96 (1H, m), 7.04-7.05 (2H, m), 7.13-7.20 (5H, m), 7.24-7.28 (3H, m).

### Example 25

### 1-(4-(2-Methoxyphenyl)butyl)-3-(1-(4-phenylbutyl)piperidin-4-yl)-1H-benzo[d]imidazol-2(3H)-one (compound 25)

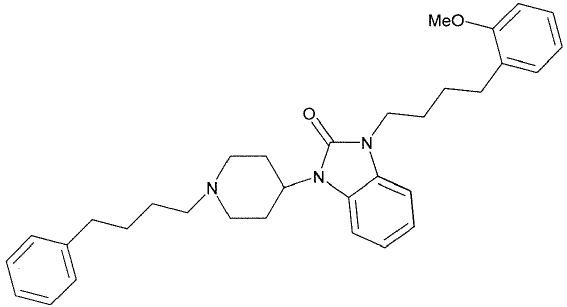

The title compound (Yield: 35%, 2 steps) was prepared according to the process in Example 16 by using the compound of Reference Example 7 and 4-phenylbutyl bromide instead of the compound of Reference Example 2 and 5-phenylpentyl bromide, respectively.

¹H-NMR (400 MHz, CDCl₃) δ: 1.23-1.28 (2H, m), 1.66-1.82 (10H, m), 2.14 (2H, br), 2.44 (3H, br), 2.66 (3H, t, *J* = 7.6 Hz), 3.09 (2H, br), 3.78 (3H, s), 3.88 (2H, t, *J* = 7.6 Hz), 4.41 (1H, br), 6.82-6.88 (2H, m), 6.96-6.98 (1H, m), 7.04-7.11 (3H, m), 7.14-7.20 (4H, m), 7.29-7.31 (2H, m).

### Example 26

### 1-(4-(2-Methoxyphenyl)butyl)-3-(1-(4-(2-methoxyphenyl)butyl)piperidin-4-yl)-1H-benzo[d]imidazol-2(3H)-one (compound 26)

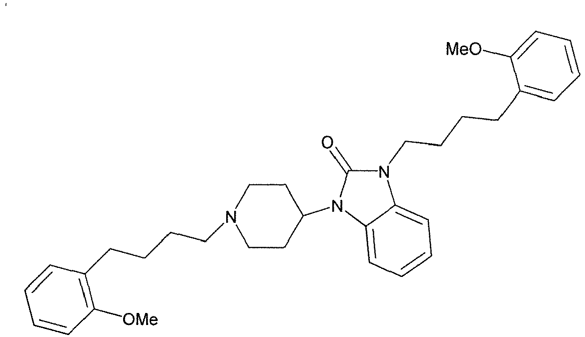

The title compound (Yield: 33%, 2 steps) was prepared according to the process in Example 16 by using the compound of Reference Example 7 and 4-(2-methoxyphenyl)butyl bromide instead of the compound of Reference Example 2 and 5-phenylpentyl bromide, respectively.

¹H-NMR (400 MHz, CDCl₃) δ: 1.61-1.81 (10H, m), 2.14 (2H, t, *J* = 11.6 Hz), 2.42-2.48 (4H, m), 2.64-2.68 (4H, m), 3.10 (2H, d, *J* = 11.6 Hz), 3.80 (3H, s), 3.83 (3H, s), 3.88 (2H, t, *J* = 7.2 Hz), 4.41 (1H, tt, *J* = 11.6, 4.8 Hz), 6.82-6.91 (4H, m), 6.96-7.20 (7H, m), 7.32-7.34 (1H, m).

### Example 27

### 1-(1-(4-(2-Chlorophenyl)butyl)piperidin-4-yl)-3-(4-phenylbutyl)-1H-benzo[d]imidazol-2(3H)-one (compound 27)

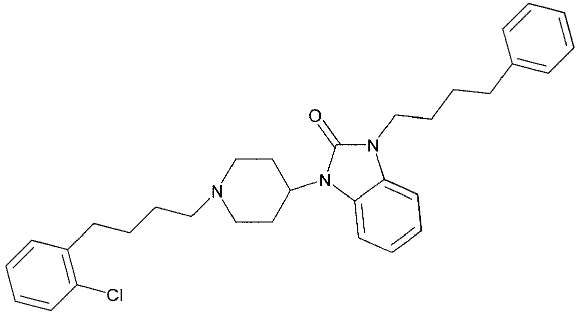

The title compound (Yield: 54%, 2 steps) was prepared according to the process in Example 16 by using 4-(2-chlorophenyl)butyl bromide instead of 5-phenylpentyl bromide.

¹H-NMR (400 MHz, CDCl₃) δ: 1.67-1.84 (11H, m), 2.16 (2H, br), 2.46 (3H, br), 2.66 (2H, t, *J* = 7.2 Hz), 2.78 (2H, t, *J* = 7.2 Hz), 3.11 (2H, br), 3.88 (2H, t, *J* = 7.2 Hz), 4.42 (1H, br), 6.94-6.97 (1H, m), 7.02-7.07 (2H, m), 7.11-7.28 (8H, m), 7.34 (2H, dd, *J = 8.0,* 1.6 Hz).

### Example 28

### 1-(4-(2-Chlorophenyl)butyl)-3-(1-(4-phenylbutyl)piperidin-4-yl)-1H-benzo[d]imidazol-2(3H)-one (compound 28)

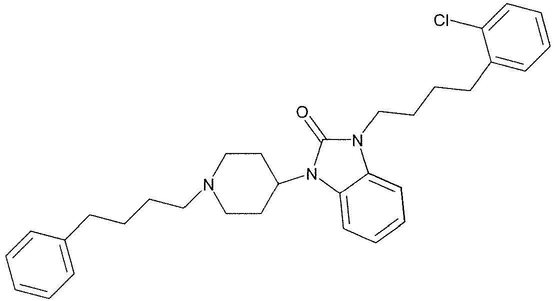

The title compound (Yield: 32%, 2 steps) was prepared according to the process in Example 16 by using the compound of Reference Example 8 and 4-phenylbutyl bromide instead of the compound of Reference Example 2 and 5-phenylpentyl bromide, respectively.

¹H-NMR (400 MHz, CDCl₃) δ: 1.26-1.49 (2H, m), 1.67-1.87 (12H, m), 2.17 (1H, br), 2.46 (2H, br), 2.66 (2H, m), 2.77 (2H, t, *J =* 7.6 Hz), 3.11 (1H, br), 3.90 (2H, t, *J* = 7.6 Hz), 4.45 (1H, m), 6.96-6.99 (1H, m), 7.06-7.20 (9H, m), 7.27-7.33 (3H, m) .

### Example 29

### 1-(4-(2-Chlorophenyl)butyl)-3-(1-(4-(2-chlorophenyl)butyl)piperidin-4-yl)-1H-benzo[d]imidazol-2(3H)-one (compound 29)

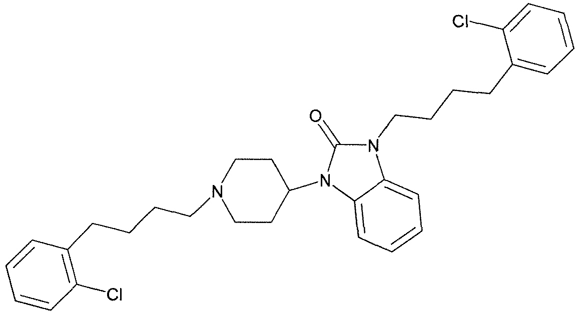

The title compound (Yield: 70%, 2 steps) was prepared according to the process in Example 16 by using the compound of Reference Example 8 and 4-(2-chlorophenyl)butyl bromide instead of the compound of Reference Example 2 and 5-phenylpentyl bromide, respectively.

¹H-NMR (400 MHz, CDCl₃) δ: 1.66-1.83 (14H, m), 2.17 (1H, br), 2.46 (2H, br), 2.76 (4H, t, *J* = 7.6 Hz), 3.11 (1H, br), 3.89 (2H, t, *J* = 7.6 Hz), 4.43 (1H, br), 6.95-6.98 (1H, m), 7.03-7.08 (2H, m), 7.08-7.23 (7H, m), 7.29-7.34 (2H, m).

### Example 30

### 1-(1-(4-(2-Fluorophenyl)butyl)piperidin-4-yl)-3-(4-phenylbutyl)-1H-benzo[d]imidazol-2(3H)-one (compound 30)

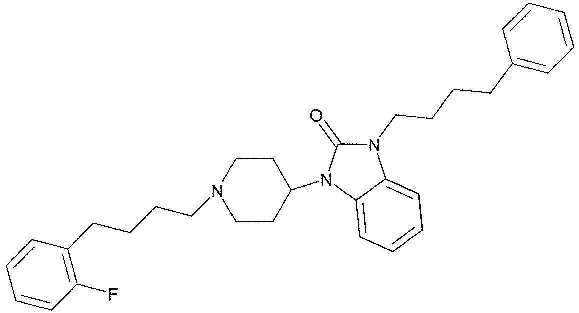

The title compound (Yield: 73%, 2 steps) was prepared according to the process in Example 16 by using 4-(2-fluorophenyl)butyl bromide instead of 5-phenylpentyl bromide.

¹H-NMR (400 MHz, CDCl₃) δ: 1.60-1.81 (10H, m), 2.14 (2H, br), 2.44 (4H, br), 2.64-2.71 (4H, m), 3.08 (2H, br), 3.88 (2H, t, *J* = 6.8 Hz), 4.41 (1H, br), 6.94-7.08 (5H, m), 7.15-7.21 (5H, m), 7.24-7.28 (3H, m), 7.32 (1H, br).

### Example 31

### 1-(4-(2-Fluorophenyl)butyl)-3-(1-(4-phenylbutyl)piperidin-4-yl)-1H-benzo[d]imidazol-2(3H)-one (compound 31)

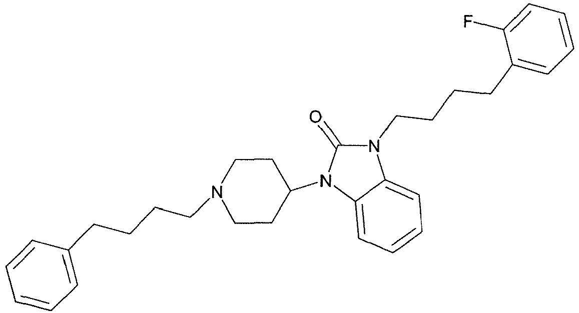

The title compound (Yield: 51%, 2 steps) was prepared according to the process in Example 16 by using the compound of Reference Example 9 and 4-phenylbutyl bromide instead of the compound of Reference Example 2 and 5-phenylpentyl bromide, respectively.

¹H-NMR (400 MHz, CDCl₃) δ: 1.60-1.74 (9H, m), 1.75-1.84 (5H, m), 2.17 (1H, br), 2.46 (2H, br), 2.64-2.71 (4H, m), 3.11 (1H, br), 3.89 (2H, t, *J* = 7.6 Hz), 4.42 (1H, br), 6.95-7.80 (5H, m), 7.13-7.20 (6H, m), 7.27-7.31 (3H, m).

### Example 32

### 1-(4-(2-Fluorophenyl)butyl)-3-(1-(4-(2-fluorophenyl)butyl)piperidin-4-yl)-1H-benzo[d]imidazol-2(3H)-one (compound 32)

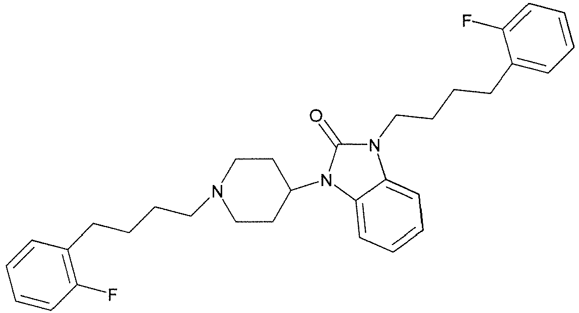

The title compound (Yield: 73%, 2 steps) was prepared according to the process in Example 16 by using the compound of Reference Example 9 and 4-(2-fluorophenyl)butyl bromide instead of the compound of Reference Example 2 and 5-phenylpentyl bromide, respectively.

¹H-NMR (400 MHz, CDCl₃) δ: 1.65-1.81 (12H, m), 2.17 (1H, br), 2.46 (3H, br), 2.69 (4H, t, *J* = 7.2 Hz), 3.10 (2H, br), 3.89 (2H, t, *J* = 7.2 Hz), 4.42 (1H, t, *J* = 12.4 Hz), 6.95-7.08 (8H, m), 7.12-7.22 (4H, m).

### Example 33

### 1-(1-(4-(3-Chlorophenyl)butyl)piperidin-4-yl)-3-(4-phenylbutyl)-1H-benzo[d]imidazol-2(3H)-one (compound 33)

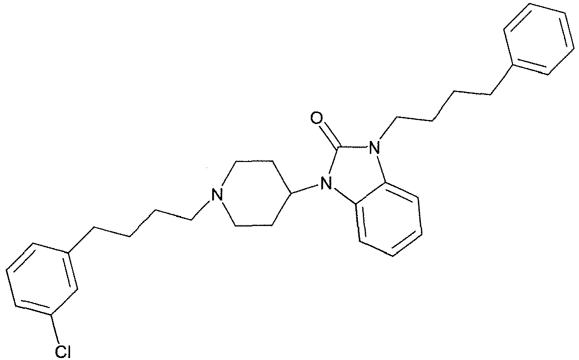

The title compound (Yield: 67%, 2 steps) was prepared according to the process in Example 16 by using 4-(3-chlorophenyl)butyl bromide instead of 5-phenylpentyl bromide.

¹H-NMR (400 MHz, CDCl₃) δ: 1.56-1.82 (11H, m), 2.14 (2H, br), 2.43 (3H, br), 2.63 (2H, t, *J* = 7.2 Hz), 2.66 (2H, t, *J* = 7.2 Hz), 3.08 (2H, d, *J* = 7.2 Hz), 3.88 (2H, t, *J* = 7.2 Hz), 4.41 (1H, t, *J=* 12.0 Hz), 6.94-6.97 (1H, m), 7.02-7.08 (3H, m), 7.15-7.21 (6H, m), 7.23-7.32 (3H, m).

### Example 34

### 1-(4-(3-Chlorophenyl)butyl)-3-(1-(4-phenylbutyl)piperidin-4-yl)-1H-benzo[d]imidazol-2(3H)-one (compound 34)

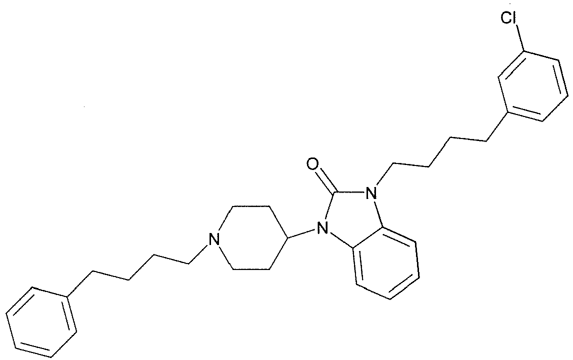

The title compound (Yield: 57%, 2 steps) was prepared according to the process in Example 16 by using the compound of Reference Example 10 and 4-phenylbutyl bromide instead of the compound of Reference Example 2 and 5-phenylpentyl bromide, respectively.

¹H-NMR (400 MHz, CDCl₃) δ: 1.62-1.87 (10H, m), 2.19 (2H, br), 2.49 (4H, m), 2.69-2.73 (4H, m), 3.14 (2H, d, *J* = 7.2 Hz), 3.94 (2H, t, *J* = 7.2 Hz), 4.46 (1H, t, *J* = 12.4 Hz), 7.00-7.02 (1H, m), 7.07-7.14 (3H, m), 7.19-7.24 (6H, m), 7.32-7.38 (3H, m).

### Example 35

### 1-(4-(3-Chlorophenyl)butyl)-3-(1-(4-(3-chlorophenyl)butyl)piperidin-4-yl)-1H-benzo[d]imidazol-2(3H)-one (compound 35)

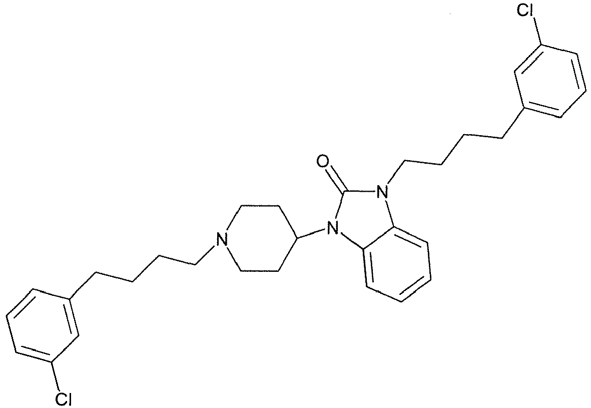

The title compound (Yield: 64%, 2 steps) was prepared according to the process in Example 16 by using the compound of Reference Example 10 and 4-(3-chlorophenyl)butyl bromide instead of the compound of Reference Example 2 and 5-phenylpentyl bromide, respectively.

¹H-NMR (400 MHz, CDCl₃) δ: 1.51-1.58 (2H, m), 1.62-1.71 (4H, m), 1.74-1.81 (4H, m), 2.10 (2H, t, *J* = 10.8 Hz), 2.38-2.42 (2H, m), 2.43 (2H, qd, *J* = 12.4, 4.0 Hz), 2.63 (4H, t, *J =* 7.6 Hz), 3.39 (2H, t, *J* = 7.6 Hz), 4.29 (1H, tt, *J* = 12.4, 4.0 Hz), 6.94-6.96 (1H, m), 7.01-7.08 (4H, m), 7.13-7.21 (6H, m), 7.29-7.31 (1H, m).

### Example 36

### 1-(1-(4-(3-Fluorophenyl)butyl)piperidin-4-yl)-3-(4-phenylbutyl)-1H-benzo[d]imidazol-2(3H)-one (compound 36)

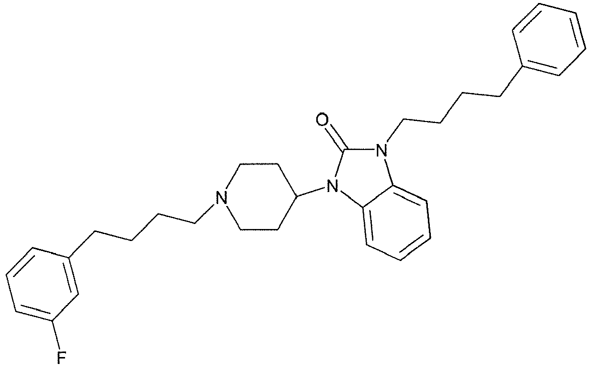

The title compound (Yield: 80%, 2 steps) was prepared according to the process in Example 16 by using 4-(3-fluorophenyl)butyl bromide instead of 5-phenylpentyl bromide.

¹H-NMR (400 MHz, CDCl₃) δ: 1.50-1.80 (10H, m), 2.10 (2H, t, J = 10.0 Hz), 2.37-2.41 (2H, m), 2.43 (2H, qd, *J* = 11.6, 4.4 Hz), 2.64 (2H, td, *J* = 7.2, 2.0 Hz), 3.04 (2H, d, *J* = 11.6 Hz), 3.87 (2H, t, *J* = 7.2 Hz), 4.38 (1H, tt, *J =* 11.6, 4.4 Hz), 6.82-6.97 (4H, m), 7.00-7.07 (2H, m), 7.14-7.31 (8H, m).

### Example 37

### 1-(4-(3-Fluorophenyl)butyl)-3-(1-(4-phenylbutyl)piperidin-4-yl)-1H-benzo[d]imidazol-2(3H)-one (compound 37)

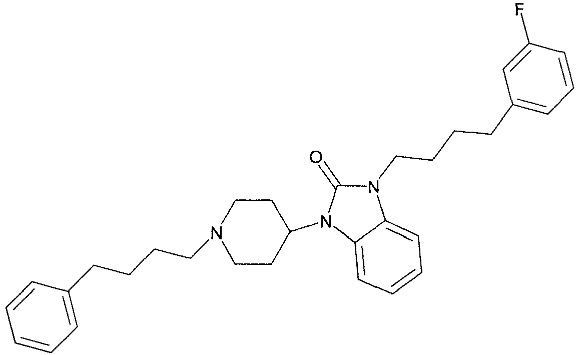

The title compound (Yield: 55%, 2 steps) was prepared according to the process in Example 16 by using the compound of Reference Example 11 and 4-phenylbutyl bromide instead of the compound of Reference Example 2 and 5-phenylpentyl bromide, respectively.

¹H-NMR (400 MHz, CDCl₃) δ: 1.55-1.81 (11H, m), 2.13 (2H, t, *J* = 12.0 Hz), 2.41-2.47 (3H, m), 2.65 (4H, t, *J* = 7.6 Hz), 3.08 (2H, d, *J* = 10.4 Hz), 3.89 (2H, t, *J* = 7.6 Hz), 4.40 (1H, tt, *J=* 12.0, 4.4 Hz), 6.84-6.88 (2H, m), 6.91-6.96 (2H, m), 7.02-7.08 (2H, m), 7.17-7.22 (4H, m), 7.27-7.31 (3H, m).

### Example 38

### 1-(4-(3-Fluorophenyl)butyl)-3-(1-(4-(3-fluorophenyl)butyl)piperidin-4-yl)-1H-benzo[d]imidazol-2(3H)-one (compound 38)

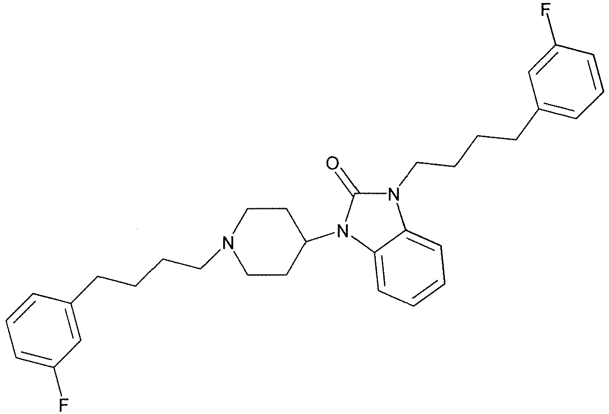

The title compound (Yield: 77%, 2 steps) was prepared according to the process in Example 16 by using the compound of Reference Example 11 and 4-(3-fluorophenyl)butyl bromide instead of the compound of Reference Example 2 and 5-phenylpentyl bromide, respectively.

¹H-NMR (400 MHz, CDCl₃) δ: 1.52-1.58 (2H, m), 1.64-1.72 (4H, m), 1.75-1.81 (4H, m), 2.10 (2H, t, *J* = 8.4 Hz), 2.38-2.42 (2H, m), 2.42 (2H, qd, *J* = 10.0, 3.6 Hz), 2.65 (4H, t, *J* = 6.0 Hz), 3.05 (2H, d, *J* = 8.4 Hz), 3.89 (2H, t, *J* = 6.0 Hz), 4.39 (1H, tt, *J* = 10.0, 3.6 Hz), 6.84-6.97 (7H, m), 7.02-7.08 (2H, m), 7.18-7.25 (2H, m), 7.29-7.31 (1H, m).

### Example 39

### 1-(1-(4-(4-Chloro)butyl)piperidin-4-yl)-3-(4-phenylbutyl)-1H-benzo[d]imidazol-2(3H)-one (compound 39)

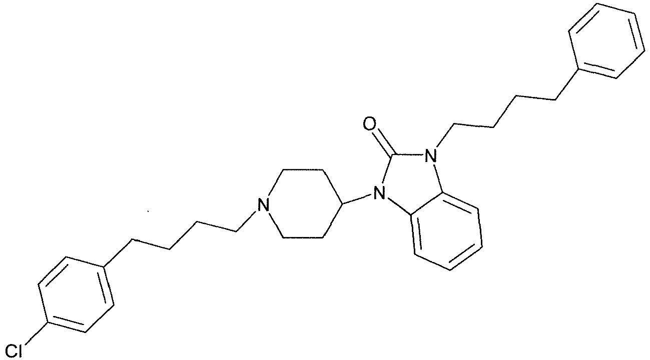

The title compound (Yield: 35%, 2 steps) was prepared according to the process in Example 16 by using 4-(4-chlorophenyl)butyl bromide instead of 5-phenylpentyl bromide.

¹H-NMR (400 MHz, CDCl₃) δ: 1.52-1.81 (10H, m), 2.10 (2H, t, *J* = 10.0 Hz), 2.37-2.41 (2H, m), 2.43 (2H, qd, *J* = 12.4, 4.0 Hz), 2.62 (2H, t, *J* = 7.6 Hz), 2.65 (2H, t, *J* = 7.6 Hz), 3.05 (2H, d, *J* = 10.0 Hz), 3.88 (2H, t, *J* = 7.6 Hz), 4.39 (1H, tt, *J* = 12.4, 4.0 Hz), 6.94-6.96 (1H, m), 7.01-7.07 (2H, m), 7.11-7.19 (5H, m), 7.23-7.30 (5H, m).

### Example 40

### 1-(4-(4-Chlorophenyl)butyl)-3-(1-(4-phenylbutyl)piperidin-4-yl)-1H-benzo[d]imidazol-2(3H)-one (compound 40)

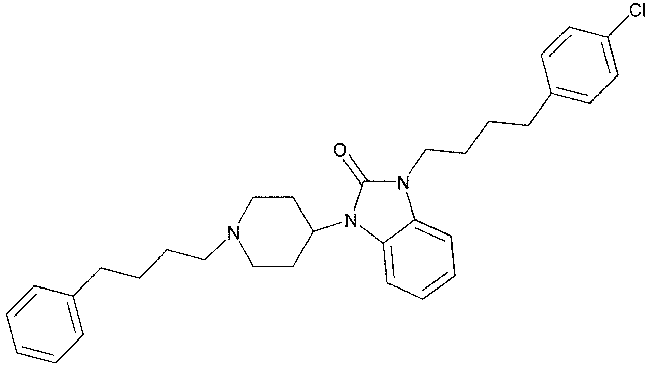

The title compound (Yield: 56%, 2 steps) was prepared according to the process in Example 16 by using the compound of Reference Example 12 and 4-phenylbutyl bromide instead of the compound of Reference Example 2 and 5-phenylpentyl bromide, respectively.

¹H-NMR (400 MHz, CDCl₃) δ: 1.52-1.80 (10H, m), 2.11 (2H, t, *J* = 11.2 Hz), 2.39-2.47 (4H, m), 2.62 (2H, t, *J* = 7.2 Hz), 2.65 (2H, t, *J* = 7.2 Hz), 3.06 (2H, d, *J* = 11.2 Hz), 3.88 (2H, t, *J* = 6.8 Hz), 4.39 (1H, tt, *J* = 12.4, 4.4 Hz), 6.93-6.95 (1H, m), 7.01-7.08 (4H, m), 7.17-7.23 (4H, m), 7.27-7.32 (4H, m).

### Example 41

### 1-(4-(4-Chlorophenyl)butyl)-3-(1-(4-(4-chlorophenyl)butyl)piperidin-4-yl)-1H-benzo[d]imidazol-2(3H)-one (compound 41)

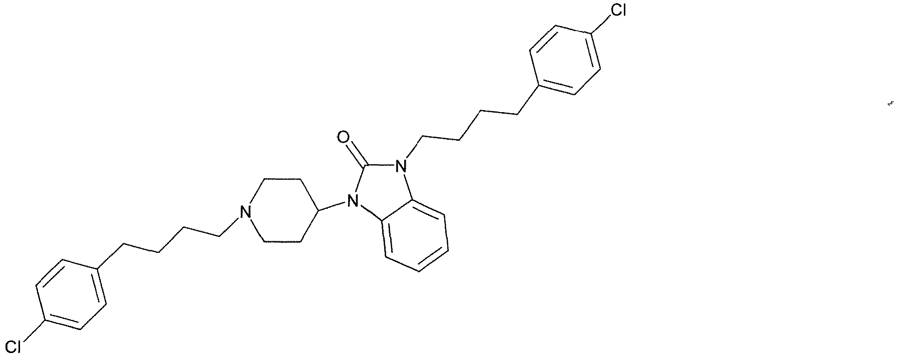

The title compound (Yield: 54%, 2 steps) was prepared according to the process in Example 16 by using the compound of Reference Example 12 and 4-(4-chlorophenyl)butyl bromide instead of the compound of Reference Example 2 and 5-phenylpentyl bromide, respectively.

¹H-NMR (400 MHz, CDCl₃) δ: 1.50-1.60 (2H, m), 1.60-1.71 (6H, m), 1.72-1.80 (4H, m), 2.10 (2H, t, *J* = 11.6 Hz), 2.37- (3H, m), 2.62 (3H, t, *J* = 7.2 Hz), 3.05 (2H, d, *J* = 11.6 Hz), 3.88 (2H, t, *J* = 7.2 Hz), 4.38 (1H, tt, *J* = 12.4, 4.4 Hz), 6.93-6.97 (1H, m), 7.03-7.13 (6H, m), 7.21-7.30 (5H, m).

### Example 42

### 1-(1-(4-(4-Fluorophenyl)butyl)piperidin-4-yl)-3-(4-phenylbutyl)-1H-benzo[d]imidazol-2(3H)-one (compound 42)

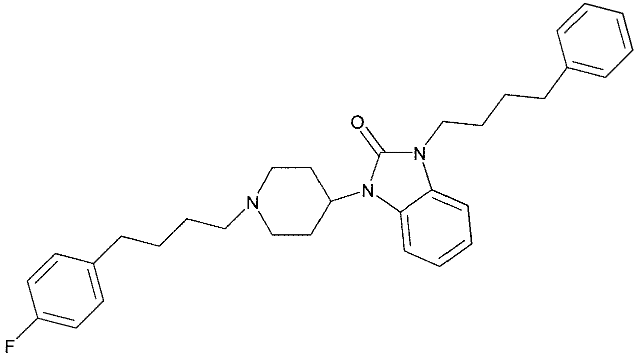

The title compound (Yield: 64%, 2 steps) was prepared according to the process in Example 16 by using 4-(4-fluorophenyl)butyl bromide instead of 5-phenylpentyl bromide.

¹H-NMR (400 MHz, CDCl₃) δ: 1.51-1.81 (10H, m), 2.10 (2H, t, *J* = 11.6 Hz), 2.38-2.47 (4H, m), 2.62 (2H, t, *J* = 7.6 Hz), 2.65 (2H, t, *J* = 7.6 Hz), 3.05 (2H, d, *J* = 11.6 Hz), 3.88 (2H, t, *J* = 7.6 Hz), 4.39 (1H, tt, *J* = 11.6, 4.0 Hz), 6.94-7.08 (5H, m), 7.12-7.19 (5H, m), 7.24-7.30 (3H, m).

### Example 43

### 1-(4-(4-Fluorophenyl)butyl)-3-(1-(4-phenylbutyl)piperidin-4-yl)-1H-benzo[d]imidazol-2(3H)-one (compound 43)

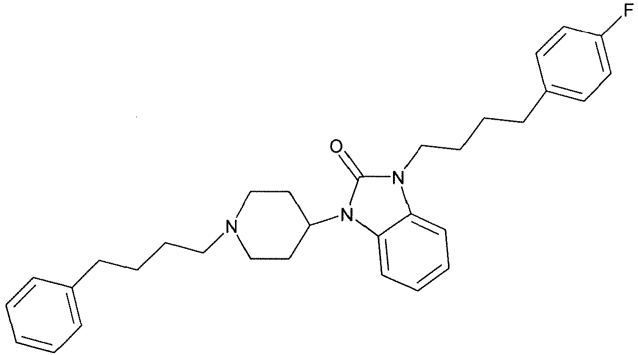

The title compound (Yield: 56%, 2 steps) was prepared according to the process in Example 16 by using the compound of Reference Example 13 and 4-phenylbutyl bromide instead of the compound of Reference Example 2 and 5-phenylpentyl bromide, respectively.

¹H-NMR (400 MHz, CDCl₃) δ: 1.60-1.71 (6H, m), 1.74-1.81 (4H, m), 2.17 (2H, br), 2.45-2.51 (4H, m), 2.62 (2H, t, *J* = 6.4 Hz), 2.66 (2H, t, *J* = 6.4 Hz), 3.11 (2H, d, *J* = 8.0 Hz), 3.88 (2H, t, *J =* 6.4 Hz), 4.42 (1H, t, *J* = 10.4 Hz), 6.91-6.96 (3H, m), 7.05-7.11 (4H, m), 7.17-7.20 (3H, m), 7.25-7.30 (2H, m), 7.35 (1H, br).

### Example 44

### 1-(4-(4-Fluorophenyl)butyl)-3-(1-(4-(4-fluorophenyl)butyl)piperidin-4-yl)-1H-benzo[d]imidazol-2(3H)-one (compound 44)

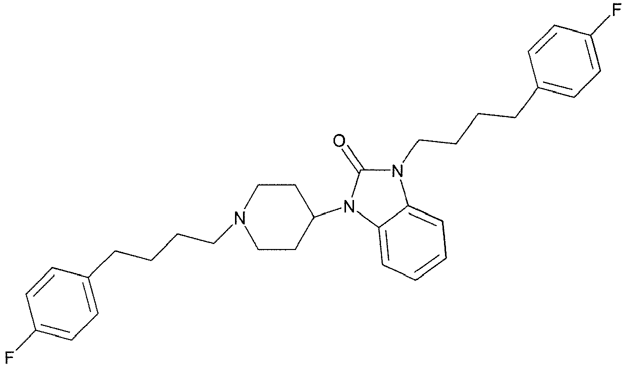

The title compound (Yield: 59%, 2 steps) was prepared according to the process in Example 16 by using the compound of Reference Example 13 and 4-(4-fluorophenyl)butyl bromide instead of the compound of Reference Example 2 and 5-phenylpentyl bromide, respectively.

¹H-NMR (400 MHz, CDCl₃) δ: 1.51-1.80 (10H, m), 2.10 (2H, t, *J* = 11.6 Hz), 2.38-2.41 (2H, m), 2.42 (2H, qd, *J* = 12.4, 3.6 Hz), 2.62 (4H, t, *J* = 7.6 Hz), 3.05 (2H, d, *J* = 11.6 Hz), 3.88 (2H, t, *J* = 7.6 Hz), 4.39 (1H, tt, *J =* 12.4, 3.6 Hz), 6.91-6.99 (5H, m), 7.03-7.06 (2H, m), 7.08-7.16 (4H, m), 7.28-7.31 (1H, m).

### Example 45

### 5-Fluoro-1-(4-phenylbutyl)-3-(1-(4-phenylbutyl)piperidin-4-yl)-1H-benzo[d]imidazol-2(3H)-one (compound 45)

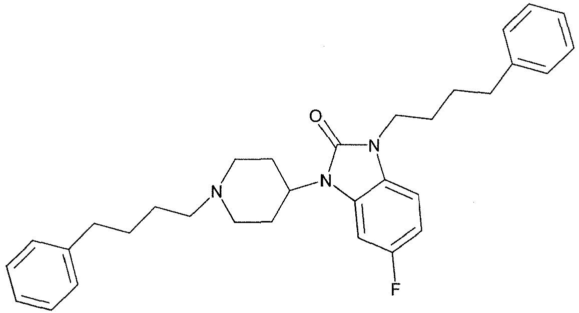

To a solution of the compound of Reference Example 14 (0.21 mmol) in dichloromethane (1 mL) was added trifluoroacetic acid (0.16 mL, 2.14 mmol) under Ar atmosphere and ice temperature, and the mixture was stirred at room temperature for 3 hours. After the reaction was completed, the solvent in the mixture was removed using a rotary evaporator, and N,N-dimethylamide (1 mL) was added to the resulting residue. To the mixture were then added 4-phenylbutyl bromide (92 mg, 0.43 mmol) and then potassium carbonate (147 mg, 1.07 mmol) at room temperature, and the mixture was stirred at room temperature for 22 hours. After the reaction was completed, the reaction solution was diluted with diethyl ether (1.5 mL) and water (1.5 mL) was further added thereto. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 5:1 to 1:2) to give the title compound (Yield: 42%, 2 steps).

¹H-NMR (400 MHz, CDCl₃) δ: 1.58-1.76 (8H, m), 2.09 (2H, t, *J* = 11.6 Hz), 2.33-2.42 (3H, m), 2.65 (3H, t, *J* = 7.2 Hz), 3.06 (2H, d, *J* = 11.6 Hz), 3.85 (2H, t, *J* = 7.2 Hz), 4.36 (1H, m), 6.73-6.83 (2H, m), 7.05-7.30 (11H, m).

### Example 46

### 5-Chloro-3-(4-phenylbutyl)-1-(1-(4-phenylbutyl)piperidin-4-yl)-1H-benzo[d]imidazol-2(3H)-one (compound 46)

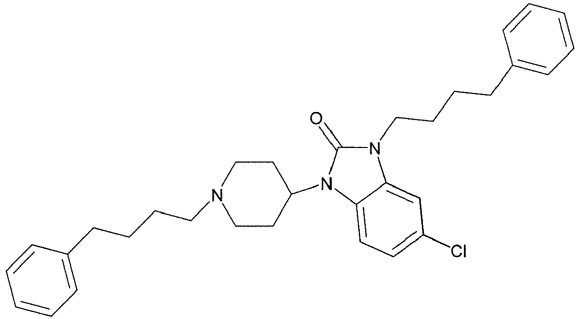

The title compound (Yield: 60%, 2 steps) was prepared according to a similar process to Example 45, except that the compound of Reference Example 15 was used instead of the compound of Reference Example 14.

¹H-NMR (500 MHz, CDCl₃) δ: 1.57-1.63 (2H, m), 1.68-1.83 (8H, m), 2.14 (2H, t, *J* = 11.0 Hz), 2.38-2.46 (4H, m), 2.70 (4H, d, *J =* 4.5 Hz), 3.10 (2H, d, *J* = 11.0 Hz), 3.87 (2H, t, *J* = 7.0 Hz), 4.38-4.44 (1H, m), 6.96 (1H, s), 7.03 (1H, d, *J* = 8.0 Hz), 7.20-7.33 (11H, m).

### Example 47

### 5-Chloro-1-(4-phenylbutyl)-3-(1-(4-phenylbutyl)piperidin-4-yl)-1H-benzo[d]imidazol-2(3H)-one (compound 47)

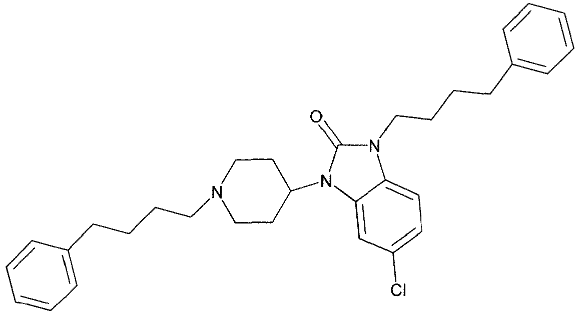

The title compound (Yield: 25%, 2 steps) was prepared according to a similar process to Example 45, except that the compound of Reference Example 16 was used instead of the compound of Reference Example 14.

¹H-NMR (400 MHz, CDCl₃) δ: 1.57-1.80 (11H, m), 2.10 (2H, t, *J* = 11.2 Hz), 2.32-2.43 (3H, m), 2.63-2.67 (4H, m), 3.80 (2H, d, *J* = 11.2 Hz), 3.85 (2H, t, *J* = 6.8 Hz), 4.33-4.40 (1H, m), 6.82 (1H, d, *J* = 8.4 Hz), 7.01 (1H, dd, *J* = 8.4, 1.6 Hz), 7.14-7.31 (11H, m).

### Example 48

### 4-Chloro-1-(4-phenylbutyl)-3-(1-(4-phenylbutyl)piperidin-4-yl)-1H-benzo[d]imidazol-2(3H)-one (compound 48)

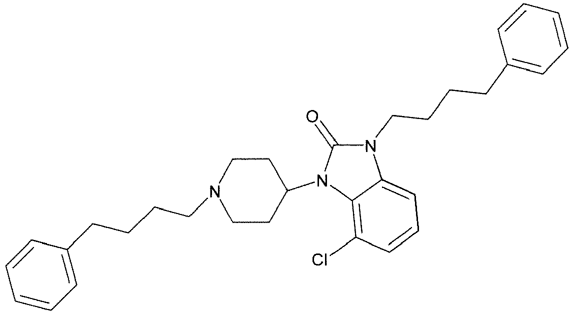

The title compound (Yield: 50%, 2 steps) was prepared according to a similar process to Example 45, except that the compound of Reference Example 17 was used instead of the compound of Reference Example 14.

¹H-NMR (400 MHz, CDCl₃) δ: 1.51-1.80 (10H, m), 2.05 (2H, t, *J* = 12.0 Hz), 2.39 (2H, t, *J =* 7.2 Hz), 2.61-2.66 (4H, m), 2.78 (2H, qd, *J* = 12.0, 3.6 Hz), 3.05 (2H, d, *J* = 12.0 Hz), 3.83 (2H, t, *J* = 7.2 Hz), 4.96 (1H, t, *J* = 12.0 Hz), 6.79 (1H, dd, *J* = 7.6, 1.2 Hz), 6.92-6.99 (2H, m), 7.14-7.19 (6H, m), 7.24-7.29 (4H, m).

### Example 49

### 1-(1-(4,4-Diphenylbutyl)piperidin-4-yl)-3-(4-phenylbutyl)-1H-benzo[d]imidazol-2(3H)-one (compound 49)

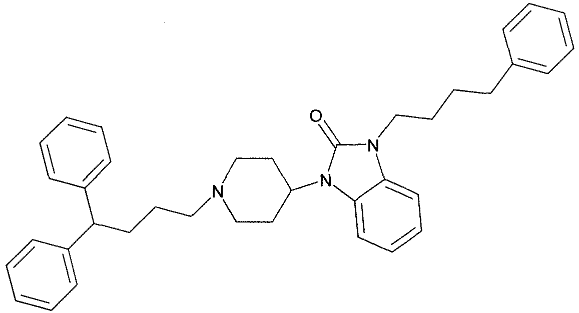

To a solution of the compound of Reference Example 2 (63 mg, 0.14 mmol) in dichloromethane (1.5 mL) was trifluoroacetic acid (0.11 mL, 1.40 mmol) under Ar atmosphere and ice temperature, and the mixture was stirred at room temperature for 3 hours. After the reaction was completed, the solvent in the mixture was removed using a rotary evaporator, and N,N-dimethylamide (1.5 mL) was added to the resulting residue. To the mixture were then added 4,4-diphenylbutyl bromide (0.28 mmol) and potassium carbonate (97 mg, 0.70 mmol) at room temperature, and the mixture was stirred at room temperature for 20 hours. After the reaction was completed, the reaction solution was diluted with diethyl ether (1.5 mL) and water (1.5 mL) was further added thereto. The organic layer was separated, and then the aqueous layer was extracted with diethyl ether (1.5 mL x 3) and combined with the separated organic layer. The organic layer was dried over anhydrous sodium sulfate, the solvent therein was removed using a rotary evaporator, and then the resulting residue was purified by silica gel column chromatography (n-hexane: ethyl acetate = 5:1 to 1:2) to give the title compound (Yield: 100%, 2 steps).

¹H-NMR (400 MHz, CDCl₃) δ: 1.45-1.53 (2H, m), 1.65-1.80 (6H, m), 2.01-2.12 (2H, m), 2.19-2.31 (2H, m), 2.36-2.42 (2H, m), 2.65 (2H, t, *J* = 7.2 Hz), 2.99 (2H, d, *J* = 12.0 Hz), 3.87 (2H, t, *J* = 7.2 Hz), 3.92 (1H, t, *J* = 7.2 Hz), 4.37 (1H, tt, *J* = 12.0, 4.8 Hz), 6.93-6.95 (1H, m), 7.00-7.05 (2H, m), 7.14-7.19 (5H, m), 7.24-7.30 (11H, m).

### Example 50

### 1-(1-(4,4-Bis(4-fluorophenyl)butyl)piperidin-4-yl)-3-(4-phenylbutyl)-1H-benzo[d]imidazol-2(3H)-one (compound 50)

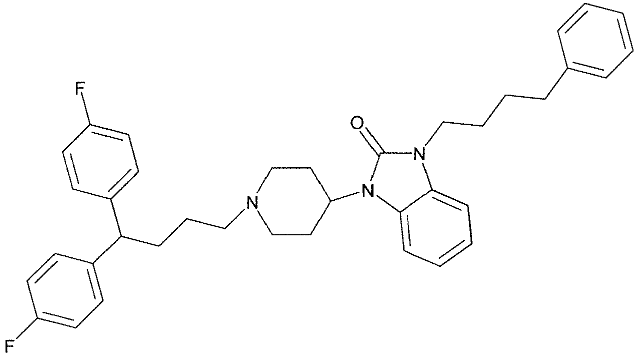

The title compound (Yield: 58%, 2 steps) was prepared according to a similar process to Example 49, except that 4,4-bis(4-fluorophenyl)butyl bromide was used instead of 4,4-diphenylbutyl bromide.

¹H-NMR (500 MHz, CDCl₃) δ: 1.43-1.52 (2H, m), 1.68-1.74 (2H, m), 1.76-1.83 (4H, m), 2.03-2.10 (4H, m), 2.40-2.45 (4H, m), 2.67 (2H, t, *J* = 7.5 Hz), 3.00 (2H, d, *J* = 12.0 Hz), 3.89 (2H, t, *J* = 7.5 Hz), 3.91 (1H, t, *J* = 7.5 Hz), 4.38 (1H, tt, *J* = 12.0, 4.0 Hz), 6.95-7.08 (7H, m), 7.16-7.21 (7H, m), 7.26-7.29 (3H, m).

### Test Example 1: Study on effect of inhibiting T-type calcium channels of the present compound (1)

The effect of the compound of the present invention on human Caᵥ3.2 T-type calcium channels was evaluated by whole-cell patch clamp method. As a control (vehicle), DMSO solution was used.

Human kidney cell-derived cell line, HEK 293 cells, in which human Caᵥ3.2 T-type calcium channels were forcibly expressed, were seeded into cell culture dishes using DMEM medium containing 10% fetal bovine serum and cultured at 37 °C under 5% CO₂ conditions. In the whole-cell patch clamp method, the following solutions were used as extracellular and intracellular solutions, the holding potential is set at -80 mV, and the barium current flowing as an inward current in a potential jump from -80 mV to -20 mV was measured with a patch clamp amplifier and AD converter (Axon Instruments). In order to eliminate the influence of high-threshold activated calcium channels, the value obtained by subtracting the current at 150 ms after the start of stimulation from the peak current was used for data analysis as the T channel current (T-current). The data analysis was performed using pClamp8 (Axon Instruments).

As for test compounds, each compound dissolved in extracellular solution was added directly to the dishes (direct addition method) or added to the dishes by perfusion (perfusion addition method).

### Extracellular solution:

97 mM N-methyl-D-glucamine (NMDG)
10 mM BaCl₂
10 mM 4-(2-hydroxyethyl)-1-piperazine ethanesulfonic acid (HEPES)
40 mM tetraethylammonium chloride (TEA-Cl)
5 mM glucose
(pH 7.4)

### Intracellular solution:

4 mM MgCl₂
140 mM CsCl
10 mM HEPES
5 mM glycol ether diamine tetraacetic acid (EGTA)
(pH 7.2)

The test results are shown in Fig. 1 for data obtained by the direct addition method and in Fig. 2 for data obtained by the perfusion addition method. Fig. 1 shows the percentages (%) of the T channel current values after the addition of pimozide and the present compounds (3 µM and 1 µM) relative to the T channel current value after the addition of vehicle (solvent, control group) (n = 3 to 10). Fig. 2 shows the percentage change in the T channel current values before the addition of vehicle and each test compound and after the addition of vehicle and each test compound (0.3 µM) (n = 3 to 8).

### Test Example 2: Study on effect of inhibiting T-type calcium channels of the present compound (2)

According to a similar method to the perfusion addition method of Test Example 1, the effect of each compound of the present invention on human Caᵥ3.2 T-type channels was evaluated. As the test compounds, pimozide at each concentration of 0.01 µM, 0.1 µM, 0.3 µM, 1.0 µM and 3.0 µM, compound 5, compound 14 and compound 15 were used and the test compounds were added by perfusion.

The test results are shown in Table 2. Table 2 shows the IC₅₀ values (µM) analyzed and calculated with Prism(GraphPad Software Inc.) from the percentage change in the T-channel current values before and after the addition of the test compound (n = 3 to 17).

**[Table 2]**

| | IC₅₀ value (µM) |
|---|---|
| Pimozide | 0.1573 |
| Compound 5 | 0.4613 |
| Compound 14 | 0.3375 |
| Compound 15 | 0.1534 |

The above test results showed that the compound of the present invention had the excellent effect of inhibiting T-type calcium channels.

### Test Example 3: Study on binding ability of the present compound to dopamine D₂ receptor

In this test, the binding ability of the present compound to dopamine D₂ receptor was studied to evaluate the dopamine D₂ receptor binding inhibitory effect of each compound. Compounds 4 to 7, 25, 26, 28, 30, 36 and 38 were used as the present compound, and pimozide and sulpiride were used as the comparative control.

The following reaction solution containing rat brain striatum homogenate was used, 5 µL of the present compound, sulpiride or pimozide was added to adjust the final concentration of each compound to 1 or 10 µM, and they were reacted for 90 minutes at room temperature. After the reaction was completed, the reaction solution was filtered through glass fiber filter, and the filter was washed and dried to measure the radioactivity thereof. The binding ability of each compound to dopamine D₂ receptor was evaluated by the percentage inhibition of the binding between dopamine D₂ receptor and [³H]-spiperone and the amount of [³H]-spiperone bond.

### Reaction Solution

Rat Brain Striatum Homogenate (200 µg protein/mL) 50 µL,
(Final concentration: 10 µg protein/tube)
[³H]-Spiperone (10nM) 5 µL
(Final concentration: 0.5 nM)
Buffer for assay 40 µL
(Composition: NaCl: 120 mM, KCl: 5 mM, MgCl₂: 1 mM, CaCl₂:1 mM, Tris-Cl: 50 mM (pH 7.4))

The test results are shown in Fig. 3 and Fig. 4. Fig. 3 shows the inhibition percentages of the present compound on the binding between dopamine D₂ receptor and [³H]-spiperone (n = 3). The inhibition percentage of each compound is represented by the ratio to the inhibition percentage of pimozide when the ratio of pimozide is defined as 100%. Fig. 4 shows the amounts of the specific binding of [³H]-spiperone to dopamine D₂ receptor after the addition of the solvent (total binding amount) and the addition of the test compound (1 µM and 10 µM) (n = 3).

As shown in the test results, the present compound did not inhibit the binding between dopamine D₂ receptor and [³H]-spiperone as compared to pimozide and sulpiride. Hence, it was shown that the present compound did not have any dopamine D₂ receptor binding inhibitory effect, that is, the present compound could reduce the dopamine D₂ receptor binding effect.

### Test Example 4: Study on reduction effect of the present compound on dopamine D₂ receptor blockade in vivo test

In this test, the catalepsy-inducing effect of the present compound was studied to evaluate whether the present compound can inhibit side effects caused by the dopamine D₂ receptor blockade. Compound 5 and compound 12 were used as the present compound and 2% DMSO solution was used as vehicle. Also, pimozide was used as the control drug.

16 male ddY mice were randomly divided into compound 5 administration group, compound 12 administration group, pimozide administration group and vehicle administration group (4 mice per group). The mice in each group were received the administration of 8 nmol (0.8 mM/10 µL) of compound 5, compound 12 and pimozide, and 10 µL of vehicle in the lateral ventricle. both forepaws of the mice were forced to place themselves on a horizontal iron bar (Height: 5 cm) and the time of keeping the posture (catalepsy) was measured to evaluate the presence or absence of catalepsy. The measurement was terminated when the forepaws touched the floor or when the mouse was on the bar.

The test results are shown in Fig. 5. Fig. 5 shows the keeping time (s) of catalepsy 30, 60, 90, 120, 150 and 180 minutes after vehicle and the test compound were administered.

As shown in Fig. 5, the pimozide administration group showed prolonged catalepsy with a peak time of 120 minutes after the administration, whereas catalepsy was induced in the compound 5 and compound 12 administration groups. Hence, it was shown that the present compound could reduce the dopamine D₂ receptor blockade and inhibit the resulting effects.

### Test Example 5: Study on effect of the present compound on Na₂S-induced pruritus

In this test, the antipruritic and analgesic effects of the present compound was studied using mouse Na₂S intrabuccal injection pruritus models. Compound 5 was used as the present invention and saline comprising 1% DMSO and 5% Tween 80 was used as vehicle.

Male ICR mice were received the intrabuccal injection of 3.8 pmol (300 pg/10 µL) of Na₂S and T-type calcium channel-independent pain was induced to prepare mouse Na₂S intrabuccal injection pruritus models. 18 mice were randomly divided into vehicle + saline administration group (control group), vehicle + Na₂S administration group and compound 5 (10 mg/kg) + Na₂S administration group (6 mice per group). The mice in each group were anesthetized with isoflurane and their right cheek hairs were shaved the day or a few days before the experiment. On the day of the experiment, the mice were placed in an acrylic observation cage with mirrors on all four sides and allowed to acclimate the environment for at least 30 minutes. After the acclimatization, Na₂S was intradermally administered to the shaved cheeks of the mice without anesthesia, and their subsequent behavior was recorded on video. Compound 5 (10 mg/kg) or vehicle (V, 10 mL/kg) was intraperitoneally administered 30 minutes before Na₂S was administered. At the later date, the video was replayed and the number of occurrences of the scratching bouts by the hind paws to the administration site (as pruritus response) and the wipes (wiping) by the forepaws (as pain response) was counted for 60 minutes to evaluate the antipruritic and analgesic effects of the present compound.

The test results are shown in Table 3 and Fig. 6. Table 3 shows the mean value ± standard error of the total counts of the scratching bouts and the wipes for 60 minutes after the intrabuccal administration on the right side, and Fig. 6 shows the graphs of the mean value ± standard error of the counts of the scratching bouts and the wipes for 10, 20, 30, 40, 50 and 60 minutes after the intrabuccal administration on the right side as well as the mean value ± standard error of the total counts thereof for 60 minutes after the administration.

**[Table 3]**

| | Scratching bouts (counts/60 minutes) (n) | Wipes (counts/60 minutes) (n) |
|---|---|---|
| Vehicle + Saline administration group (control group) | 23.7 ± 4.4 | 21.2 ± 2.4 |
| Vehicle + Na₂S administration group | 200.5 ± 37.5 | 80.3 ± 11.7 |
| Compound 5 (10 mg/kg) + Na₂S administration group | 36.2 ± 9.5 | 30.7 ± 4.0 |

As shown in the test results, pruritus and pain were not inhibited in the vehicle + Na₂S administration group, but pruritus and pain were significantly inhibited in the compound 5 administration group. Hence, it was shown that the present compound had the antipruritic and analgesic effects.

### Test Example 6: Study on effect of the present compound on Na₂S-induced pain

In this test, the analgesic effect of the present compound was studied using mouse Na₂S intraplantar injection pain models. Compound 5 was used as the present compound, and saline comprising 1% DMSO and 5% Tween 80 was used as vehicle.

Male ICR mice were received the intraplantar injection of 10 pmol/paw/10 µL of Na₂S and T-type calcium channel-dependent pain was induced to prepare mouse Na₂S intraplantar injection pain models. The mice were randomly divided into the vehicle + vehicle administration group (control group), vehicle + Na₂S administration group, compound 5 (1 mg/kg) + Na₂S administration group, compound 5 (3 mg/kg) + Na₂S administration group and compound 5 (10 mg/kg) + Na₂S administration group (n = 7 to 11). Vehicle (V, 10 mL/kg) or 1, 3 or 10 mg/kg of compound 5 was intraperitoneally administered 30 minutes before Na₂S was injected intraplantarlly in the hind paws of the mice.

Pain thresholds were measured every 15 minutes until 60 minutes after the administration of Na₂S by the von Frey method to evaluate the analgesic effect of the present compound.

The test results are shown in Table 4 and Fig. 7. Table 4 shows the mean value ± standard error of the pain thresholds (g) 15 and 30 minutes after the intraplantar injection in the hind paws of the mice in each group, and Fig. 7 shows the graph representing the mean value ± standard error of the pain thresholds (g) before, immediately after, and 15, 30, 45 and 60 minutes after the intraplantar injection in the hind paws of the mice.

**[Table 4]**

| | Pain Threshold (g) |
|---|---|
| Vehicle + Vehicle (Control group) | |
| 15 minutes after administration | 0.627 ± 0.018 |
| 30 minutes after administration | 0.582 ± 0.019 |

| Vehicle + Na₂S | |
|---|---|
| 15 minutes after administration | 0.160 ± 0.023 |
| 30 minutes after administration | 0.228 ± 0.025 |

| Compound 5 (1 mg/kg) + Na₂S | |
|---|---|
| 15 minutes after administration | 0.317 ± 0.082 |
| 30 minutes after administration | 0.296 ± 0.078 |

| Compound 5 (3 mg/kg) + Na₂S | |
|---|---|
| 15 minutes after administration | 0.347 ± 0.079 |
| 30 minutes after administration | 0.417 ± 0.064 |

| Compound 5 (10 mg/kg) + Na₂S | |
|---|---|
| 15 minutes after administration | 0.514 ± 0.054 |
| 30 minutes after administration | 0.604 ± 0.049 |

As shown in the test results, pain was not inhibited in the vehicle + Na₂S administration group, but pain was inhibited in the compound 5 administration group. Hence, it was shown that the present compound produced analgesic effects.

### Test Example 7: Study on antipruritic effect of the present compound

In this test, the antipruritic effect of the present compound was studied using mouse intrabuccal injection pruritus models in which histamine or chloroquine is intra-buccally administered as a pruritogen. Compound 5 and compound 38 were used as the present compound, and saline comprising 1% DMSO and 5% Tween 80 was used as control. Compound 5 and compound 38 were dissolved into saline comprising 1% DMSO and 5% Tween 80 to adjust the concentration of each compound. Also, male ICR mice with a body weight of 23 to 40 g were used as experimental animals and the animals were freely given tap water and solid feed and was kept under 12-hour light/dark cycle at room temperature of about 24°C.

According to the method in Shimada SG & LaMotte RH (2008) Behavioral differentiation between itch and pain in mouse. Pain 139 (3): 681-687, histamine or chloroquine, which is a pruritogen, was dissolved into saline and the solution was administered into the buccal skin of each male ICR mouse and pruritus was induced to prepare mouse intrabuccal injection pruritus models. The mice were randomly divided into saline comprising 1% DMSO and 5% Tween 80 (Solvent 1) + saline (Solvent 2) administration group (control group), Solvent 1 + histamine administration group, compound 5 (10 mg/kg) + histamine administration group, Solvent 1 + chloroquine administration group, compound 5 (10 mg/kg) + chloroquine administration group, compound 38 (1 mg/kg) + chloroquine administration group,compound 38 (3 mg/kg) + chloroquine administration group and compound 38 (10 mg/kg) + chloroquine administration group (n = 6 to 10). The mice in each group were anesthetized with isoflurane and their right cheek hairs were shaved a few days before the experiment. On the day of the experiment, the mice were placed in an acrylic observation cage (10 x 14 x 30 cm) with mirrors on all four sides and allowed to acclimate the environment for at least 30 minutes. After the acclimatization, saline in a volume of 10µL/site, histamine in a volume of 300 µL/site or chloroquine in a volume of 100 µL/site was intradermally administered to the shaved cheeks of the mice without anesthesia, and their subsequent behavior was recorded on video for 60 minutes. Compound 5 (10mg/kg), compound 38 (1 mg/kg, 3 mg/kg or 10 mg/kg) or Solvent 1 (10 mL/kg) was intraperitoneally administered 30 minutes before saline or the pruritogen was administered. At the later date, the video was replayed and the number of occurrences of the scratching bouts by the hind paws to the administration site (as pruritus response) was counted for 60 minutes to evaluate the antipruritic effect of the present compound.

The test results are shown in Table 5 to Table 7 and Fig. 8 to Fig. 10. Table 5 and Fig. 8 show the mean value ± standard error (the total (n)) of the total counts of the scratching bouts by the hind paws to the administration site for 60 minutes after the administration in control group, Solvent 1 + histamine administration group and compound 5 (10 mg/kg) + histamine administration group and the graph thereof, and Table 6 and Fig. 9 show the mean value ± standard error (the total (n)) of the total counts of the scratching bouts by the hind paws to the administration site for 60 minutes after the intrabuccal administration on the right side in control group, Solvent 1+chloroquine administration group, compound 5(10mg/kg)+chloroquine administration group and the graph thereof, and Table 7 and Fig. 10 show the mean value ± standard error (the total (n)) of the total counts of the scratching bouts by the hind paws to the administration site for 60 minutes after the intrabuccal administration on the right side in control group,Solvent 1+chloroquine administration group, compound 38 (1 mg/kg) + chloroquine administration group, compound 38 (3 mg/kg) + chloroquine administration group and compound 38 (10 mg/kg) + chloroquine administration group and the graph thereof.

**[Table 5]**

| | Scratching bouts (counts/60 minutes) (n) |
|---|---|
| Solvent 1 + Solvent 2 administration group (control group) | 12.0 ± 2.2 (6) |
| Solvent 1 + histamine administration group | 56.4 ± 6.0 (7) |
| Compound 5 (10 mg/kg) + histamine administration group | 13.3 ± 5.0 (7) |

**[Table 6]**

| | Scratching bouts (counts/60 minutes) (n) |
|---|---|
| Solvent 1 + Solvent 2 administration group (control group) | 12.6 ± 4.3 (10) |
| Solvent 1 + chloroquine administration group | 61.4 ± 11.6 (10) |
| Compound 5 (10 mg/kg) + chloroquine administration group | 24.9 ± 5.5 (10) |

**[Table 7]**

| | Scratching bouts (counts/60 minutes) (n) |
|---|---|
| Solvent 1 + Solvent 2 administration group (control group) | 4.4 ± 1.4 (7) |
| Solvent 1 + chloroquine administration group | 66.8 ± 10.3 (8) |
| Compound 38 (1 mg/kg) + chloroquine administration group | 73.0 ± 10.5 (6) |
| Compound 38 (3 mg/kg) + chloroquine administration group | 50.7 ± 9.7 (6) |
| Compound 38 (10 mg/kg) + chloroquine administration group | 28.8 ± 10.5 (8) |

As shown in the test results, compound 5 (10mg/k g) strongly inhibited the histamine-dependent scratching bouts and the histamine-independent scratching bouts (Tables 5 to 6 and Figs. 8 to 9), and compound 38 strongly inhibited the histamine-independent scratching bouts in a dose of 10 mg/kg (Table 7 and Fig. 10). Hence, it was shown that the present compound had the antipruritic effect.

The present compound strongly inhibited both histamine-dependent pruritus and histamine-independent pruritus. As a result, it was suggested that the present compound could be used as a medicament for treating intractable pruritus.

### INDUSTRIAL APPLICABILITY

According to the present invention, a new benzimidazolone compound with the excellent effect of inhibiting T-type calcium channels can be provided. Also, the present compound can be used as a new medicament for treating a disease associated with the activation of T-type calcium channels, for example, pain and pruritus, which can reduce the effect of inhibiting D₂ receptor and inhibit the risk of side effects.

## Claims

1. A compound of formula (I): 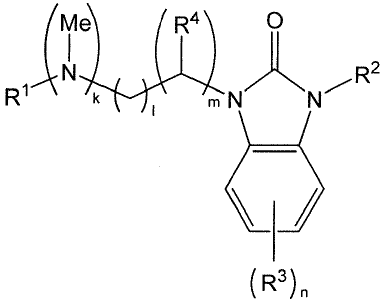 or a pharmaceutically acceptable salt thereof, wherein
R¹ is C₁₋₁₀ alkyl, C(O)-C₁₋₁₀ alkyl, C₁₋₆ alkylene-R^{1a}, C(O)-C₁₋₆ alkylene-R^{1a}, C₁₋₆ alkylene-CHR^{1b}R^{1b} or C(O)-C₁₋₆ alkylene-CHR^{1b}R^{1b} wherein the alkyl and alkylene may be optionally substituted with the same or different one or more groups selected from the group consisting of halogen, amino and hydroxy at any replaceable positions;
R² is hydrogen, C₁₋₁₀ alkyl, C(O)-C₁₋₁₀ alkyl, C₁₋₆ alkylene-R^{2a}, C(O)-C₁₋₆ alkylene-R^{2a}, C₁₋₆ alkylene-CHR^{2b}R^{2b} or C(O)-C₁₋₆ alkylene-CHR^{2b}R^{2b} wherein the alkyl and alkylene may be optionally substituted with the same or different one or more groups selected from the group consisting of halogen, amino and hydroxy at any replaceable positions;
R^{1a} and R^{2a} are each independently selected from the group consisting of C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl and optionally-substituted 5- to 10-membered heteroaryloxy;
R^{1b} and R^{2b} are each independently selected from the group consisting of optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl;
R³ is each independently selected from the group consisting of halogen, C₁₋₆ alkyl, C₁₋₆ haloalkyl, amino and hydroxy;
R⁴ is C₁₋₆ alkyl, C₁₋₆ haloalkyl, C₁₋₆ alkoxy, C₂₋₆ alkenyl or C₂₋₆ alkynyl;
k is 0 or 1;
1 is 0 to 2;
m is 0 or 1, provided that when k is 1 and m is 1, the terminal carbon atom of R⁴ may be combined with the carbon atom bonded to N in the parenthesis for k to form a 4- to 6-membered nitrogen-containing heterocycle which may be optionally substituted with the same or different one or more groups selected from the group consisting of halogen, hydroxy, C₁₋₆ alkyl and C₁₋₆ alkoxy at any replaceable positions; and
n is 0 to 4,
provided that the following compounds are excluded:
1-[1-[4,4-bis(4-fluorophenyl)butyl]-4-piperidinyl]-1,3-dihydro-2H-benzimidazol-2-one;
1-(1-benzylpiperidin-4-yl)-1H-benzo[d]imidazol-2(3H)-one;
1-(1-(2-phenylethyl)piperidin-4-yl)-1H-benzo[d]imidazol-2(3H)-one;
1-(1-(3-phenylpropyl)piperidin-4-yl)-1H-benzo[d]imidazol-2(3H)-one;
1-(1-(4-phenylbutyl)piperidin-4-yl)-1H-benzo[d]imidazol-2(3H)-one.

2. The compound according to claim 1 or a pharmaceutically acceptable salt thereof, wherein the formula (I) is formula (Ia) : 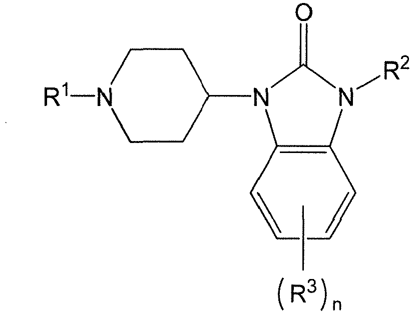 wherein R¹, R², R³ and n are as defined in claim 1.

3. The compound according to claim 1 or a pharmaceutically acceptable salt thereof, wherein the formula (I) is formula (Ib) : 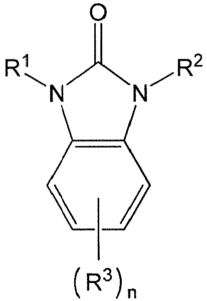 wherein R¹, R², R³ and n are as defined in claim 1.

4. The compound according to any one of claims 1 to 3 or a pharmaceutically acceptable salt thereof, wherein R¹ is C₁₋₆ alkyl, C(O)-C₁₋₆ alkyl, C₁₋₆ alkylene-R^{1a}, C(O)-C₁₋₆ alkylene-R^{1a}, C₁₋₄ alkylene-CHR^{1b}R^{1b} or C(O)-C₁₋₄ alkylene-CHR^{1b}R^{1b} wherein the alkyl and alkylene may be optionally substituted with the same or different one or more groups selected from the group consisting of halogen, amino and hydroxy at any replaceable positions;
R² is C₁₋₆ alkyl, C(O)-C₁₋₆ alkyl, C₁₋₆ alkylene-R^{2a}, C(O)-C₁₋₆ alkylene-R^{2a}, C₁₋₄ alkylene-CHR^{2b}R^{2b} or C(O)-C₁₋₄ alkylene-CHR^{2b}R^{2b} wherein the alkyl and alkylene may be optionally substituted with the same or different one or more groups selected from the group consisting of halogen, amino and hydroxy at any replaceable positions;
R^{1a} and R^{2a} are each independently selected from the group consisting of C₃₋₈ cycloalkyl, C₃₋₈ cycloalkoxy, C₆₋₁₀ aryl and C₆₋₁₀ aryloxy wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions;
R^{1b} and R^{2b} are each independently selected from the group consisting of C₃₋₈ cycloalkyl and C₆₋₁₀ aryl wherein each of said groups may be optionally substituted with the same or different one or more groups selected from the group consisting of halogen, C₁₋₆ alkyl, C₁₋₆ alkoxy, C₁₋₆ haloalkyl, amino and hydroxy at any replaceable positions;
R³ is each independently selected from the group consisting of halogen, amino and hydroxy; and
n is 0 or 1.

5. The compound according to any one of claims 1 to 4 or a pharmaceutically acceptable salt thereof, wherein R¹ is C₁₋₆ alkyl, C₁₋₆ alkylene-R^{1a}, C(O)-C₁₋₆ alkylene-R^{1a} or C₁₋₄ alkylene-CHR^{1b}R^{1b} wherein the alkyl and alkylene may be optionally substituted with the same or different one or more groups selected from the group consisting of halogen, amino and hydroxy at any replaceable positions;
R^{1a} is C₃₋₆ cycloalkyl, C₃₋₆ cycloalkyloxy, phenyl or phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; and
R^{1b} is each independently selected from the group consisting of C₃₋₆ cycloalkyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions.

6. The compound according to any one of claims 1 to 5 or a pharmaceutically acceptable salt thereof, wherein R¹ is C₁₋₆ alkyl, C₁₋₆ alkylene-R^{1a}, C(O)-C₁₋₆ alkylene-R^{1a} or C₁₋₄ alkylene-CHR^{1b}R^{1b} wherein the alkyl and alkylene may be optionally substituted with the same or different one or more groups selected from the group consisting of halogen, amino and hydroxy at any replaceable positions;
R^{1a} is selected from the group consisting of C₅₋₆ cycloalkyl, C₅₋₆ cycloalkyloxy, phenyl and phenoxy wherein each of said groups may be optionally substituted with the same or different one or more groups selected from the group consisting of halogen, C₁₋₅ alkyl, C₁₋₆ alkoxy and C₁₋₆ haloalkyl at any replaceable positions; and
R^{1b} is each independently selected from the group consisting of C₅₋₆ cycloalkyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions.

7. The compound according to any one of claims 1 to 6 or a pharmaceutically acceptable salt thereof, wherein R¹ is C₁₋₆ alkyl, C₁₋₆ alkylene-R^{1a}, C(O)-C₁₋₆ alkylene-R^{1a}, C₁₋₄ alkylene-CHR^{1b}R^{1b}; and
R^{1a} is cyclohexyl, phenyl or phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; and
R^{1b} is each independently selected from the group consisting of cyclohexyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions.

8. The compound according to any one of claims 1 to 7 or a pharmaceutically acceptable salt thereof, wherein R¹ is selected from the group consisting of C₁₋₅ alkyl, 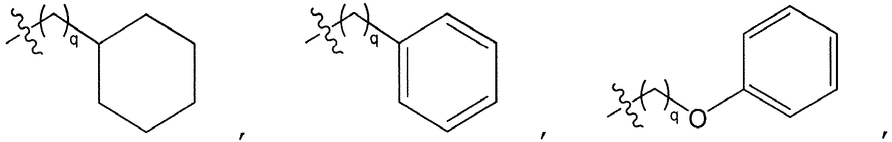 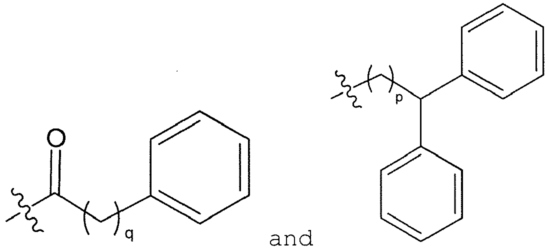 wherein p is 1 to 4 and q is 1 to 5 and wherein each of said groups may be optionally substituted with the same or different one or more groups selected from the group consisting of halogen and C₁₋₆ alkoxy at any replaceable positions.

9. The compound according to any one of claims 1 to 8 or a pharmaceutically acceptable salt thereof, wherein R¹ is selected from the group consisting of butyl, 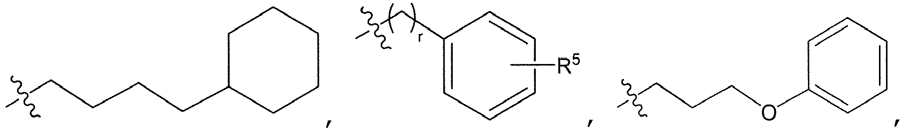 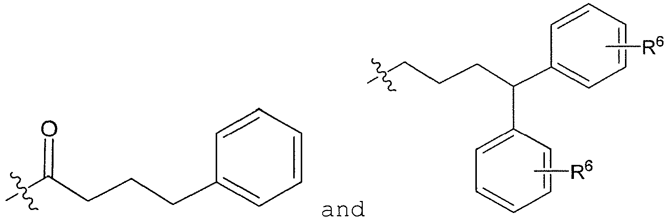 wherein R⁵ is hydrogen, halogen or methoxy, R⁶ is each independently hydrogen or halogen, and r is 1 to 5.

10. The compound according to any one of claims 1 to 9 or a pharmaceutically acceptable salt thereof, wherein R² is C₁₋₆ alkyl, C₁₋₆ alkylene-R^{2a}, C(O)-C₁₋₆ alkylene-R^{2a} or C₁₋₄ alkylene-CHR^{2b}R^{2b};
R^{2a} is selected from the group consisting of C₃₋₆ cycloalkyl, C₃₋₆ cycloalkyloxy, phenyl and phenoxy wherein each of said groups may be optionally substituted with the same or different one or more groups selected from the group consisting of halogen, C₁₋₆ alkyl, C₁₋₆ alkoxy and C₁₋₆ haloalkyl at any replaceable positions; and
R^{2b} is each independently selected from the group consisting of C₃₋₆ cycloalkyl and phenyl wherein each of said groups may be optionally substituted with the same or different one or more groups selected from the group consisting of halogen, C₁₋₆ alkyl, C₁₋₆ alkoxy and C₁₋₆ haloalkyl at any replaceable positions.

11. The compound according to any one of claims 1 to 10 or a pharmaceutically acceptable salt thereof, wherein R² is C₁₋₆ alkyl, C₁₋₆ alkylene-R^{2a} or C₁₋₄ alkylene-CHR^{2b}R^{2b};
R^{2a} is selected from the group consisting of C₅₋₆ cycloalkyl, C₅₋₆ cycloalkyloxy, phenyl and phenoxy wherein each of said groups may be optionally substituted with halogen or C₁₋₆ alkoxy at any replaceable positions; and
R^{2b} is each independently selected from the group consisting of C₅₋₆ cycloalkyl and phenyl wherein each of said groups may be optionally substituted with halogen or C₁₋₆ alkoxy at any replaceable positions.

12. The compound according to any one of claims 1 to 11 or a pharmaceutically acceptable salt thereof, wherein R² is C₁₋₆ alkyl, C₁₋₆ alkylene-R^{2a} or C₁₋₄ alkylene-CHR^{2b}R^{2b};
R^{2a} is cyclohexyl, phenyl or phenoxy wherein each of said groups may be optionally substituted with halogen or C₁₋₆ alkoxy at any replaceable positions; and
R^{2b} is each independently selected from the group consisting of cyclohexyl and phenyl wherein each of said groups may be optionally substituted with halogen or C₁₋₆ alkoxy at any replaceable positions.

13. The compound according to any one of claims 1 to 12 or a pharmaceutically acceptable salt thereof, wherein R² is selected from the group consisting of C₁₋₅ alkyl, 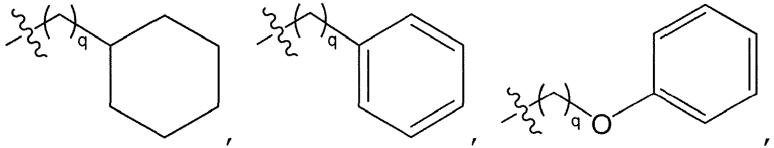 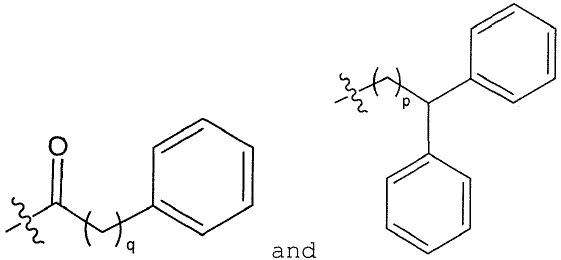 wherein p and q are as defied in claim 8 and wherein each of said groups may be optionally substituted with the same or different one or more groups selected from the group consisting of halogen and C₁₋₆ alkoxy at any replaceable positions.

14. The compound according to any one of claims 1 to 13 or a pharmaceutically acceptable salt thereof, wherein R² is butyl, 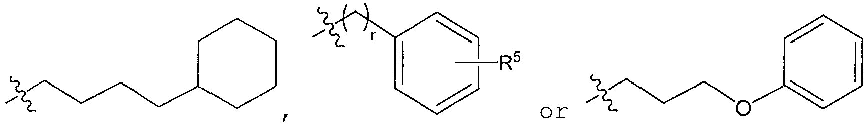 wherein R⁵ is hydrogen, halogen or methoxy, and r is 1 to 5.

15. The compound according to claim 1 which selected from:
1-(4-phenylbutyl)-3-(1-(4-phenylbutyl)piperidin-4-yl)-1H-benzo[d]imidazol-2(3H)-one (compound 5),
1-butyl-3-(1-(4-phenylbutyl)piperidin-4-yl)-1H-benzo[d]imidazol-2(3H)-one (compound 6),
1-(4-cyclohexylbutyl)-3-(1-(4-phenylbutyl)piperidin-4-yl)-1H-benzo[d]imidazol-2(3H)-one (compound 7),
1-(1-(4-cyclohexylbutyl)piperidin-4-yl)-1H-benzo[d]imidazol-2(3H)-one (compound 8),
1-(4-cyclohexylbutyl)-3-(1-(4-cyclohexylbutyl)piperidin-4-yl)-1H-benzo[d]imidazol-2(3H)-one (compound 9),
1,3-bis(4-phenylbutyl)-1H-benzo[d]imidazol-2(3H)-one (compound 10),
1-(4-cyclohexylbutyl)-3-(4-phenylbutyl)-1H-benzo[d]imidazol-2(3H)-one (compound 11),
1-(1-(4-cyclohexylbutyl)piperidin-4-yl)-3-(4-phenylbutyl)-1H-benzo[d]imidazol-2(3H)-one (compound 12),
1-(1-butylpiperidin-4-yl)-3-(4-phenylbutyl)-1H-benzo[d]imidazol-2(3H)-one (compound 13),
1-(4-phenylbutyl)-3-(1-(3-phenylpropyl)piperidin-4-yl)-1H-benzo[d]imidazol-2(3H)-one (compound 14),
1-(1-(4-phenylbutanoyl)piperidin-4-yl)-3-(4-phenylbutyl)-1H-benzo[d]imidazol-2(3H)-one (compound 15),
1-(4-phenylbutyl)-3-(1-(5-phenylpentyl)piperidin-4-yl)-1H-benzo[d]imidazol-2(3H)-one (compound 16),
1-(1-(4-phenylbutyl)piperidin-4-yl)-3-(3-phenylpropyl)-1H-benzo[d]imidazol-2(3H)-one (compound 17),
1-(1-(4-phenylbutyl)piperidin-4-yl)-3-(5-phenylpentyl)-1H-benzo[d]imidazol-2(3H)-one (compound 18),
1-(1-benzylpiperidin-4-yl)-3-(4-phenylbutyl)-1H-benzo[d]imidazol-2(3H)-one (compound 19),
1-benzyl-3-(1-(4-phenylbutyl)piperidin-4-yl)-1H-benzo[d]imidazol-2(3H)-one (compound 20),
1-(1-(3-phenoxypropyl)piperidin-4-yl)-3-(4-phenylbutyl)-1H-benzo[d]imidazol-2(3H)-one (compound 21),
1-(3-phenoxypropyl)-3-(1-(4-phenylbutyl)piperidin-4-yl)-1H-benzo[d]imidazol-2(3H)-one (compound 22),
1-(3-phenoxypropyl)-3-(1-(3-phenoxypropyl)piperidin-4-yl)-1H-benzo[d]imidazol-2(3H)-one (compound 23),
1-(1-(4-(2-methoxyphenyl)butyl)piperidin-4-yl)-3-(4-phenylbutyl)-1H-benzo[d]imidazol-2(3H)-one (compound 24),
1-(4-(2-methoxyphenyl)butyl)-3-(1-(4-phenylbutyl)piperidin-4-yl)-1H-benzo[d]imidazol-2(3H)-one (compound 25),
1-(4-(2-methoxyphenyl)butyl)-3-(1-(4-(2-methoxyphenyl)butyl)piperidin-4-yl)-1H-benzo[d]imidazol-2(3H)-one (compound 26),
1-(1-(4-(2-chlorophenyl)butyl)piperidin-4-yl)-3-(4-phenylbutyl)-1H-benzo[d]imidazol-2(3H)-one (compound 27),
1-(4-(2-chlorophenyl)butyl)-3-(1-(4-phenylbutyl)piperidin-4-yl)-1H-benzo[d]imidazol-2(3H)-one (compound 28),
1-(4-(2-chlorophenyl)butyl)-3-(1-(4-(2-chlorophenyl)butyl)piperidin-4-yl)-1H-benzo[d]imidazol-2(3H)-one (compound 29),
1-(1-(4-(2-fluorophenyl)butyl)piperidin-4-yl)-3-(4-phenylbutyl)-1H-benzo[d]imidazol-2(3H)-one (compound 30),
1-(4-(2-fluorophenyl)butyl)-3-(1-(4-phenylbutyl)piperidin-4-yl)-1H-benzo[d]imidazol-2(3H)-one (compound 31),
1-(4-(2-fluorophenyl)butyl)-3-(1-(4-(2-fluorophenyl)butyl)piperidin-4-yl)-1H-benzo[d]imidazol-2(3H)-one (compound 32),
1-(1-(4-(3-chlorophenyl)butyl)piperidin-4-yl)-3-(4-phenylbutyl)-1H-benzo[d]imidazol-2(3H)-one (compound 33),
1-(4-(3-chlorophenyl)butyl)-3-(1-(4-phenylbutyl)piperidin-4-yl)-1H-benzo[d]imidazol-2(3H)-one (compound 34),
1-(4-(3-chlorophenyl)butyl)-3-(1-(4-(3-chlorophenyl)butyl)piperidin-4-yl)-1H-benzo[d]imidazol-2(3H)-one (compound 35),
1-(1-(4-(3-fluorophenyl)butyl)piperidin-4-yl)-3-(4-phenylbutyl)-1H-benzo[d]imidazol-2(3H)-one (compound 36),
1-(4-(3-fluorophenyl)butyl)-3-(1-(4-phenylbutyl)piperidin-4-yl)-1H-benzo[d]imidazol-2(3H)-one (compound 37),
1-(4-(3-fluorophenyl)butyl)-3-(1-(4-(3-fluorophenyl)butyl)piperidin-4-yl)-1H-benzo[d]imidazol-2(3H)-one (compound 38),
1-(1-(4-(4-chlorophenyl)butyl)piperidin-4-yl)-3-(4-phenylbutyl)-1H-benzo[d]imidazol-2(3H)-one (compound 39),
1-(4-(4-chlorophenyl)butyl)-3-(1-(4-phenylbutyl)piperidin-4-yl)-1H-benzo[d]imidazol-2(3H)-one (compound 40),
1-(4-(4-chlorophenyl)butyl)-3-(1-(4-(4-chlorophenyl)butyl)piperidin-4-yl)-1H-benzo[d]imidazol-2(3H)-one (compound 41),
1-(1-(4-(4-fluorophenyl)butyl)piperidin-4-yl)-3-(4-phenylbutyl)-1H-benzo[d]imidazol-2(3H)-one (compound 42),
1-(4-(4-fluorophenyl)butyl)-3-(1-(4-phenylbutyl)piperidin-4-yl)-1H-benzo[d]imidazol-2(3H)-one (compound 43),
1-(4-(4-fluorophenyl)butyl)-3-(1-(4-(4-fluorophenyl)butyl)piperidin-4-yl)-1H-benzo[d]imidazol-2(3H)-one (compound 44),
5-fluoro-1-(4-phenylbutyl)-3-(1-(4-phenylbutyl)piperidin-4-yl)-1H-benzo[-d]imidazol-2(3H)-one (compound 45),
5-chloro-3-(4-phenylbutyl)-1-(1-(4-phenylbutyl)piperidin-4-yl)-1H-benzo[d]imidazol-2(3H)-one (compound 46),
5-chloro-1-(4-phenylbutyl)-3-(1-(4-phenylbutyl)piperidin-4-yl)-1H-benzo[d]imidazol-2(3H)-one (compound 47),
4-chloro-1-(4-phenylbutyl)-3-(1-(4-phenylbutyl)piperidin-4-yl)-1H-benzo[d]imidazol-2(3H)-one (compound 48),
1-(1-(4,4-diphenylbutyl)piperidin-4-yl)-3-(4-phenylbutyl)-1H-benzo[d]imidazol-2(3H)-one(compound 49), or
1-(1-(4,4-bis(4-fluorophenyl)butyl)piperidin-4-yl)-3-(4-phenylbutyl)-1H-benzo[d]imidazol-2(3H)-one (compound 50), or a pharmaceutically acceptable salt thereof.

16. A pharmaceutical composition comprising a compound of formula (I): 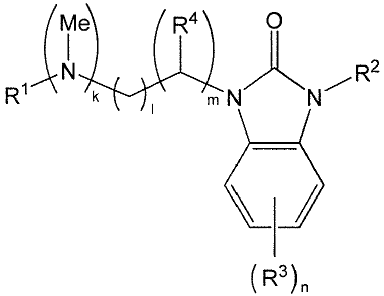 or a pharmaceutically acceptable salt thereof, wherein
R¹ is C₁₋₁₀ alkyl, C(O)-C₁₋₁₀ alkyl, C₁₋₆ alkylene-R^{1a}, C (0) -C₁₋₆ alkylene-R^{1a}, C₁₋₆ alkylene-CHR^{1b}R^{1b} or C (O)-C₁₋₆ alkylene-CHR^{1b}R^{1b} wherein the alkyl and alkylene may be optionally substituted with the same or different one or more groups selected from the group consisting of halogen, amino and hydroxy at any replaceable positions;
R² is hydrogen, C₁₋₁₀ alkyl, C(O)-C₁₋₁₀ alkyl, C₁₋₆ alkylene-R^{2a}, C(O)-C₁₋₆ alkylene-R^{2a}, C₁₋₆ alkylene-CHR^{2b}R^{2b} or C(O)-C₁₋₆ alkylene-CHR^{2b}R^{2b} wherein the alkyl and alkylene may be optionally substituted with the same or different one or more groups selected from the group consisting of halogen, amino and hydroxy at any replaceable positions;
R^{1a} and R^{2a} are each independently selected from the group consisting of C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl and optionally-substituted 5- to 10-membered heteroaryloxy;
R^{1b} and R^{2b} are each independently selected from the group consisting of optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl;
R³ is each independently selected from the group consisting of halogen, C₁₋₆ alkyl, C₁₋₆ haloalkyl, amino and hydroxy;
R⁴ is C₁₋₆ alkyl, C₁₋₆ haloalkyl, C₁₋₆ alkoxy, C₂₋₆ alkenyl or C₂₋₆ alkynyl;
k is 0 or 1;
1 is 0 to 2;
m is 0 or 1, provided that when k is 1 and m is 1, the terminal carbon atom of R⁴ may be combined with the carbon atom bonded to N in the parenthesis for k to form a 4- to 6-membered nitrogen-containing heterocycle which may be optionally substituted with the same or different one or more groups selected from the group consisting of halogen, hydroxy, C₁₋₆ alkyl and C₁₋₆ alkoxy at any replaceable positions; and
n is 0 to 4,
provided that 1-[1-[4,4-bis(4-fluorophenyl)butyl]-4-piperidinyl]-1,3-dihydro-2H-benzimidazol-2-one is excluded, and a pharmaceutically acceptable carrier.

17. The pharmaceutical composition according to claim 16 for treating a disease associated with the activation of T-type calcium channels.

18. The pharmaceutical composition according to claim 17, wherein the disease is selected from pain, epilepsy, essential tremor, schizophrenia, Parkinson's disease, manic depression, bipolar disorder, depression, anxiety, dementia, Huntington's disease, sleep disorder, stroke, pruritus, atopic dermatitis, hyperaldosteronism, edema, ischemic heart disease, age-related macular degeneration, cancer, diabetes, sterility, sexual dysfunction, arrhythmia, hypertension, renal disease or overactive bladder.

19. The pharmaceutical composition according to claim 17 or 18, wherein the disease is pain.

20. The pharmaceutical composition according to claim 17 or 18, wherein the disease is pruritus.

21. A T-type calcium channel inhibitor comprising a compound of formula (I): 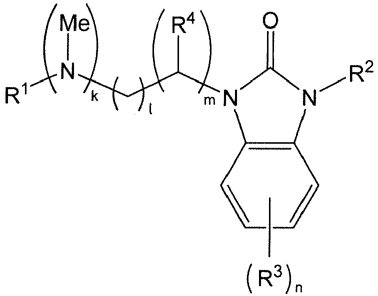 or a pharmaceutically acceptable salt thereof, wherein
R¹ is C₁₋₁₀ alkyl, C(O)-C₁₋₁₀ alkyl, C₁₋₆ alkylene-R^{1a}, C(O)-C₁₋₆ alkylene-R^{1a}, C₁₋₆ alkylene-CHR^{1b}R^{1b} or C(O)-C₁₋₆ alkylene-CHR^{1b}R^{1b} wherein the alkyl and alkylene may be optionally substituted with the same or different one or more groups selected from the group consisting of halogen, amino and hydroxy at any replaceable positions;
R² is hydrogen, C₁₋₁₀ alkyl, C(O)-C₁₋₁₀ alkyl, C₁₋₆ alkylene-R^{2a}, C(O)-C₁₋₆ alkylene-R^{2a}, C₁₋₆ alkylene-CHR^{2b}R^{2b} or C(O)-C₁₋₆ alkylene-CHR^{2b}R^{2b} wherein the alkyl and alkylene may be optionally substituted with the same or different one or more groups selected from the group consisting of halogen, amino and hydroxy at any replaceable positions;
R^{1a} and R^{2a} are each independently selected from the group consisting of C₁₋₆ alkoxy, optionally-substituted C₃₋₈ cycloalkyl, optionally-substituted C₃₋₈ cycloalkoxy, optionally-substituted 3- to 10-membered heterocycloalkyl, optionally-substituted 3- to 10-membered heterocycloalkoxy, optionally-substituted C₆₋₁₀ aryl, optionally-substituted C₆₋₁₀ aryloxy, optionally-substituted 5- to 10-membered heteroaryl and optionally-substituted 5- to 10-membered heteroaryloxy;
R^{1b} and R^{2b} are each independently selected from the group consisting of optionally-substituted C₃₋₈ cycloalkyl, optionally-substituted 3- to 10-membered heterocycloalkyl, optionally-substituted C₆₋₁₀ aryl and optionally-substituted 5- to 10-membered heteroaryl;
R³ is each independently selected from the group consisting of halogen, C₁₋₆ alkyl, C₁₋₆ haloalkyl, amino and hydroxy;
R⁴ is C₁₋₆ alkyl, C₁₋₆ haloalkyl, C₁₋₆ alkoxy, C₂₋₆ alkenyl or C₂₋₆ alkynyl;
k is 0 or 1;
1 is 0 to 2;
m is 0 or 1, provided that when k is 1 and m is 1, the terminal carbon atom of R⁴ may be combined with the carbon atom bonded to N in the parenthesis for k to form a 4- to 6-membered nitrogen-containing heterocycle which may be optionally substituted with the same or different one or more groups selected from the group consisting of halogen, hydroxy, C₁₋₆ alkyl and C₁₋₆ alkoxy at any replaceable positions; and
n is 0 to 4,
provided that 1-[1-[4,4-bis(4-fluorophenyl)butyl]-4-piperidinyl]-1,3-dihydro-2H-benzimidazol-2-one is excluded.
